# FASCICULE DE BREVET EUROPEEN

(11) **EP 2 401 620 B1**
(45) Date de publication et mention de la délivrance du brevet: **02.10.2013**
(21) Numéro de dépôt: 10710087.7
(22) Date de dépôt: 25.02.2010
(51) Int. Cl.: G01N 33/564

(54) **PROCÉDÉ DE DIAGNOSTIC D'UNE GRANULOMATOSE DE WEGENER**
VERFAHREN ZUR DIAGNOSE VON WEGENER-GRANULOMATOSE
METHOD FOR DIAGNOSING WEGENER'S GRANULOMATOSIS

(30) Priorité: 25.02.2009 FR 0951205
(43) Date de publication de la demande: 04.01.2012
(60) Demande divisionnaire: 13176657.8
(73) Titulaire: Assistance Publique Hôpitaux De Paris, 75004 Paris 4 (FR)
(72) Inventeur: MOUTHON, Luc, F-94160 Saint Mande (FR); DIB, Hanadi, F-94260 Fresnes (FR); REGENT, Alexis, F-75005 Paris (FR)
(74) Mandataire: Chajmowicz, Marion
(86) Numéro de dépôt international: PCT/FR2010/050331
(87) Numéro de publication internationale: WO 2010/097553

(56) Documents cités:
- US-A- 5 091 303
- GÖBEL U; EICHHORN J; KETTRITZ R; BRIEDIGKEIT L; SIMA D; LINDSCHAU C; HALLER H; LUFT F C: "Disease activity and autoantibodies to endothelial cells in patients with Wegener's granulomatosis." AMERICAN JOURNAL OF KIDNEY DISEASES : THE OFFICIAL JOURNAL OF THE NATIONAL KIDNEY FOUNDATION AUG 1996, vol. 28, no. 2, août 1996 (1996-08), pages 186-194, XP002544456 ISSN: 0272-6386
- LEE K H; ET AL: "Human alpha-Enolase From Endothelial Cells as a Target Antigen of Anti-Enothelial Cell Antibody in Behcet's Disease" ARTHRITIS AND RHEUMATISM, LIPPINCOTT, PHILADELPHIA, US, vol. 48, no. 7, 1 juillet 2003 (2003-07-01), pages 2025-2035, XP002995517 ISSN: 0004-3591
- DATABASE WPI Week 200477 Thomson Scientific, London, GB; AN 2004-784906 XP002544454 & WO 2004/094638 A1 (MEDICAL & BIOLOGICAL LAB CO LTD) 4 novembre 2004 (2004-11-04) cité dans la demande & DATABASE EPODOC EUROPEAN PATENT OFFICE, THE HAGUE, NL; 4 novembre 2004 (2004-11-04), KATO TOMOHIRO [JP]; NISHIOKA KUSUKI [JP]; OZAKI SHOICHI [JP]; KARASAWA RIE [JP]: "VASCULITIS ANTIGEN AND METHOD OF DIAGNOSING VASCULITIS" Database accession no. WO2004094638
- YOUINOU ET AL: "New target antigens for antiendothelial cell antibodies" IMMUNOBIOLOGY, URBAN UND FISCHER VERLAG, DE LNKD- DOI:10.1016/J.IMBIO.2005.10.011, vol. 210, no. 10, 13 décembre 2005 (2005-12-13), pages 789-797, XP025312689 ISSN: 0171-2985 [extrait le 2005-12-13]
- YOUINOU ET AL: "Endothelium, a target for immune-mediated assault in connective tissue disease" AUTOIMMUNITY REVIEWS, ELSEVIER, AMSTERDAM, NL LNKD- DOI:10.1016/J.AUTREV.2005.06.012, vol. 5, no. 3, 1 mars 2006 (2006-03-01), pages 222-228, XP005290690 ISSN: 1568-9972
- ALARD JEAN-ERIC ET AL: "HSP60 and anti-HSP60 antibodies in vasculitis: they are two of a kind." CLINICAL REVIEWS IN ALLERGY & IMMUNOLOGY OCT 2008 LNKD- PUBMED:18188708, vol. 35, no. 1-2, octobre 2008 (2008-10), pages 66-71, XP002579002 ISSN: 1080-0549
- DEROANNE C F; COLIGE A C; NUSGENS B V; LAPIERE C M: "Modulation of expression and assembly of vinculin during in vitro fibrillar collagen-induced angiogenesis and its reversal" EXPERIMENTAL CELL RESEARCH 19960501 US, vol. 224, no. 2, 1 mai 1996 (1996-05-01), pages 215-223, XP002544457 ISSN: 0014-4827
- BOUTIERE B ET AL: "Interet et limites du diagnostic biologique des anticorps anti-cellule endotheliale" REVUE FRANÇAISE DES LABORATOIRES, EDITIONS SCIENTIFIQUES ET MEDICALES ELSEVIER LNKD- DOI:10.1016/S0338-9898(97)80153-9, vol. 1997, no. 293, 1 mai 1997 (1997-05-01), pages 73-77, XP004895146 ISSN: 0338-9898

## Description

L'invention concerne un procédé *in vitro* de détection d'une granulomatose de Wegener comprenant la détermination de la présence et/ou de la quantité d'anticorps (Ac) anti-cellules endothéliales (AECA) ou anti-cellules musculaires lisses vasculaires (CMLV) anti-vinculine dans un échantillon biologique provenant d'un patient tel que défini dans les revendications.

### Etat de la technique

Les vascularites sont des pathologies orphelines dont la prévalence est faible, de l'ordre de 24 à 150 par million d'habitants. Elles représentent un groupe de maladies caractérisées par la présence de lésions inflammatoires au niveau des parois des vaisseaux. Les vascularites sont classées selon la taille des vaisseaux atteints et répondent à différents mécanismes pathogéniques dominants : une production de cytokines proinflammatoires et une activation des macrophages dans les vascularites intéressant les vaisseaux de gros calibre, comme que la maladie de Horton (ou artérite à cellules géantes) ; le dépôt de complexes immuns circulants responsables de l'activation de la voie classique du complément, et le recrutement de neutrophiles dans les vascularites intéressant les vaisseaux de taille moyenne, telles que la périartérite noueuse associée à une infection par le virus de l'hépatite B ; et une activation des neutrophiles par les Ac anti-cytoplasme de polynucléaires neutrophiles (ANCA), préférentiellement dans les vascularites intéressant les vaisseaux de petit calibre, telles que la granulomatose de Wegener et la polyangéite microscopique.

La maladie de Horton se manifeste par des céphalées dues à l'atteinte de l'artère temporale, associées à une altération de l'état général, à une pseudo-polyarthrite rhizomélique dans un cas sur deux et dans la très grande majorité des cas à un syndrome inflammatoire,

La granulomatose de Wegener est une vascularite granulomateuse intéressant surtout les sinus de la face, le poumon et le rein, associée dans les formes systémiques, dans 90 % des cas, à des Ac anti-protéinase 3.

La polyangéite microscopique est une vascularite nécrosante qui intéresse les vaisseaux de petit calibre et peut être à l'origine d'une atteinte glomérulaire et capillaire pulmonaire à l'origine d'un syndrome pneumo-rénal en plus des manifestations systémiques en rapport avec la vascularite.

Le syndrome de Churg et Strauss correspond à un asthme à début tardif d'évolution sévère associé à une hyperéosinophilie et une vascularite nécrosante.

Actuellement le diagnostic de vascularite ANCA-positive repose toujours sur une biopsie, qu'il s'agisse d'une biopsie cutanée, d'une biopsie rénale, d'une biopsie neuromusculaire ou autre. Les ANCA constituent une aide importante au diagnostic de vascularite systémique. Des ANCA anti-myéloperoxydase (MPO) sont ainsi présents chez 60 à 75% des malades atteints de polyangéite microscopique et 38% des malades atteints du syndrome de Churg et Strauss. En corollaire, une proportion notable de patients n'a pas d'ANCA, ce qui rend difficile le diagnostic, l'évaluation du pronostic, et la prise en charge thérapeutique.

Au cours de la maladie de Horton il n'y a pas aujourd'hui de marqueur biologique identifié. Le diagnostic d'une maladie de Horton repose dans la majorité des cas sur une biopsie de l'artère temporale, positive dans 80% des cas de maladie de Horton.

Il existe donc un besoin d'identifier des marqueurs immunologiques d'intérêt diagnostique et/ou pronostique au cours de la maladie de Horton et d'autres marqueurs immunologiques d'intérêt que les ANCA au cours des vascularites ANCA positives. La demande internationale WO2004/094638, en langue japonaise, fait mention d'une recherche d'anticorps anti-peroxirédoxine 2 dans le sérum de patients atteints de vascularite, mais reste semble-t-il isolée dans ce domaine.

Dans cette perspective, les inventeurs se sont intéressés aux anticorps (Ac) anti-cellules endothéliales (AECA), anticorps anti-cellules musculaires lisses vasculaires (CMLV). En particulier les AECA sont détectés et semblent jouer un rôle clé dans la pathogénie des vascularites (Guilpain et Mouthon, Clinic Rev Allerg Imunol, 2008 Oct;35(1-2):59-65). Ainsi, la fixation des AECA aux cellules endothéliales peut entraîner une destruction de la cellule cible par un mécanisme de cytotoxicité cellulaire dépendant des Ac (ADCC), peut induire l'apoptose et augmenter l'expression des molécules d'adhésion. Cependant, les cibles antigéniques de ces anticorps n'avaient jusqu'à présent pas été identifiées.

Göbel et al, American Journal of Kidney Diseases, 1996, 28(2):186-194, rapporte la présence d'auto-anticorps anti-cellules endothéliales dans le sérum de patients atteints de granulomatose de Wegener.

### Résumé de l'invention

Les inventeurs ont maintenant identifié des cibles antigéniques des anticorps anti-cellules endothéliales (AECA) et des anticorps anti-cellules musculaires lisses vasculaires (CMLV) dans les vascularites, en particulier dans la maladie de Horton et les vascularites ANCA-positives.

Sur cette base, l'invention fournit un procédé *in vitro* de détection d'une granulomatose de Wegener chez un sujet, comprenant la détermination de la présence et/ou de la quantité d'au moins un anticorps anti-cellules endothéliales (AECA) ou anti-cellules musculaires lisses vasculaires (CMLV), qui est un anticorps anti-vinculine, dans un échantillon biologique provenant d'un patient, la présence de l'anticorps anti-vinculine étant indicatrice d'une granulomatose de Wegener.

Dans un mode de réalisation particulier, le procédé de l'invention de détection d'une granulomatose de Wegener comprend en outre la détermination de la présence et/ou de la quantité d'au moins un autre anticorps anti-cellules endothéliales (AECA) ou anti-cellules musculaires lisses vasculaires (CMLV), ledit autre anticorps étant dirigé contre un antigène choisi parmi le groupe constitué de FUbp2 (Far upstream element-binding protein 2), Caldesmone, protéine précurseur de 78kDa régulée par le glucose, Heat shock cognate 71 kDa protein, Précurseur mitochondrial de la protéine de Stress-70, Lamne-A/C, ribonucléoprotéine K nucléaire hétérogène, sous-unité epsilon de la protéine 1 du complexe T, Précurseur mitochondrial de la protéine 60 kDa heat shock, Précurseur protéique de la disulfure-isomérase A1, Précurseur protéique de la disulfure-isomérase A3, sous-unité theta de la protéine 1 du complexe T, sous-unité beta de la protéine 1 du complexe T, Précurseur mitochondrial de la sous-unité alpha de l'ATP synthase, ribonucleoprotéine H nucléaire hétérogène, Chaîne bêta Tubuline, Fructose-bisphosphate aldolase A, Précurseur mitochondrial de la sous-unité alpha de l'ATP synthase, Précurseur de Calumenine, Reticulocalbine-3, Sous-unité 13 régulatrice non-ATPase du protéasome 26S, pyrophosphatase inorganique, Annexine A5, 14-3-3 protéine epsilon, 6-phosphogluconoladonase, Galectine-1, Précurseur mitochondrial de la Succinyl-CoA:3-ketoacid-coenzyme A transférase 1, et la ribonucléoprotéine D0 nucléaire hétérogène, dans un échantillon biologique provenant d'un patient, la présence de l'anticorps anti-vinculine et dudit au moins autre anticorps étant indicatrice d'une granulomatose de Wegener.

De préférence, la présence dudit au moins un anticorps dans l'échantillon biologique est comparée à une valeur contrôle, la présence dudit au moins un anticorps en une quantité supérieure à la valeur contrôle étant indicatrice d'une vascularite ou d'un risque de développer une vascularite.

### Description détaillée de l'invention

Les inventeurs ont utilisé des cellules endothéliales de veines ombilicales humaines normales (HUVEC) comme source d'antigènes et testé les sérums de patients ayant de maladie de Horton, ou une vascularite systémique associée à des anticorps anti-cytoplasme de polynucléaires neutrophiles (ANCA) et des sujets sains.

Pour identifier les cibles des anticorps, les inventeurs ont utilisé un immunotransfert en deux dimensions l'identification des antigènes se faisant par spectrométrie de masse.

Les inventeurs ont également testé les sérums de patients atteints d'une maladie de Horton.

### Définitions :

Le terme « échantillon biologique » se réfère à tout échantillon biologique provenant d'un patient. Des exemples d'échantillons incluent des liquides biologiques, des biopsies tissulaires. De manière préférentielle, l'échantillon peut être du sang, du sérum, de la salive, de l'urine, du sperme. De manière davantage préférée, l'échantillon biologique est un échantillon de sang ou de sérum.

Le terme « patient » se réfère à tout sujet susceptible d'être testé. De préférence il s'agit d'un humain, mais le terme inclut tout autre mammifère, tel que des chiens, chats, rongeurs, bétail, chevaux, singes etc. Le patient peut être testé quel que soit son sexe ou son âge. Le patient peut être un sujet à risque, être asymptomatique ou présenter des signes précoces ou avancés d'une vascularite. Le terme « diagnostic » signifie l'identification de la pathologie ou l'évaluation de l'état de sévérité de la pathologie.

Le terme « pronostic » signifie l'évaluation du risque d'aggravation, et de ses conséquences.

Le terme « valeur contrôle » se réfère à une valeur basale correspondant à la moyenne des valeurs obtenues avec l'échantillon biologique de sujets sains, non affectés par une vascularite ou une maladie susceptible d'entraîner une vascularite. Il peut s'agir d'une valeur statistique de référence.

Pour évaluer l'évolution de la pathologie, il peut être utile de tester un patient et de contrôler l'effet d'un traitement ou l'évolution de la pathologie, en testant de nouveau le patient, par exemple à plusieurs mois d'intervalle. Dans ce cas, les résultats du second test sont comparés aux résultats du premier test, ainsi que souvent à la valeur dite « contrôle ».

Une quantité d'anticorps « supérieure à la valeur contrôle » signifie généralement une augmentation statistiquement significative, par exemple d'au moins deux déviations standards au dessus de la moyenne des densités optiques des réactivités IgG de l'ensemble des sujets sains.

Par « antigène de capture », on entend un antigène, de préférence fixé sur une phase solide, qui est capable de retenir ledit au moins un anticorps présent dans un échantillon biologique, par liaison affine. L'antigène de capture peut être marqué.

Le terme « marqué » se réfère aussi bien à un marquage direct (par le biais d'enzymes, radioisotopes, fluorochromes, composés luminescents, etc...) qu'à un marquage indirect (par exemple, par le biais d'anticorps eux-mêmes marqués de manière directe ou à l'aide de réactifs d'une "paire d'affinité " marquée, telle que, mais non exclusivement, la paire avidine marquée-biotine, etc.

Par « vascularite », on entend toute vascularite systémique primitive ainsi que des vascularites secondaires en particulier une vascularite médicamenteuse, vascularite associée à une connectivite, ou vascularite d'origine infectieuse. Les vascularites visées comprennent ainsi les vascularites atteignant les vaisseaux de petit calibre, comme la granulomatose de Wegener, la polyangéite microscopique et le syndrome de Churg et Strauss, les vascularites atteignant les vaisseaux de calibre moyen, telles que la périartérite noueuse, les vascularites atteignant les vaisseaux de gros calibre, telles que la maladie de Horton.

### Anticorps identifiés :

Comme indiqué dans la partie « exemples », les inventeurs ont identifié plusieurs anticorps anti-cellules endothéliales (AECA) ou anti-cellules musculaires lisses vasculaires (CMLV) chez des malades ayant une vascularite.

Ces cibles antigéniques sont impliquées notamment dans le stress oxydatif, le métabolisme cellulaire et la maintenance de l'homéostasie cellulaire.

La détection et/ou la quantification de ces anticorps peut être mise en oeuvre pour détecter une vascularite.

Les antigènes reconnus par les anticorps identifiés sont listés ci-dessous (cf aussi les tableaux 1 à 9, de la partie « Exemples »). Un listing de ces séquences protéiques est en outre annexé.

Les numéros d'accès dans la base de données SwissProt et les séquences correspondantes sont donnés à titre indicatif.
Vinculine (Swiss-Prot: P18206, SEQ ID NO:1)
FUbp2 (Far upstream element-binding protein 2) (Swiss-Prot: Q92945, SEQ ID NO:2)
Caldesmone (Swiss-Prot: Q92945, SEQ ID NO:3)
protéine précurseur de 78kDa régulée par le glucose (78 kDa glucose-regulated protein precursor) (Swiss-Prot: P11021, SEQ ID NO:4)
Heat shock cognate 71 kDa protein (Swiss-Prot: P11142, SEQ ID NO:5)
Précurseur mitochondrial de la protéine de Stress-70 (Swiss-Prot: P38646, SEQ ID NO:6)
Lamine-A/C (Swiss-Prot: P02545, SEQ ID NO:7)
ribonucleoproteine K nucléaire hétérogène (Swiss-Prot: P61978, SEQ ID NO:8)
sous-unité epsilon de la protéine 1 du complexe T (Swiss-Prot: P48643, SEQ ID NO:9)
Précuseur mitochondrial de la protéine 60 kDa heat shock (Swiss-Prot: P10809, SEQ ID NO:10)
Précuseur protéique de la disulfure-isomérase A1 (Swiss-Prot: P07237, SEQ ID NO:11)
Précurseur protéique de la disulfure-isomérase A3 (Swiss-Prot: P30101, SEQ ID NO:12)
sous-unité thêta de la protéine 1 du complexe T (Swiss-Prot: P50990, SEQ ID NO:13)
sous-unité beta de la protéine 1 du complexe T (Swiss-Prot: P78371, SEQ ID NO:14)
Précurseur mitochondrial de la sous-unité alpha de l'ATP synthase (Swiss-Prot: P25705, SEQ ID NO:15)
ribonucléoprotéine H nucléaire hétérogène (Swiss-Prot: P31943, SEQ ID NO:16)
Chaîne beta Tubuline (Swiss-Prot: P07437, SEQ ID NO:17)
Fructose-bisphosphate aldolase A (Swiss-Prot: P04075, SEQ ID NO:18)
Précurseur de Calumenine (Swiss-Prot: 043852, SEQ ID NO:19)
Réticulocalbine-3 (Swiss-Prot: Q96D15, SEQ ID NO:20)
Sous-unité 13 régulatrice non-ATPase du protéasome 26S (Swiss-Prot: Q9UNM6, SEQ ID NO:21)
pyrophosphatase inorganique (Swiss-Prot: Q15181, SEQ ID NO:22)
Annexine A5 (Swiss-Prot: P08758, SEQ ID NO:23)
14-3-3 protéine epsilon (Swiss-Prot: P62258, SEQ ID NO:24)
6-phosphogluconolactonase (Swiss-Prot: 095336, SEQ ID NO:25)
Galectine-1 (Swiss-Prot: P09382, SEQ ID NO:26)
Précurseur mitochondrial de la Succinyl-CoA:3-ketoacid-coenzyme A transférase 1 (Swiss-Prot: P55809, SEQ ID NO:27)
ribonucléoprotéine D0 nucléaire hétérogène (Swiss-Prot: Q14103, SEQ ID NO:28)
Sous-unité 7 régulatrice de la protéase 26S (Swiss-Prot: P35998, SEQ ID NO:29)
Hème oxygénase 2 (Swiss-Prot: P30519, SEQ ID NO:30)
Histone H2B type F-S (Swiss-Prot: P57053, SEQ ID NO:31)
sous-unité alpha type-5 du Protéasome (Swiss-Prot: P28066, SEQ ID NO:32)
sous-unité béta type-2 du Protéasome (Swiss-Prot: P49721, SEQ ID NO:33)
Protéine 4 associée au cytosquelette (Swiss-Prot: Q07065, SEQ ID NO:34)
Uroporphyrinogen decarboxylase (Swiss-Prot: P06132, SEQ ID NO:35)
Adénine phosphoribosyltransferase (Swiss-Prot: P07741, SEQ ID NO:36)
Profiline-1 (Swiss-Prot: P07737, SEQ ID NO:37)
Plastine-3 (Swiss-Prot: P13797, SEQ ID NO:38)
protéine 2 liée au récepteur du facteur de croissance (Swiss-Prot: P62993, SEQ ID NO:39)
ribonucléoprotéine L nucléaire hétérogène (Swiss-Prot: P14866, SEQ ID NO:40)
Précurseur de la Reticulocalbin-1 (Swiss-Prot: Q15293, SEQ ID NO:41)
Serpine B9 (Swiss-Prot: P50453, SEQ ID NO:42)
Précuseur mitochondrial de la sous-unité alpha de la Isocitrate déshydrogenase [NAD] (Swiss-Prot: P50213, SEQ ID NO:43)
GMP synthase [hydrolysant la glutamine] (Swiss-Prot: P49915, SEQ ID NO:44)
Sous-unité zeta de la protéine 1 du complexe T (Swiss-Prot: P40227, SEQ ID NO:45)
Cofiline-1 (Swiss-Prot: P23528, SEQ ID NO:46)
Précurseur mitochondrial de l'aconitate hydratase (Swiss-Prot: Q99798, SEQ ID NO:47)
Protéine de la membrane interne des mitochondries (Swiss-Prot: Q16891, SEQ ID NO:48)
ribonucleoprotéine K nucléaire hétérogène (Swiss-Prot: P61978, SEQ ID NO :49)
Précurseur mitochondrial du facteur d'élongation Tu (Swiss-Prot: P49411, SEQ ID NO:50)
Alcool déshydrogénase [NADP+] (Swiss-Prot: P14550, SEQ ID NO:51)
Sialic acid synthase (Swiss-Prot: Q9NR45, SEQ ID NO:52)
S-formylglutathion hydrolase (Swiss-Prot: P10768, SEQ ID NO:53)
sous-unité beta-2-like 1 de la protéine de liaison au nucleotide guanine (Swiss-Prot: P63244, SEQ ID NO:54)
Purine nucleoside phosphorylase (Swiss-Prot: P00491, SEQ ID NO:55)
Prohibitine (Swiss-Prot: P35232, SEQ ID NO:56)
Précurseur mitochondrial de la protéine de liaison au C1q (Swiss-Prot: Q07021, SEQ ID NO:57)
ATPase du réticulum endoplasmique transitionnel (Swiss-Prot: P55072, SEQ ID NO:58)
et Nucleoside diphosphate kinase A (Swiss-Prot: P15531, SEQ ID NO:59).
   ainsi que
annexine A2 (Swiss-Prot: P07355, SEQ ID NO:60).
alpha-énolase (Swiss-Prot: P06733, SEQ ID NO:61).
FUbp1 (Far upstream element-binding protein 1) (Swiss-Prot: Q96AE4, SEQ ID NO:62).
précurseur mitochondrial de la dihydrolipoiyl déshydrogénase (Swiss-Prot: P09622, SEQ ID NO:63).
inosine-5'-monophosphate déshydrogénase 2 (Swiss-Prot: P12268, SEQ ID NO:64).
le précurseur 1 de la tripeptidy-peptidase (Swiss-Prot: PO14773, SEQ ID NO:88). le précurseur mitochondrial de la fumarate hydratase (Swiss-Prot: P07954, SEQ ID NO:65).
la ribonucléoprotéine D0 nucléaire hétérogène (Swiss-Prot: Q14103, SEQ ID NO:66).
la protéine 1 des domaines PDZ et LIM (Swiss-Prot: 000151, SEQ ID NO:67).
la protéine ribosomique P0 acide 60S (Swiss-Prot: P05388, SEQ ID NO:68).
la protéine 2 du canal anion-sélectif voltage-dépendant (Swiss-Prot: P45880, SEQ ID NO:69).
protéine DJ-1 (Swiss-Prot: Q99497, SEQ ID NO:70).
peptidyl-prolyl cis-trans isomérase A (Swiss-Prot: P62937, SEQ ID NO:71).
précurseur mitochondrial de la peroxyde réductase thioredoxine-dépendante (Swiss-Prot: P30048, SEQ ID NO:72).
le précurseur membre 11 sous-famille B homologue DNAJ (Swiss-Prot: Q9UBS4, SEQ ID NO:73).
la glutaredoxine-3 (Swiss-Prot: P076003, SEQ ID NO:74).
la protéine inhibitrice 2 de dissociation Rho GDP (Swiss-Prot: P52566, SEQ ID NO:75).
la glutathion S-transférase P (Swiss-Prot: P09211, SEQ ID NO:76).
et la peroxyrédoxine (Swiss-Prot: P32119, SEQ ID NO:77).
   et encore
la protéine putative heat shock HSP 90-alpha A2 (Swiss-Prot: Q14568, SEQ ID NO:78).
Coatomer sous-unité alpha (Swiss-Prot: P53621, SEQ ID NO:79).
UDP-glucose 6-deshydrogénase (Swiss-Prot: 060701, SEQ ID NO:80).
Actine, cytoplasmique 1 (Swiss-Prot: P60709, SEQ ID NO:81).
membre E POTE ankyrin domain family (Swiss-Prot: Q6S8J3, SEQ ID NO:82).
Nucléophosmine (Swiss-Prot: P06748, SEQ ID NO:83).
Facteur d'élongation 2 (Swiss-Prot: P13639, SEQ ID NO:84).
Member 1 sous-famille A, DnaJ homolog (Swiss-Prot: P31689, SEQ ID NO:85).
Actine, cytoplasmique 2 (Swiss-Prot: P63261, SEQ ID NO:86).
sous-unité 8 régulatrice de la protéase 26S (Swiss-Prot: P62195, SEQ ID NO:87).

Parmi les auto-anticorps détectés, plusieurs sont particulièrement pertinents. Il s'agit des anticorps dirigés contre les antigènes suivants :
Caldesmone
protéine précurseur de 78kDa régulée par le glucose
Heat shock cognate 71 kDa protein
sous-unité epsilon de la protéine 1 du complexe T
Précurseur protéique de la disulfure-isomérase A3
   ou
Précurseur de Caluménine,
et surtout les anticorps dirigés contre la vinculine ou la lamine.

Les six premiers antigènes sont reconnus par plus de 60% des pools des trois sérums de patients atteints de granulomatose de Wegener testés, deux d'entre eux (caldesmone et précurseur de caluménine) étant en outre reconnus par les pools de trois sérums de patients ayant un syndrome de Churg et Strauss sans ANCA.

Les anticorps identifiés par les inventeurs peuvent être mis en oeuvre dans les procédés décrits seuls ou en combinaison. La détection et/ou la quantification peut être réalisée vis-à-vis d'un seul des anticorps identifiés, ou peut concerner une pluralité d'anticorps. On ainsi peut imaginer la réalisation du procédé sur un support solide, par exemple une microplaque, sur lequel sont disposés de manière définie et ordonnée les antigènes correspondant à la pluralité d'anticorps à détecter et/ou quantifier.

Selon un mode de réalisation, les procédés décrits mettent en oeuvre la détection d'un anticorps dirigé contre un antigène identifié dans le tableau 1, 8 ou 9, pour le diagnostic, le pronostic ou le suivi d'une granulomatose de Wegener.

Plus particulièrement, est décrit un procédé pour le diagnostic, le prognostic ou le suivi d'une granulomatose de Wegener, ledit procédé comprenant la détection d'un anticorps dirigé contre un antigène choisi parmi Caldesmone, protéine précurseur de 78kDa régulée par le glucose, Heat shock cognate 71 kDa protein , sous-unité epsilon de la protéine 1 du complexe T, Précurseur protéique de la disulfure-isomérase A3 , ou Précurseur de Calumenine.

Selon un autre mode de réalisation, les procédés décrits mettent en oeuvre la détection d'un anticorps dirigé contre un antigène identifié dans le tableau 2 ou le tableau 5, pour le diagnostic, le pronostic ou le suivi d'une polyangéite microscopique. Plus particulièrement les procédés décrits peuvent utiliser la détection d'un anticorps dirigé contre un antigène identifié dans le tableau 2, 8 ou 9, pour le diagnostic, le pronostic ou le suivi d'une polyangéite microscopique avec ANCA anti-MPO. Par ailleurs, les procédés décrits peuvent utiliser la détection d'un anticorps dirigé contre un antigène identifié dans le tableau 5, 8 ou 9, pour le diagnostic, le pronostic ou le suivi d'une polyangéite microscopique sans ANCA anti-MPO.

Selon un autre mode de réalisation, les procédés décrits mettent en oeuvre la détection d'un anticorps dirigé contre un antigène identifié dans le tableau 3 ou 4, 8 ou 9, pour le diagnostic, le pronostic ou le suivi d'un syndrome de Churg et Strauss.

Plus particulièrement, les procédés décrits mettent en oeuvre la détection d'un anticorps dirigé contre un antigène identifié dans le tableau 3, 8 ou 9, pour le diagnostic, le pronostic ou le suivi d'un syndrome de Churg et Strauss avec ANCA anti-MPO.

Par ailleurs, les procédés décrits peuvent utiliser la détection d'un anticorps dirigé contre un antigène identifié dans le tableau 4, pour le diagnostic, le pronostic ou le suivi d'un syndrome de Churg et Strauss sans ANCA anti-MPO.

Selon un autre mode de réalisation, les procédés décrits mettent en oeuvre la détection d'un anticorps dirigé contre un antigène identifié dans l'un des tableaux 6, 7, 8 ou 9, pour le diagnostic, le pronostic ou le suivi d'une maladie de Horton, l'antigène étant de préférence la vinculine ou la lamine.

### Dosage des anticorps :

L'échantillon biologique est de préférence un échantillon de sérum, de préférence dilué au 1/100ème, ou plus, par exemple au 1/200ème ou 1/400ème.

De manière avantageuse, la quantité d'anticorps peut être déterminée par un immunoessai.

L'échantillon biologique peut être éventuellement traité dans une étape préalable, ou mis directement en présence d'au moins un antigène de capture.

Le procédé selon l'invention peut être réalisé selon divers formats bien connus de l'homme du métier: en phase solide ou en phase homogène; en un temps ou en deux temps; en méthode compétitive, à titre d'exemples non limitatifs.

Selon un mode de réalisation préféré, l'antigène de capture est immobilisé sur une phase solide. On peut utiliser, à titre d'exemples non limitatifs de phase solide, des microplaques, en particulier des microplaques de polystyrène, telles que celles commercialisées par la société Nunc, Danemark. On peut également utiliser des particules ou des billes solides, des billes paramagnétiques, telles que celles fournies par Dynal ou Merck-Eurolab (France) (sous la marque EstaporTM), ou encore des tubes à essai en polystyrène ou polypropylène, etc.

Un format d'immunoessai de détection des anticorps par compétition est également possible. D'autres modalités d'immunoessai sont encore envisageables et bien connues de l'homme du métier.

Dosages ELISA, radioimmunoessais, ou toute autre technique de détection peuvent être mis en oeuvre pour révéler la présence des complexes antigènesanticorps formés.

Selon un mode particulier de réalisation préféré, l'antigène de capture correspond à une protéine entière ou à un fragment de ladite protéine. Par exemple, le procédé de l'invention comprend la mise en contact d'un échantillon biologique avec une protéine entière reconnue par l'anticorps à détecter et/ou quantifier.

Dans un exemple particulier, l'antigène de capture peut être couplé à une glutathion S transférase (GST), avant d'être déposé sur une microplaque.

A titre illustratif, les échantillons de sérum à tester, par exemple dilués au 1/100ème, sont mis à incuber sur la microplaque. Après lavage, des anticorps anti-Fcy humain marqués (par exemple avec une phosphatase alcaline) sont ajoutés, les complexes étant révélés (par exemple par ajout d'un substrat de la phosphatase dont le clivage peut être détecté par lecture de l'absorbance).

### Patients visés :

Les patients visés sont atteints d'une vascularite, suspectés d'être atteints d'une vascularite ou sont susceptibles de développer une vascularite.

Il peut s'agir de vascularites chez des patients ANCA-positifs, ou chez des patients qui n'ont pas d'auto-anticorps ANCA.

Les méthodes décrites permettent de diagnostiquer, pronostiquer ou de suivre l'évolution de tout type de vascularite, et particulier une granulomatose de Wegener, une polyangéite microscopique, un syndrome de Churg et Strauss, ou une maladie de Horton.

Les exemples suivants illustrent l'invention sans en limiter la portée.

### Exemples

### Exemple 1 : Caractérisation des cibles antigéniques des anticorps AECA dans les vascularites ANCA-positives

Les sérums de 45 patients ayant une vascularite ANCA-positive (15 ayant une granulomatose de Wegener (WG), 12 ayant une polyangéite microscopique (MPA), 12 ayant un syndrome de Churg Strauss (CSS) ont été testés par pools de trois et comparés à un pool de sérums de 12 sujets sains. Les réactivités IgG sériques étaient analysées à l'aide de gels d'électrophorèse en deux dimensions suivis d'immunotransferts utilisant des antigènes de cellules endothéliales de veines ombilicales humaines normales (HUVEC) (cf Servettaz et al, Proteomics. 2008 Mar;8(5):1000-8).

Les IgG sériques des pools de patients atteints de WG avec ANCA anti-protéinase 3 (PR3) (n=5), MPA avec ANCA anti-myeloperoxydase (MPO) (n=2), MPA sans ANCA anti-MPO (n=2), CSS avec ANCA anti-MPO (n=1) et CSS sans ANCA anti-MPO (n=2), reconnaissaient 107±17, 148, 211, 128, et 101 spots protéiques, respectivement, tandis que les IgG sériques de sujets sains reconnaissaient 79 tâches protéiques. Les IgG sériques de patients atteints de WG avec anti-PR3, MPA avec anti-MPO, MPA sans anti-MPO, CSS avec anti-MPO et CSS sans anti-MPO reconnaissaient spécifiquement 37, 12, 22, 15 et 23 tâches protéiques, respectivement. Les antigènes cibles étaient impliqués dans le stress oxydatif, le métabolisme cellulaire et d'autres fonctions biologiques cellulaires clés.

### Méthode d'identification :

### Electrophorèse à deux dimensions

Les inventeurs ont utilisé un pH de 3 à 10 et un gradient d'acrylamide de 7% à 18% dans toutes les expériences, ce qui a permis d'étudier une grande quantité d'antigènes de 10 à 200kDa.

Les protéines ont été soumises à une iso-électrofocalisation sur le Protean IEF Cell System, comme décrit dans (Gôrg et al, 2000, Electrophoresis, 21(6):1037-53).

Brièvement, immédiatement après l'iso-électrofocalisation, les échantillons ont été décongelés et dilués dans du tampon IPG contenant de l'urée ultra-pure 7M (VWR, Fontenay-Sous-Bois, France), 2M thiourée (Sigma), 4% CHAPS (Sigma), 0.002% Triton X100 (Sigma), 60µl de véhicule ampholyte pH 3-10 (Pharmalytes 3-10, Amersham Biosciences, Uppsala, Sweden) et du bleu de bromophénol (Sigma). Pour la préparation des gels 2-D, 100µg de protéines d'HUVEC ont été chargés sur les bandes IPG. Celles-ci ont été réhydratées et soumises à une électrophorèse automatisée pendant 12 h à 50V, 1 h à 200V, 1 h à 1000V, et 7 h à 10,000V (6 h linéaire et 1 h rapide).

Avant la deuxième dimension, les bandes ont été équilibrées pendant 15 min dans 10ml de la première solution d'équilibrage (51 mM Tris [Amersham Biosciences], 6mM urée, 40% (v/v) glycérol, 52mM SDS [Amersham Biosciences], 32.4mM DTT), puis pendant 20 min dans une seconde solution d'équilibrage (51mM Tris, 6mM urée, 40% [v/v] glycérol, 52mM SDS, 86.5mM iodoacétamide). Les bandes équilibrées ont été transféré sur le gel de gradient 7%-18% de polyacrylamide. Dix microlitres de marqueurs de poids moléculaire (PM) Precision Plus Protein Unstained Standards (Bio-Rad) ont été chargés sur chaque gel. La seconde dimension a été menée sur un système Laemmli sur gels de gradients linéaires 7%-18% polyacrylamide (20cm x 20cm x 1.5mm): d'une solution à 18.5% d'acrylamide PAGE (Amersham Biosciences) 2.5M, piperazine diacrylamide/diacrylyl (PDA) (Bio-Rad) 24.7mM, 0.375M Tris-HCl (Amersham Biosciences) pH 8.8, glycérol (Sigma) 15% (v/v), SDS 3.5mM, TEMED (Bio-Rad) 0.05% (v/v), et ammonium persulfate (APS) (Bio-Rad) 1.6mM, et une solution à 7% d'acrylamide 1.0M, PDA 10mM, 0.375M Tris-HCl pH 8.8, SDS 3.5mM, eau bidistillée, TEMED 0.06% (v/v), et APS 2.4mM ont été mélangées. Les gels d'IPG équilibrés ont été scellés sur le dessus des gels de polyacrylamide avec 1 % d'agarose contenant du bleu de bromophénol, et du tampon d'électrophorèse (24.8mM Tris, 192mM glycine, et 0.1% SDS) a été ajouté. Les gels ont été soumis à une électrophorèse initialement à 40V (constant) pendant 1h puis à 15mA/gel pendant 21 h 15 min.

### Electro-transfert et immunoblot

Les gels ont été transférés sur membranes de PVDF membranes (Millipore, Bedford, MA, USA) par transfert semi-sec (Bio-Rad) à 320mA pendant 1 h 30 min. Après blocage avec de PBS-0.2% Tween pendant 90 min, les membranes ont été incubées une nuit at 4°C avec les pools de serums de 3 patients phénotypiquement identiques (granulomatose de Wegener, polyangéite microscopique ou syndrome de Churg et Strauss) et les pools de sérums de 14 donneurs de sang sains, à une dilution de 1:100. Les membranes ont été lavées avant incubation avec un second Ac de lapin anti-Fcy humain couplé à la phosphatase alkaline (Dako, Glostrup, Denmark) pendant 90 min à température ambiante. Les immunoréactivités ont été révélées en utilisation un substrat NBT-BCIP substrate (Sigma). Les réactivités spécifiques ont été déterminées par densitomètre (GS-800, Bio-Rad) à l'aide du logiciel Quantity one (Bio-Rad). Les membranes ont ensuite été colorées à l'or colloïdal (Protogold, British Biocell International, Cardiff, UK) et soumises à une seconde analyse densitométrique pour enregistrer les spots des protéines marquées pour chaque gel.

### Marquage des gels

Les gels analytiques ont été colorés avec du nitrate d'argent ammoniacal.

### Analyse des images des gels et transferts 2-D

Les images des gels et des membranes obtenues à l'aide du densitomètre GS-800 (Bio-Rad) ont été analysées par le système Image Master 2-D Platinum 6 (Amersham Biosciences), avant et après coloration à l'or colloïdal. Les marquages spécifiques ont été reliés manuellement aux tâches protéiques sondées IgG sur les deux images. L'algorithme transférait automatiquement ces marquages de l'image du blot 2-D coloré à l'or colloïdal aux images des gels colorés au nitrate d'argent.

### Digestion du gel par la trypsine

La digestion du gel a été menée par le robot Freedom EVO 100 digester/spotter (Tecan, Männedorf, CH). Les tâches ont été décolorées deux fois avec un mélange de 100mM ammonium bicarbonate (ABC) et 50% ACN pendant 45 min à 22°C puis séchées avec 100% ACN pendant 15 min. Elles ont ensuite été soumises à un traitement de 25mM ABC contenant 10mM DTT pendant 1 h à 60°C puis ensuite alkylées avec 55mM iodoacétamide dans 25mM ABC pendant 30 min dans le noir à 22°C. Les morceaux de gel ont été lavés deux fois dans 25mM ABC et réduits deux fois dans 100% ACN pendant 15 min et séchés dans 100% ACN pendant 10 min. Les bandes étaient complètement déshydratées après 1 h à 60°C. Les morceaux de gel ont été incubés dans 13µl de trypsine (Sequencing Grade Modified Trypsin de Promega, WI, USA; 12.5µg/ml dans 40mM ABC-10% ACN pH 8.0) une nuit à 40°C. Après digestion, les peptides ont été lavés avec 30µl de 25mM ABC, réduits avec 100% ACN et extraits deux fois avec un mélange de 50% ACN-5% d'acide formique (FA). Les extraits ont ensuite été séchés par centrifugation sous vide (Eppendorf, Hamburg, Germany). Finalement les peptides ont été désalés à l'aide de C18-ZipTips (Millipore) et deux élutions, la première avec 50% ACN-5% FA puis avec 80% ACN-5% FA. Les élutions regroupées ont été mises à sécher à temperature ambiante.

### Identification des protéines par spectrométrie de masse (MS)

Pour les analyses MS et MS/MS, les peptides ont été redissous dans 4µl CHCA (5mg/ml dans 50% ACN-0.1% TFA). Un microlitre et demi de chaque échantillon a été déposé directement sur une plaque MALDI (Applied Biosystems, Foster City, CA, USA). Les gouttes ont été mises à sécher à température ambiante. L'analyse des échantillons a utilisé un spectromètre de masse MALDI-TOF-TOF 4800 (Applied Biosystems). L'acquisition des spectres et leur processing ont été réalisés par le logiciel 4000 series explorer (Applied Biosystems) version 3.5.28193. Le calibrage externe de la plaque a été réalisé par 4 points déposés aux 4 coins de la plaque avec un mélange de 5 standards externes (PepMix 1, LaserBio Labs, Sophia Antipolis, France). Les masses de peptides ont été acquises par étapes de 50 spectres de 900 à 4000Da. Les spectres MS ont été additionnés à partir de 1000 coups de laser avec un laser Nd-YAG opérant à 355nm et 200Hz. Après filtration des pics contaminants de trypsine, kératine et de matrice, jusqu'à 15 ions parents ont été sélectionnés pour une fragmentation MS/MS subséquente, selon leur masse, l'intensité du signal, le rapport signal sur bruit, et l'absence de masses voisines dans le spectre MS. Les spectres MS/MS ont été acquis en mode 1 kV positif, et 1000 coups ont été additionnés 50 par 50. La recherche sur bases de données a été menée à l'aide du logiciel Mascot 2.2 (MatrixScience, London, UK) via GPS explorer (Applied Biosystems) version 3.6 combinant les interrogations MS et MS/MS sur les protéines humaines de la banque Swissprot 54.5 (www.expasy.org). Les paramètres de recherche étaient les suivants: carbamidomethylation possible des cystéines et oxydation possible des méthionines. Jusqu'à un clivage trypsique manqué était permis, et une tolérance de 30ppm pour l'exactitude de la masse pour les précurseurs, et 0.3Da pour les fragments a été permise pour toutes les recherches de masses trypsiques.

L'identification a été basée sur un score Mascot au dessus du niveau de significativité (i.e. < 5%). Dans le cas où des peptides correspondent à des membres multiples d'une famille de protéines, la protéine rapportée est celle avec le plus grande nombre de correspondances (« peptide matches »).

### Résultats :

Les inventeurs ont identifié 37 tâches protéiques correspondant à 28 antigènes cibles différents reconnus spécifiquement par les IgG d'au moins 20% des patients ayant une granulomatose de Wegener, 15 tâches protéiques correspondant à 14 antigènes cibles reconnus spécifiquement par les patients ayant une polyangéite microscopique sans Ac anti-MPO, 5 antigènes cibles reconnus spécifiquement par les patients ayant une polyangéite microscopique avec Ac anti-MPO, 15 tâches protéiques correspondant à 10 antigènes cibles reconnus spécifiquement par les patients ayant un syndrome de Churg et Strauss sans Ac anti-MPO, et 7 antigènes cibles reconnus spécifiquement par les patients ayant un syndrome de Churg et Strauss avec Ac anti-MPO.

Les résultats détaillés sont présentés dans les tableaux 1 à 5 ci-dessous.

**Tableau 1. Antigènes cibles des AECA des patients atteints de Granulomatose de Wegener**

| | | | | | spectrométrie de masse | | | |
|---|---|---|---|---|---|---|---|---|
| N° sur le gel | Protéine | Numéro d'accès SwissProt | PM théorique / estimé (kDa) | PI théorique / estimé | Nombre de peptides identifies uniques^{#} | score ion total | Meilleur score ion | Couverture de la séquence (%) |
| 228 | Vinculine | VINC_HUMAN | 124/116 | 5.5/6.6 | 3/13 | 53 | 34 | 15 |
| 382 | Far upstream element-binding protein 2 | FUBP2_HUMAN | 73/92 | 6.8/7.3 | 6/9 | 174 | 59 | 16 |
| 387 | Far upstream element-binding protein 2 | FUBP2_HUMAN | 73/91 | 6.8/7.1 | 2/9 | 90 | 53 | 17 |
| **438** | **Caldesmone** | **CALD1_HUMAN** | **93/83** | **5.6/6.6** | **2/9** | **58** | **44** | **13** |
| **518** | **78 kDa glucose-regulated protein precursor** | **GRP78_HUMAN** | **72/75** | **5.1/5.4** | **13/21** | **1210** | **144** | **42** |
| 546 | Heat shock cognate 71 kDa protein | HSP7C_HUMAN | 71/75 | 5.4/5.9 | 9/13 | 284 | 73 | 29 |
| **575*** | **Heat shock cognate 71 kDa protein** | **HSP7C_HUMAN** | **71/75** | **5.4/5.7** | **9/10** | **308** | **77** | **21** |
| 579* | Précurseur mitochondrial de la protéine de Stress-70 | GRP75_HUMAN | 73/75 | 5.9/6.4 | 9/23 | 849 | 192 | 46 |
| 631 | Lamine-A/C | LMNA_HUMAN | 74/71 | 6.6/6.9 | 6/10 | 184 | 48 | 15 |
| 646 | Lamine-A/C | LMNA_HUMAN | 74/70 | 6.6/7 | 12/28 | 482 | 71 | 46 |
| 712* | Heat shock cognate 71 kDa protein | HSP7C_HUMAN | 71/64 | 5.4/5.4 | 8/13 | 298 | 53 | 26 |
| 738 | ribonucleoproteine K nucléaire hétérogène | HNRPK_HUMAN | 51/62 | 5.4/5.8 | 6/10 | 224 | 54 | 31 |
| **740** | **sous-unité epsilon de la protéine 1 du complexe T** | **TCPE_HUMAN** | **60/61** | **5.5/5.8** | **10/16** | **264** | **64** | **36** |
| 747* | Précuseur mitochondrial de la protéine 60 kDa heat shock | CH60_HUMAN | 61/62 | 5.7/5.6 | 7/7 | 509 | 123 | 21 |
| 776* | Précuseur protéique de la disulfure-isomérase A1 | PDIA1_HUMAN | 57/59 | 4.8/5 | 10/20 | 769 | 119 | 52 |
| **797** | **Précurseur protéique de la disulfure-Isomérase A3** | **PDIA3_HUMAN** | **57/57** | **6/6.3** | **10/15** | **782** | **163** | **38** |
| 809 | Précurseur protéique de la disulfure-isomérase A3 | PDIA3_HUMAN | 57/56 | 6/6.1 | 12/15 | 785 | 145 | 40 |
| 813 | sous-unité theta de la protéine 1 du complexe T | TCPQ_HUMAN | 60/57 | 5.4/6 | 2/2 | 67 | 33 | 3 |
| 814 | Précurseur protéique de la disulfure-isomérase A3 | PDIA3_HUMAN | 57/56 | 6/6.0 | 6/14 | 318 | 80 | 36 |
| 820 | sous-unité beta de la protéine 1 du complexe T | TCPB_HUMAN | 57/55 | 6/6.6 | 11/12 | 421 | 92 | 38 |
| 821 | sous-unité theta de la protéine 1 du complexe T | TCPB_HUMAN | 57/56 | 6/6.6 | 12/17 | 608 | 89 | 51 |
| 844 | Précurseur mitochondrial de la sous-unité alpha de l'ATP synthase | ATPA_HUMAN | 60/54 | 9.2/9.3 | 7/12 | 288 | 75 | 32 |
| 874* | ribonucleoprotéine H nucléaire hétérogène | HNRH1_HUMAN | 49/52 | 5.9/6.4 | 9/12 | 649 | 124 | 40 |
| 910* | Chaîne beta Tubuline | TBB5_HUMAN | 50/50 | 4.8/5.4 | 11/18 | 578 | 91 | 56 |
| 966* | Fructose-bisphosphate aldolase A | ALDOA_HUMAN | 39/50 | 8.3/9.7 | 2/2 | 68 | 50 | 6 |
| 1031* | Précurseur mitochondrial de la sous-unité alpha de l'ATP synthase | ATPA_HUMAN | 60/47 | 9.2/9.5 | 5/6 | 276 | 96 | 15 |
| **1050*** | **Précurseur de Calumenine** | **CALU_HUMAN** | **37/46** | **4.5/5.0** | **2/2** | **51** | **35** | **5** |
| 1113 | Reticulocalbine-3 | RCN3_HUMAN | 37/43 | 4.7/5.1 | 4/9 | 147 | 60 | 49 |
| 1165 | Sous-unité 13 régulatrice non-ATPase du protéasome 26S | PSD13_HUMAN | 43/40 | 5.5/6.3 | 4/6 | 59 | 27 | 18 |
| 1328 | pyrophosphatase inorganique | IPYR_HUMAN | 33/34 | 5.5/6 | 9/11 | 359 | 82 | 47 |
| 1359 | Annexine A5 | ANXA5_HUMAN | 36/33 | 4.9/5.3 | 10/12 | 538 | 86 | 52 |
| 1480 | 14-3-3 protéine epsilon | 1433E_HUMAN | 29/28 | 4.6/5.2 | 4/4 | 276 | 115 | 26 |
| 1514* | 6-phosphogluconolactonase | 6PGL_HUMAN | 28/26 | 5.7/6.1 | 6/7 | 214 | 62 | 36 |
| 1860* | Galectine-1 | LEG1_HUMAN | 15/15 | 5.3/5.3 | 4/4 | 156 | 49 | 34 |
| 2137 | Caldesmone | CALD1_HUMAN | 93/75 | 5.6/6.9 | 2/2 | 113 | 69 | 4 |
| 2151* | Précurseur mitochondrial de la Succinyl-CoA:3-ketoacid-coenzyme A transferase 1 | SCOT_HUMAN | 56/60 | 7.1/6.8 | 5/6 | 200 | 68 | 21 |
| 2161* | ribonucléoprotéine D0 nucléaire hétérogène | HNRPD HUMAN | 38/46 | 7.6/9.3 | 3/3 | 158 | 77 | 11 |

| | | | | | | | | |
|---|---|---|---|---|---|---|---|---|
| ^{#} MSMS et MS+MSMS | | | | | | | | |

Tous les spots dans ce tableau sont reconnus par les patients atteints de granulomatose de Wegener et pas par les sujets sains, les spots avec une étoile sont reconnus spécifiquement par les patients atteints de granulomatose de Wegener et pas par les patients atteints d'autres vascularites. Les antigènes en gras sont reconnus par les IgG sériques de plus de 60% des pools de 3 serums de patients atteints de granulomatose de Wegener

**Tableau 2. Antigènes cibles des AECA chez les patients ayant une polyangéite microscopique avec ANCA anti-MPO**

| | | | | | Spectométrie de masse | | | |
|---|---|---|---|---|---|---|---|---|
| N° sur le gel | Protéine | Numéro d'accès SwissProt | PM théorique / estimé (kDa) | PI théorique / estimé | Nombre de peptides identifies uniques^{#} | score ion total | Meilleur score ion | Couverture de la sequence (%) |
| 851 | Sous-unité 7 régulatrice de la protéase 26S | PRS1_HUMAN | 49/50 | 5.7/6.3 | 10/14 | 306 | 68 | 36 |
| 2077 | Heme oxygénase 2 | HMOX2_HUMAN | 36/36 | 5.3/5.8 | 2/7 | 77 | 54 | 35 |
| 2088 | Histone H2B type F-S | H2BFS_HUMAN | 14/22 | 10.4/5.6 | 2/4 | 77 | 60 | 33 |
| 2128 | sous-unité alpha type-5 du Protéasome | PSA5_HUMAN | 26/34 | 4.7/5.7 | 4/5 | 210 | 91 | 24 |
| 2143 | sous-unité beta type-2 du Protéasome | PSB2_HUMAN | 23 /32 | 6.5/7.1 | 5/5 | 236 | 68 | 32 |

**Tableau 3. Antigènes cibles des AECA chez les patients ayant un syndrome de Churg et Strauss avec ANCA anti-MPO**

| | | | | | Spectométrie de masse | | | |
|---|---|---|---|---|---|---|---|---|
| N° sur le gel | Protéine | Numéro d'accès SwissProt | PM théorique / estimé (kDa) | PI théorique / estimé | Nombre de peptides identifies uniques^{#} | score ion total | Meilleur score ion | Couverture de la séquence (%) |
| 671 | Protéine 4 associée au cytosquelette | CKAP4_HUMAN | 5.6/5.2 | 66/67 | 3/6 | 80 | 32 | 12 |
| 1123 | Uroporphyrinogen decarboxylase | DCUP_HUMAN | 41/43 | 5.8/5.7 | 3/3 | 45 | 16 | 7 |
| 1669 | Adénine phosphoribosyltransferase | APT_HUMAN | 20/20 | 5.8/5.9 | 6/6 | 251 | 62 | 55 |
| 1836 | Profiline-1 | PROF1_HUMAN | 15/15 | 8.4/8.3 | 5/9 | 284 | 78 | 72 |
| 2083 | Plastine-3 | PLST_HUMAN | 70/73 | 5.5/6.3 | 9/17 | 388 | 91 | 34 |
| 2146 | Growth factor receptor-bound protein 2 protéine 2 liée au récepteur du facteur de croissance | GRB2_HUMAN | 25/25 | 5.9/6.4 | 5/7 | 104 | 37 | 32 |
| 2152 | ribonucléoprotéine L nucléaire hétérogène | HNRPL_HUMAN | 64/69 | 8.5/7.3 | 4/10 | 52 | 19 | 24 |

**Tableau 4. Antigènes cibles des AECA chez les patients ayant un syndrome de Churg et Strauss sans ANCA anti-MPO**

| | | | | | Spectrométrie de masse | | | |
|---|---|---|---|---|---|---|---|---|
| N° sur le gel | Protéine | Numéro d'accès SwissProt | PM théorique / estimé (kDa) | PI théorique / estimé | Nombre de peptides identifies uniques^{#} | score ion total | Meilleur score ion | Couverture de la séquence (%) |
| 370 | Far upstream element-binding protein 2 | FUBP2_HUMAN | 73/91 | 6.6/7.1 | 4/8 | 151 | 64 | 16 |
| 382 | Far upstream element-binding protein 2 | FUBP2_HUMAN | 73/91 | 6.8/7.1 | 6/9 | 174 | 59 | 16 |
| 382 | Far upstream element-binding protein 2 | FUBP2_HUMAN | 73/92 | 6.8/7.3 | 6/9 | 174 | 59 | 16 |
| 387 | Far upstream element-binding protein 2 | FUBP2_HUMAN | 73/91 | 6.8/7.1 | 2/9 | 90 | 53 | 17 |
| 387 | Far upstream element-binding protein 2 | FUBP2_HUMAN | 73/91 | 6.8/7.1 | 2/9 | 90 | 53 | 17 |
| 1049 | Précurseur de la Reticulocalbin-1 | RCN1_HUMAN | 39/46 | 4.9/4.4 | 5/8 | 143 | 98 | 30 |
| **1077** | **Précurseur de la Calumenine** | **CALU_HUMAN** | **37/45** | **4.5/4.4** | **6/10** | **148** | **32** | **41** |
| 1105 | Serpine B9 | SPB9_HUMAN | 42/43 | 5.6/6.2 | 6/12 | 182 | 64 | 31 |
| 1281 | Précuseur mitochondrial de la sous-unité alpha de la Isocitrate dehydrogenase [NAD] | IDH3A_HUMAN | 40/37 | 6.5/6.1 | 3/5 | 119 | 59 | 15 |
| 1808 | Profiline-1 | PROF1_HUMAN | 15/15 | 8.4/8.9 | 2/3 | 39 | 23 | 27 |
| 2108 | GMP synthase [hydrolysant la glutamine] | GUAA_HUMAN | 77/75 | 6.4/7.1 | 7/11 | 172 | 45 | 23 |
| 2132 | Sous-unité zeta de la protein 1 du complexe T | TCPZ_HUMAN | 58/88 | 6.2/7.5 | 2/3 | 39 | 20 | 6 |
| **2137** | **Caldesmone** | **CALD1_HUMAN** | **93/75** | **5.6/6.9** | **2/2** | **113** | **69** | **4** |
| 2141 | Cofiline-1 | COF1_HUMAN | 18/17 | 8.2/8.2 | 4/6 | 242 | 68 | 40 |

| | | | | | | | | |
|---|---|---|---|---|---|---|---|---|
| * les antigènes en gras sont également reconnus par les IgG sériques de plus de 60% des pools de 3 serums de patients atteints de granulomatose de Wegener | | | | | | | | |

**Tableau 5. Antigènes cibles des AECA chez les patients ayant une polyangéite microscopique sans ANCA anti-MPO**

| | | | | | Spectrométrie de masse | | | |
|---|---|---|---|---|---|---|---|---|
| N° sur le gel | Protéine | Numéro d'accès SwissProt | PM théorique / estimé (kDa) | PI théorique / estimé | Nombre de peptides identifies uniques^{#} | score ion total | Meilleur score ion | Couverture de la séquence (%) |
| 359 | Précurseur mitochondrial de l'aconitate hydratase | ACON_HUMAN | 85/96 | 7.4/7.6 | 10/18 | 385 | 56 | 31 |
| 457 | Protéine de la membrane interne des mitochondries | IMMT_HUMAN | 84/81 | 6.1/7.4 | 4/8 | 85 | 40 | 14 |
| 497 | Heat shock cognate 71 kDa protein | HSP7C_HUMAN | 71/76 | 5.4/6.2 | 4/9 | 68 | 31 | 20 |
| 741 | ribonucleoprotéine K nucléaire hétérogène | HNRPK_HUMAN | 51/62 | 5.4/6.2 | 7/14 | 298 | 75 | 39 |
| 994 | Précurseur mitochondrial du facteur d'élongation Tu | EFTU_HUMAN | 50/50 | 7.3/7.2 | 6/8 | 218 | 60 | 25 |
| 1033 | Alcohol dehydrogenase [NADP+] | AK1A1_HUMAN | 37/47 | 6.3/6.0 | 5/5 | 175 | 66 | 18 |
| 1156 | Alcohol dehydrogenase [NADP+] | AK1A1_HUMAN | 37/41 | 6.3/7.0 | 4/4 | 125 | 54 | 14 |
| 1171 | Sialic acid synthase | SIAS_HUMAN | 40/40 | 6.3/7.1 | 6/7 | 214 | 61 | 31 |
| 1391 | S-formylglutathione hydrolase | ESTD_HUMAN | 31/31 | 6.5/6.9 | 6 | 167 | 53 | 39 |
| 1394 | sous-unité bete-2-like 1 de la protéine de liaison au nucleotide guanine | GBLP_HUMAN | 35/31 | 7.6/5.3 | 2/2 | 94 | 67 | 5 |
| 1398 | Purine nucleoside phosphorylase | PNPH_HUMAN | 32/31 | 6.5/6.6 | 6/7 | 363 | 106 | 32 |
| 1439 | Prohibitine | PHB_HUMAN | 30/29 | 5.6/6 | 9/13 | 451 | 75 | 64 |
| 1505 | Précurseur mitochondrial de la protéine de liaison au C1q | C1QBP_HUMAN | 31/27 | 4.7/6.4 | 3/4 | 99 | 45 | 29 |
| 2130 | ATPase du réticulum endoplasmique transitionnel | TERA_HUMAN | 89/75 | 5.1/6.1 | 5/12 | 144 | 57 | 20 |
| 2162 | Nucleoside diphosphate kinase A | NDKA_HUMAN | 17/16 | 5.8/6.2 | 4/5 | 170 | 81 | 36 |

| | | | | | | | | |
|---|---|---|---|---|---|---|---|---|
| ^{#} MSMS et MS+MSMS | | | | | | | | |

### Exemple 2 : Caractérisation des cibles antigéniques des anticorps AECA dans la maladie de Horton :

On a recherché les cibles des anticorps AECA dans les sérums de 9 patients ayant une maladie de Horton, de 12 sujets sains, et des pools de sérums de patients atteints de microangiopathie thrombotique (4 pools de trois) ou de vascularites (polyangéite microscopique - 4 pools de trois, maladie de Wegener - 5 pools de trois, et maladie de Churg et Strauss- 3 pools de trois).

### Les réactivités IgG sériques étaient analysées à l'aide de gels d'électrophorèse à deux dimensions suivis d'immunoblot utilisation des antigènes de cellules endothéliales d'HUVEC, comme décrit à l'exemple 1.

Les IgG sériques de patients atteints de maladie de Horton reconnaissaient 162±3 taches protéiques au sein d'extraits d'HUVEC, tandis que ceux des sujets sains reconnaissaient 79 taches protéiques. 28 taches protéiques étaient reconnues par au moins 2/3 des pools de malades atteints de maladie de Horton et pas par les sujets sains dont 15 ont été identifiées. 26 taches protéiques d'HUVEC étaient reconnues par au moins un pool de sérums de patients atteints de maladie de Horton et non par les sérums de contrôle ni par ceux des sujets sains dont 9 ont été identifiés.

Les résultats détaillés sont présentés dans les tableaux 6 et 7.

**Tableau 6. Spots protéiques reconnus par au moins 2/3 des pools de sérums de patients atteints de maladie de Horton, et non reconnus par les sujets sains.**

| | | | | | Spectrométrie de masse | | | |
|---|---|---|---|---|---|---|---|---|
| N° sur le gel | Protéine | Numéro d'accès SwissProt | PM thérorique/ estimé (kDa) | PI théroriquel/ estimé | Nombre de peptides identifies uniques # | Score ion total | meilleur score ion | Couverture de la séquence (%) |
| 557 | Far upstream element-binding protein 1 | FUBP1_HUMAN | 67/75 | 7.2/7.2 | 3/7 | 114 | 47 | 13 |
| 631 | Lamine-A/C | LMNA_HUMAN | 74/71 | 6.6/6.9 | 6/10 | 184 | 48 | 15 |
| 646 | Lamine-A/C | LMNA_HUMAN | 74/70 | 6.6/7 | 12/28 | 482 | 71 | 46 |
| 784 | Précurseur mitochondrial de la dihydrolipoyl deshydrogénase, | DLDH_HUMAN | 54/59 | 7.6/7.3 | 2/2 | 42 | 22 | 5 |
| 789 | Inosine-5'-monophosphate deshydrogénase 2 | IMDH2_HUMAN | 56/58 | 6.4/7.1 | 4/7 | 169 | 94 | 17 |
| 950 | Précurseur de la Tripeptidyl-peptidase 1 | TPP1_HUMAN | 61/50 | 6/6.4 | 3/5 | 89 | 34 | 15 |
| 1017 | Précurseur mitochondrial de la Fumarate hydratase | FUMH_HUMAN | 55/48 | 8.9/8 | 6/7 | 243 | 71 | 24 |
| 1085 | ribonucléoprotéine D0 nucléaire hétérogène | HNRPD_HUMAN | 38/43 | 7.6/7.8 | 3/3 | 122 | 69 | 11 |
| 1214 | PDZ et LIM domain protéine 1 | PDLI1_HUMAN | 36/37 | 6.6/7.4 | 5/10 | 269 | 62 | 44 |
| 1249 | proteéne ribosomique acide 60S P0 | RLA0_HUMAN | 34/37 | 5.7/6 | 2/5 | 56 | 35 | 21 |
| 1352 | Protéine 2 du canal anion-sélectif voltage-dépendant | VDAC2_HUMAN | 32/33 | 7.5/7.4 | 4/4 | 155 | 75 | 18 |
| 1359 | Annexine A5 | ANXA5_HUMAN | 36/33 | 4.9/5.3 | 10/12 | 538 | 86 | 52 |
| 1614 | Proteine DJ-1 | PARK7_HUMAN | 20/25 | 6.3/6.6 | 5/5 | 202 | 75 | 51 |
| 1734 | Peptidyl-prolyl cis-trans isomérase A | PPIA_HUMAN | 18/18 | 7.7/8 | 3/5 | 78 | 48 | 36 |
| 1817 | précurseur mitochondrial de la Thioredoxin-dependent peroxide reductase. | PRDX3 _HUMAN | 28/15 | 7.7/6.8 | 5/6 | 211 | 61 | 44 |

| | | | | | | | | |
|---|---|---|---|---|---|---|---|---|
| ^{#} nombre de peptides identifies unique lors des recherché MSMS et MS+MSMS | | | | | | | | |

**Tableau 7- Spots protéiques reconnus par au moins 1 pool de sérums de patients atteints de maladie de Horton, non reconnus par les sujets sains, ni par les sujets atteints d'autres vascularites ou de microangiopathies thrombotiques**

| | | | | | Spectrométrie de masse | | | |
|---|---|---|---|---|---|---|---|---|
| N° sur le gel | Protéine | Numéro d'accès SwissProt | PM thérorique / estimé (kDa) | PI thérorique / estimé | Nombre de peptides identifies uniques # | Score ion total | meilleur score ion | Couverture de la séquence (%) |
| 228 | Vinculine | VINC_HUMAN | 124/116 | 5.5/6.6 | 3/13 | 53 | 34 | 15 |
| 407 | Far upstream element-binding protein 2 | FUBP2_HUMAN | 73/89 | 6.8/7.9 | 5/11 | 86 | 33 | 17 |
| 820 | sous-unité beta de la protéine 1 du complexe T | TCPB_HUMAN | 57/55 | 6/6.6 | 11/12 | 421 | 92 | 38 |
| 1115 | Précurseur membre 11 sousfamille B DnaJ homologue | DJB11_HUMAN | 40/43 | 5.8/6.5 | 7/8 | 316 | 116 | 34 |
| 1174 | Glutarédoxine-3 | GLRX3_HUMAN | 37/39 | 5.3/5.9 | 4/5 | 173 | 69 | 18 |
| 1328 | pyrophosphatase inorganique | IPYR_HUMAN | 33/34 | 5.5/6 | 9/11 | 359 | 82 | 47 |
| 1493 | Rho GDP-dissociation inhibiteur 2 | GDIR2_HUMAN | 23/27 | 5.1/5.7 | 5/10 | 246 | 77 | 58 |
| 1607 | Glutathione S-transferase P | GSTP1_HUMAN | 23/25 | 5.4/6 | 8/10 | 602 | 117 | 63 |
| 1633 | Peroxirédoxine-2 | PRDX2 HUMAN | 22/23 | 5.7/6 | 6/10 | 367 | 95 | 50 |

| | | | | | | | | |
|---|---|---|---|---|---|---|---|---|
| ^{#} nombre de peptides identifies unique lors des recherché MSMS et MS+MSMS | | | | | | | | |

### Exemple 3 : Caractérisation des cibles antigéniques des anticorps anti-cellules musculaires lisses vasculaires dans la maladie de Horton et des vascularites associées

Les sérums de 15 patients atteints d'une maladie de Horton (MH) et de 33 patients atteints d'une vascularite associées aux ANCA (15 ayant une granulomatose de Wegener GW, 9 une polyangéite microscopique MPA, 9 un syndrome de Churg et Strauss CSS), ont été testés par pools de trois et comparés à un pool de sérums de 12 sujets sains.

Les réactivités IgG sériques étaient analysées à l'aide de gels d'électrophorèse à deux dimensions suivis d'immunoblot, pratiquement comme décrit à l'exemple 1, mais en utilisant des antigènes de cellules musculaires lisses vasculaires (CMLV) immortalisées issues d'artère mammère.

Les IgG sériques des pools de trois patients atteints de maladie de Horton (n=5), de GW avec ANCA anti-protéinase 3 (PR3) (n=5), MPA sans ou avec anti-myeloperoxydase (n=3), CSS sans ou avec ANCA anti-MPO (n=3) reconnaissaient 89±28, 94±34, 56±12, 42±16, spots protéiques, respectivement. Plusieurs antigènes étaient reconnus de façon spécifique par au moins 60% des groupes de patients et d'autres antigènes étaient reconnus de façon plus intense par les patients que par les sujets sains.

Les résultats détaillés sont présentés dans les Tableaux 8 et 9.

**Tableau 8 : Antigènes reconnus spécifiquement par les patients.**

| Protéine | Numéro SwissProt | PM thérorique / estimé (kDa) | PI thérorique/ estimé | Nombre de peptides identifies uniques # | Score ion total | meilleur score ion | Couverture de la séquence (%) |
|---|---|---|---|---|---|---|---|
| vinculine | VINC_HUMAN | 122/124 | 5,5/6,4 | 14 | 39 | | |
| protéine putative heat shock HSP 90-alpha A2 | HS902_HUMAN | 39/94 | 4.6/5.6 | 5 | 40 | | |
| Far upstream element-binding protein 2 | FUBP2_HUMAN | 73/88 | 6,8/7,2 | 4/10-9 | 67-46 | 24 | 21 |
| Far upstream element-binding protein 2 | FUBP2_HUMAN | 73/88 | 6,8/7,2 | 5/12-11 | 84-119 | 24 | 25 |
| Far upstream element-binding protein 2 | 3FUBP2_HUMAN | 73/88 | 6.8/7.8 | 11 | 85 | | |
| Lamine-A/C | LMNA_HUMAN | 74/67 | 6.6/6.5 | 10 | 24 | | |
| Coatomer sous-unité alpha | COPA_HUMAN | 138/67 | 7.7/6.5 | 3 | 37 | | |
| UDP-glucose 6-dehydrogenase | UGDH_HUMAN | 55/66 | 6.6/6.7 | 4 | 46 | | |
| Protéine disulfure-isomérase A3 | PDIA3_HUMAN | 57/59 | 6.7/7.5 | 12 et 16 | 528-804 | | |
| Protéine disulfure-isomérase A3 | PDIA3_HUMAN | 57/59 | 6.0/6.3 | 14-13 | 460-362 | | |
| sous-unité beta de la protéine 1 du complexe T | TCPB_HUMAN | 57/57 | 6.0/6.5 | 14-14 | 503-519 | | |
| Actine, cytoplasmique 1 | ACTB_HUMAN | 42/47 | 5,3/5,7 | 5/10-9 | 390-418 | 131 | 53 |
| membre E POTE ankyrin domain family | POTEE_HUMAN | 121/47 | 5,8/5,7 | 6 | 251 | | |
| Nucleophosmine | NPM_HUMAN | 33/39 | 4,6/5,1 | 3/5-5 | 83-203 | 39 | 24 |
| Annexine A2 | ANXA2_HUMAN | 39/35 | 7.6/8.0 | 13 et 10 | 650-265 | | |

| | | | | | | | |
|---|---|---|---|---|---|---|---|
| * nombre de peptides identifies unique lors des recherché MSMS et MS+MSMS | | | | | | | |

**Tableau 9 : Antigènes reconnus plus Intensément par les patients que par les sujets sains.**

| Protéine | Numéro SwissProt | PM thérorique / estimé (kDa) | PI thérorique/ estimé | Nombre de peptides identifies uniques # | Score ion total | meilleur score ion | Couverture de la séquence (%) |
|---|---|---|---|---|---|---|---|
| Facteur d'élongation 2 | EF2_HUMAN | 95/101 | 6.4/7.4 | 16 | 198 | | |
| Caldesmon | CALD1_HUMAN | 93/84 | 5,6/6,7 | 3/6-8 | 72-288 | 39 | 9 |
| Inosine-5'-monophosphate deshydrogénase 2 | IMDH2_HUMAN | 56/60 | 6.4/7.1 | 14 | 474 | | |
| Alpha-énolase | ENOA_HUMAN | 47/57 | 7.0/7,7 | 7/12-7 | 245-66 | 72 | 44 |
| Member 1 sous-famille A, DnaJ homolog | DNJA1_HUMAN | 45/49 | 6.7/7.4 | 9 | 58 | | |
| Actine, cytoplasmique 2 | ACTG_HUMAN | 42/45 | 5,3/5,5 | 5/7-13 | 258-430 | 116 | 34 |
| sous-unité 8 régulatrice de la protéase 26S | PRS8_HUMAN | 46/46 | 7.1/7.6 | 15 | 180 | | |

| | | | | | | | |
|---|---|---|---|---|---|---|---|
| ^{#} nombre de peptides identifies unique lors des recherché MSMS et MS+MSMS | | | | | | | |

## Revendications

1. Procédé *in vitro* de détection d'une granulomatose de Wegener chez un sujet, comprenant la détermination de la présence et/ou de la quantité d'au moins un anticorps anti-cellules endothéliales (AECA) ou anti-cellules musculaires lisses vasculaires (CMLV), qui est un anticorps anti-vinculine, dans un échantillon biologique provenant d'un patient, la présence de l'anticorps anti-vinculine étant indicatrice d'une granulomatose de Wegener.

2. Procédé selon la revendication 1, de détection d'une granulomatose de Wegener, comprenant en outre la détermination de la présence et/ou de la quantité d'au moins un autre anticorps anti-cellules endothéliales (AECA) ou anti-cellules musculaires lisses vasculaires (CMLV), ledit autre anticorps étant dirigé contre un antigène choisi parmi le groupe constitué de FUbp2 (Far upstream element-binding protein 2), Caldesmone , protéine précurseur de 78kDa régulée par le glucose, Heat shock cognate 71 kDa protein, Précurseur mitochondrial de la protéine de Stress-70, Lamine-A/C, ribonucléoprotéine K nucléaire hétérogène, sous-unité epsilon de la protéine 1 du complexe T, Précurseur mitochondrial de la protéine 60 kDa heat shock, Précurseur protéique de la disulfure-isomérase A1, Précurseur protéique de la disulfure-isomérase A3, sous-unité theta de la protéine 1 du complexe T, sous-unité beta de la protéine 1 du complexe T, Précurseur mitochondrial de la sous-unité alpha de l'ATP synthase,ribonucleoprotéine H nucléaire hétérogène, Chaîne bêta Tubuline, Fructose-bisphosphate aldolase A, Précurseur mitochondrial de la sous-unité alpha de l'ATP synthase, Précurseur de Calumenine, Reticulocalbine-3, Sous-unité 13 régulatrice non-ATPase du protéasome 26S, pyrophosphatase inorganique, Annexine A5, 14-3-3 protéine epsilon, 6-phosphogluconolactonase, Galectine-1, Précurseur mitochondrial de la Succinyl-CoA:3-ketoacid-coenzyme A transférase 1, et la ribonucléoprotéine D0 nucléaire hétérogène,
dans un échantillon biologique provenant d'un patient, la présence de l'anticorps anti-vinculine et dudit au moins autre anticorps étant indicatrice d'une granulomatose de Wegener.

3. Procédé selon la revendication 1, comprenant en outre la détermination de la présence et/ou de la quantité d'au moins un autre anticorps anti-cellules endothéliales (AECA) ou anti-cellules musculaires lisses vasculaires (CMLV), ledit autre anticorps étant dirigé contre un antigène choisi parmi le groupe constitué de Caldesmone, protéine précurseur de 78kDa régulée par le glucose, Heat shock cognate 71 kDa protein , sous-unité epsilon de la protéine 1 du complexe T, Précurseur protéique de la disulfure-isomérase A3 , et Précurseur de Calumenine.

4. Procédé selon l'une des revendications 1 à 3, dans lequel l'échantillon biologique est un échantillon de sang ou de sérum.

5. Procédé selon l'une des revendications 1 à 4, dans lequel la présence dudit au moins un anticorps dans l'échantillon biologique est comparée à une valeur contrôle, la présence dudit au moins un anticorps en une quantité supérieure à la valeur contrôle étant indicatrice d'une granulomatose de Wegener.

6. Procédé selon l'une des revendications 1 à 5, dans lequel la quantité dudit au moins un anticorps est déterminée par un immunoessai.

7. Procédé selon la revendication 6, dans lequel l'immunoessai est un dosage ELISA.

8. Procédé selon l'une des revendications 1 à 7, dans lequel le patient est un humain.

9. Procédé selon l'une des revendications 1 à 8, dans lequel le patient n'a pas d'auto-anticorps ANCA.

## Patentansprüche

1. *In vitro* Verfahren zum Nachweis einer Wegener-Granulomatose bei einer Person, umfassend die Bestimmung des Vorhandenseins und/oder der Menge wenigstens eines Anti-Endothelzell-Antikörpers (AECA) oder Anti-Gefäßglattmuskelzell-Antikörpers (CMLV), der ein Anti-Vinculin-Antikörper ist, in einer von einem Patienten stammenden biologischen Probe, wobei das Vorhandensein des Anti-Vinculin-Antikörpers ein Hinweis auf eine Wegener-Granulomatose ist.

2. Verfahren gemäß Anspruch 1 zum Nachweis einer Wegener-Granulomatose, umfassend außerdem die Bestimmung des Vorhandenseins und/oder der Menge wenigstens eines weiteren Anti-Endothelzell-Antikörpers (AECA) oder Anti-Gefäßglattmuskelzell-Antikörpers (CMLV), wobei besagter weiterer Antikörper gegen ein Antigen gerichtet ist, das aus der Gruppe ausgewählt ist, bestehend aus FUbp2 (Far upstream element-binding protein 2), Caldesmon, von Glucose reguliertem 78 kDa Vorläuferprotein, Hitzeschock cognate 71 kDa Protein, mitochondrialem Vorläufer des Stress-70-Proteins, Lamin-A/C, heterogenem nukleärem Ribonukleoprotein K, T-Komplex Protein 1 epsilon-Untereinheit, mitochondrialem Vorläufer des 60 kDa Hitzeschockproteins, Proteinvorläufer der Disulfid-Isomerase A1, Proteinvorläufer der Disulfid-Isomerase A3, T-Komplex Protein 1 theta-Untereinheit, T-Komplex Protein 1 beta-Untereinheit, mitochondrialem Vorläufer der alpha-Untereinheit der ATP-Synthase, heterogenem nukleärem Ribonukleoprotein H, beta-Tubulinkette, Fructosebisphosphat-Aldolase A, mitochondrialem Vorläufer der alpha-Untereinheit der ATP-Synthase, Calumenin-Vorläufer, Reticulocalbin-3, regulatorischer Nicht-ATPase-Untereinheit 13 des 26 S Proteasoms, anorganischer Pyrophosphatase, Annexin A5, 14-3-3 Protein epsilon, 6-Phosphogluconolactonase, Galectin-1, mitochondrialem Vorläufer der Succinyl-CoA:3-ketosäure-Coenzym A-Transferase 1 und heterogenem nukleärem Ribonukleoprotein D0, in einer von einem Patienten stammenden biologischen Probe, wobei das Vorhandensein des Anti-Vinculin-Antikörpers und des besagten wenigstens anderen Antikörpers ein Hinweis auf eine Wegener-Granulomatose ist.

3. Verfahren gemäß Anspruch 1, umfassend außerdem die Bestimmung des Vorhandenseins und/oder der Menge wenigstens eines weiteren Anti-Endothelzell-Antikörpers (AECA) oder Anti-Gefäßglattmuskelzell-Antikörpers (CMLV), wobei besagter weiterer Antikörper gegen ein Antigen gerichtet ist, ausgewählt aus der Gruppe, bestehend aus Caldesmon, von Glucose reguliertem 78 kDa Vorläufer-Protein, Hitzeschock cognate 71 kDa Protein, T-Komplex Protein 1 epsilon-Untereinheit, Proteinvorläufer der Disulfid-Isomerase A3 und Calumenin-Vorläufer.

4. Verfahren gemäß einem der Ansprüche 1 bis 3, wobei die biologische Probe eine Blut- oder Serumprobe ist.

5. Verfahren gemäß einem der Ansprüche 1 bis 4, wobei das Vorhandensein des besagten wenigstens einen Antikörpers in der biologischen Probe mit einem Kontrollwert verglichen wird, wobei das Vorhandensein des besagten wenigstens einen Antikörpers in einer den Kontrollwert übersteigenden Menge ein Hinweis auf eine Wegener-Granulomatose ist.

6. Verfahren gemäß einem der Ansprüche 1 bis 5, wobei die Menge des besagten wenigstens einen Antikörpers mit einem Immunassay bestimmt wird.

7. Verfahren gemäß Anspruch 6, wobei der Immunassay eine ELISA-Messung ist.

8. Verfahren gemäß einem der Ansprüche 1 bis 7, wobei der Patient ein Mensch ist.

9. Verfahren gemäß einem der Ansprüche 1 bis 8, wobei der Patient keine ANCA-Autoantikörper aufweist.

## Claims

1. An *in vitro* method for detecting Wegener's granulomatosis in a subject, comprising determining the presence and/or amount of at least one anti-endothelial cells antibody (AECA) or anti-vascular smooth muscle cell (VSMC) antibody, which is an anti-vinculin antibody, in a biological sample from a patient, the presence of the anti-vinculin antibody being indicative of Wegener's granulomatosis.

2. The method according to claim 1, for detecting Wegener's granulomatosis, further comprising determining the presence and/or amount of at least another anti-endothelial cells antibody (AECA) or anti-vascular smooth muscular cells antibody (VSMC), said other antibody being directed against an antigen selected from the group consisting of FUbp2 (Far upstream element-binding protein 2), Caldesmon, 78 kDa glucose-regulated precursor protein, heat shock cognate 71 kDa protein, stress protein 70 mitochondrial precursor, lamin-A/C, heterogeneous nuclear ribonucleoprotein K, epsilon subunit of T-complex protein 1, 60 kDa heat shock protein mitochondrial precursor, disulfide isomerase A1 protein precursor, disulfide isomerase A3 protein precursor, theta subunit of T-complex protein 1, beta subunit of T-complex protein 1, mitochondrial precursor of ATP synthase alpha subunit, heterogeneous nuclear ribonucleoprotein H, tubulin beta-chain, fructose-bisphosphate aldolase A, mitochondrial precursor of ATP synthase alpha subunit, calumenin precursor, reticulocalbin-3, 26S proteasome non-ATPase regulatory subunit 13, inorganic pyrophosphatase, annexin A5, 14-3-3 protein epsilon, 6-phosphogluconolactonase, galectin-1, mitochondrial precursor of succinyl-CoA:3-ketoacid-coenzyme A transferase 1 and heterogeneous nuclear ribonucleoprotein D0,
in a biological sample from a patient, the presence of the anti-vinculin antibody and said at least one other antibody being indicative of Wegener's granulomatosis.

3. The method according to claim 1, further comprising determining the presence and/or amount of at least another anti-endothelial cells antibody (AECA) or anti-vascular smooth muscular cells (VSMC), said other antibody being directed against an antigen selected into the group consisting of caldesmon, 78 kDa glucose-regulated precursor protein, heat shock cognate 71 kDa protein, epsilon subunit of T complex protein 1, disulfide isomerase A3 protein precursor and calumenin precursor.

4. The method according to one of claims 1 to 3, wherein the biological sample is a blood or serum sample.

5. The method according to one of claims 1 to 4, wherein the presence of said at least one antibody in the biological sample is compared to a control value, the presence of said at least one antibody in an amount greater than the control value being indicative of Wegener's granulomatosis.

6. The method according to one of claims 1 to 5, wherein the amount of said at least one antibody is determined by an immunoassay.

7. The method according to claim 6, wherein the immunoassay is an ELISA assay.

8. The method according to one of claims 1 to 7, wherein the patient is a human.

9. The method according to one of claims 1 to 8, wherein the patient does not have ANCA auto-antibodies.


### SEQUENCE LISTING

<110> Assistance Publique - Hôpitaux de Paris
<120> Procédé de diagnostic d'une vascularite
<130> B845
<160> 88
<170> PatentIn version 3.3
<210> 1
   <211> 1134
   <212> PRT
   <213> Homo sapiens
<220>
   <221> MISC_FEATURE
   <223> Vinculine
<400> 1
<210> 2
   <211> 710
   <212> PRT
   <213> Homo sapiens
<220>
   <221> MISC_FEATURE
   <223> Far upstream element-binding protein 2
<400> 2
   Met Ser Asp Tyr Ser Thr Gly Gly Pro Pro Pro Gly Pro Pro Pro Pro
   1 5 10 15
   Ala Gly Gly Gly Gly Gly Ala Gly Gly Ala Gly Gly Gly Pro Pro Pro
   20 25 30
   Gly Pro Pro Gly Ala Gly Asp Arg Gly Gly Gly Gly Pro Cys Gly Gly
   35 40 45
   Gly Pro Gly Gly Gly Ser Ala Gly Gly Pro Ser Gln Pro Pro Gly Gly
   50 55 60
   Gly Gly Pro Gly Ile Arg Lys Asp Ala Phe Ala Asp Ala Val Gln Arg
   65 70 75 80
   Ala Arg Gln Ile Ala Ala Lys Ile Gly Gly Asp Ala Ala Thr Thr Val
   85 90 95
   Asn Asn Ser Thr Pro Asp Phe Gly Phe Gly Gly Gln Lys Arg Gln Leu
   100 105 110
   Glu Asp Gly Asp Gln Pro Glu Ser Lys Lys Leu Ala Ser Gln Gly Asp
   115 120 125
   Ser Ile Ser Ser Gln Leu Gly Pro Ile His Pro Pro Pro Arg Thr Ser
   130 135 140
   Met Thr Glu Glu Tyr Arg Val Pro Asp Gly Met Val Gly Leu Ile Ile
   145 150 155 160
   Gly Arg Gly Gly Glu Gln Ile Asn Lys Ile Gln Gln Asp Ser Gly Cys
   165 170 175
   Lys Val Gln Ile Ser Pro Asp Ser Gly Gly Leu Pro Glu Arg Ser Val
               180                 185                 190
   Ser Leu Thr Gly Ala Pro Glu Ser Val Gln Lys Ala Lys Met Met Leu
           195                 200                 205
   Asp Asp Ile Val Ser Arg Gly Arg Gly Gly Pro Pro Gly Gln Phe His
       210                 215                 220
   Asp Asn Ala Asn Gly Gly Gln Asn Gly Thr Val Gln Glu Ile Met Ile
   225                 230                 235                 240
   Pro Ala Gly Lys Ala Gly Leu Val Ile Gly Lys Gly Gly Glu Thr Ile
                   245                 250                 255
   Lys Gln Leu Gln Glu Arg Ala Gly Val Lys Met Ile Leu Ile Gln Asp
               260                 265                 270
   Gly Ser Gln Asn Thr Asn Val Asp Lys Pro Leu Arg Ile Ile Gly Asp
           275                 280                 285
   Pro Tyr Lys Val Gln Gln Ala Cys Glu Met Val Met Asp Ile Leu Arg
       290                 295                 300
   Glu Arg Asp Gln Gly Gly Phe Gly Asp Arg Asn Glu Tyr Gly Ser Arg
   305                 310                 315                 320
   Ile Gly Gly Gly Ile Asp Val Pro Val Pro Arg His Ser Val Gly Val
                   325                 330                 335
   Val Ile Gly Arg Ser Gly Glu Met Ile Lys Lys Ile Gln Asn Asp Ala
               340                 345                 350
   Gly Val Arg Ile Gln Phe Lys Gln Asp Asp Gly Thr Gly Pro Glu Lys
           355                 360                 365
   Ile Ala His Ile Met Gly Pro Pro Asp Arg Cys Glu His Ala Ala Arg
       370                 375                 380
   Ile Ile Asn Asp Leu Leu Gln Ser Leu Arg Ser Gly Pro Pro Gly Pro
   385                 390                 395                 400
   Pro Gly Gly Pro Gly Met Pro Pro Gly Gly Arg Gly Arg Gly Arg Gly
                   405                 410                 415
   Gln Gly Asn Trp Gly Pro Pro Gly Gly Glu Met Thr Phe Ser Ile Pro
               420                 425                 430
   Thr His Lys Cys Gly Leu Val Ile Gly Arg Gly Gly Glu Asn Val Lys
           435                 440                 445
   Ala Ile Asn Gln Gln Thr Gly Ala Phe Val Glu Ile Ser Arg Gln Leu
       450                 455                 460
   Pro Pro Asn Gly Asp Pro Asn Phe Lys Leu Phe Ile Ile Arg Gly Ser
   465                 470                 475                 480
   Pro Gln Gln Ile Asp His Ala Lys Gln Leu Ile Glu Glu Lys Ile Glu
                   485                 490                 495
   Gly Pro Leu Cys Pro Val Gly Pro Gly Pro Gly Gly Pro Gly Pro Ala
               500                 505                 510
   Gly Pro Met Gly Pro Phe Asn Pro Gly Pro Phe Asn Gln Gly Pro Pro
           515                 520                 525
   Gly Ala Pro Pro His Ala Gly Gly Pro Pro Pro His Gln Tyr Pro Pro
       530                 535                 540
   Gln Gly Trp Gly Asn Thr Tyr Pro Gln Trp Gln Pro Pro Ala Pro His
   545                 550                 555                 560
   Asp Pro Ser Lys Ala Ala Ala Ala Ala Ala Asp Pro Asn Ala Ala Trp
                   565                 570                 575
   Ala Ala Tyr Tyr Ser His Tyr Tyr Gln Gln Pro Pro Gly Pro Val Pro
               580                 585                 590
   Gly Pro Ala Pro Ala Pro Ala Ala Pro Pro Ala Gln Gly Glu Pro Pro
           595                 600                 605
   Gln Pro Pro Pro Thr Gly Gln Ser Asp Tyr Thr Lys Ala Trp Glu Glu
       610                 615                 620
   Tyr Tyr Lys Lys Ile Gly Gln Gln Pro Gln Gln Pro Gly Ala Pro Pro
   625                 630                 635                 640
   Gln Gln Asp Tyr Thr Lys Ala Trp Glu Glu Tyr Tyr Lys Lys Gln Ala
                   645                 650                 655
   Gln Val Ala Thr Gly Gly Gly Pro Gly Ala Pro Pro Gly Ser Gln Pro
   660 665 670
   Asp Tyr Ser Ala Ala Trp Ala Glu Tyr Tyr Arg Gln Gln Ala Ala Tyr
   675 680 685
   Tyr Gly Gln Thr Pro Val Pro Gly Pro Gln Pro Pro Pro Thr Gln Gln
   690 695 700
   Gly Gln Gln Gln Ala Gln
   705 710
<210> 3
   <211> 710
   <212> PRT
   <213> Homo sapiens
<220>
   <221> MISC_FEATURE
   <223> Caldesmone
<400> 3
   Met Ser Asp Tyr Ser Thr Gly Gly Pro Pro Pro Gly Pro Pro Pro Pro
   1 5 10 15
   Ala Gly Gly Gly Gly Gly Ala Gly Gly Ala Gly Gly Gly Pro Pro Pro
   20 25 30
   Gly Pro Pro Gly Ala Gly Asp Arg Gly Gly Gly Gly Pro Cys Gly Gly
   35 40 45
   Gly Pro Gly Gly Gly Ser Ala Gly Gly Pro Ser Gln Pro Pro Gly Gly
   50 55 60
   Gly Gly Pro Gly Ile Arg Lys Asp Ala Phe Ala Asp Ala Val Gln Arg
   65 70 75 80
   Ala Arg Gln Ile Ala Ala Lys Ile Gly Gly Asp Ala Ala Thr Thr Val
   85 90 95
   Asn Asn Ser Thr Pro Asp Phe Gly Phe Gly Gly Gln Lys Arg Gln Leu
   100 105 110
   Glu Asp Gly Asp Gln Pro Glu Ser Lys Lys Leu Ala Ser Gln Gly Asp
   115 120 125
   Ser Ile Ser Ser Gln Leu Gly Pro Ile His Pro Pro Pro Arg Thr Ser
   130 135 140
   Met Thr Glu Glu Tyr Arg Val Pro Asp Gly Met Val Gly Leu Ile Ile
   145 150 155 160
   Gly Arg Gly Gly Glu Gln Ile Asn Lys Ile Gln Gln Asp Ser Gly Cys
   165 170 175
   Lys Val Gln Ile Ser Pro Asp Ser Gly Gly Leu Pro Glu Arg Ser Val
   180 185 190
   Ser Leu Thr Gly Ala Pro Glu Ser Val Gln Lys Ala Lys Met Met Leu
   195 200 205
   Asp Asp Ile Val Ser Arg Gly Arg Gly Gly Pro Pro Gly Gln Phe His
   210 215 220
   Asp Asn Ala Asn Gly Gly Gln Asn Gly Thr Val Gln Glu Ile Met Ile
   225 230 235 240
   Pro Ala Gly Lys Ala Gly Leu Val Ile Gly Lys Gly Gly Glu Thr Ile
   245 250 255
   Lys Gln Leu Gln Glu Arg Ala Gly Val Lys Met Ile Leu Ile Gln Asp
   260 265 270
   Gly Ser Gln Asn Thr Asn Val Asp Lys Pro Leu Arg Ile Ile Gly Asp
   275 280 285
   Pro Tyr Lys Val Gln Gln Ala Cys Glu Met Val Met Asp Ile Leu Arg
   290 295 300
   Glu Arg Asp Gln Gly Gly Phe Gly Asp Arg Asn Glu Tyr Gly Ser Arg
   305 310 315 320
   Ile Gly Gly Gly Ile Asp Val Pro Val Pro Arg His Ser Val Gly Val
   325 330 335
   Val Ile Gly Arg Ser Gly Glu Met Ile Lys Lys Ile Gln Asn Asp Ala
   340 345 350
   Gly Val Arg Ile Gln Phe Lys Gln Asp Asp Gly Thr Gly Pro Glu Lys
   355 360 365
   Ile Ala His Ile Met Gly Pro Pro Asp Arg Cys Glu His Ala Ala Arg
   370 375 380
   Ile Ile Asn Asp Leu Leu Gln Ser Leu Arg Ser Gly Pro Pro Gly Pro
   385                 390                 395                 400
   Pro Gly Gly Pro Gly Met Pro Pro Gly Gly Arg Gly Arg Gly Arg Gly
                   405                 410                 415
   Gln Gly Asn Trp Gly Pro Pro Gly Gly Glu Met Thr Phe Ser Ile Pro
               420                 425                 430
   Thr His Lys Cys Gly Leu Val Ile Gly Arg Gly Gly Glu Asn Val Lys
           435                 440                 445
   Ala Ile Asn Gln Gln Thr Gly Ala Phe Val Glu Ile Ser Arg Gln Leu
       450                 455                 460
   Pro Pro Asn Gly Asp Pro Asn Phe Lys Leu Phe Ile Ile Arg Gly Ser
   465                 470                 475                 480
   Pro Gln Gln Ile Asp His Ala Lys Gln Leu Ile Glu Glu Lys Ile Glu
                   485                 490                 495
   Gly Pro Leu Cys Pro Val Gly Pro Gly Pro Gly Gly Pro Gly Pro Ala
               500                 505                 510
   Gly Pro Met Gly Pro Phe Asn Pro Gly Pro Phe Asn Gln Gly Pro Pro
           515                 520                 525
   Gly Ala Pro Pro His Ala Gly Gly Pro Pro Pro His Gln Tyr Pro Pro
       530                 535                 540
   Gln Gly Trp Gly Asn Thr Tyr Pro Gln Trp Gln Pro Pro Ala Pro His
   545                 550                 555                 560
   Asp Pro Ser Lys Ala Ala Ala Ala Ala Ala Asp Pro Asn Ala Ala Trp
                   565                 570                 575
   Ala Ala Tyr Tyr Ser His Tyr Tyr Gln Gln Pro Pro Gly Pro Val Pro
               580                 585                 590
   Gly Pro Ala Pro Ala Pro Ala Ala Pro Pro Ala Gln Gly Glu Pro Pro
           595                 600                 605
   Gln Pro Pro Pro Thr Gly Gln Ser Asp Tyr Thr Lys Ala Trp Glu Glu
       610                 615                 620
   Tyr Tyr Lys Lys Ile Gly Gln Gln Pro Gln Gln Pro Gly Ala Pro Pro
   625 630 635 640
   Gln Gln Asp Tyr Thr Lys Ala Trp Glu Glu Tyr Tyr Lys Lys Gln Ala
   645 650 655
   Gln Val Ala Thr Gly Gly Gly Pro Gly Ala Pro Pro Gly Ser Gln Pro
   660 665 670
   Asp Tyr Ser Ala Ala Trp Ala Glu Tyr Tyr Arg Gln Gln Ala Ala Tyr
   675 680 685
   Tyr Gly Gln Thr Pro Val Pro Gly Pro Gln Pro Pro Pro Thr Gln Gln
   690 695 700
   Gly Gln Gln Gln Ala Gln
   705 710
<210> 4
   <211> 654
   <212> PRT
   <213> Homo sapiens
<220>
   <221> MISC_FEATURE
   <223> 78 kDa glucose-regulated protein precursor
<400> 4
   Met Lys Leu Ser Leu Val Ala Ala Met Leu Leu Leu Leu Ser Ala Ala
   1 5 10 15
   Arg Ala Glu Glu Glu Asp Lys Lys Glu Asp Val Gly Thr Val Val Gly
   20 25 30
   Ile Asp Leu Gly Thr Thr Tyr Ser Cys Val Gly Val Phe Lys Asn Gly
   35 40 45
   Arg Val Glu Ile Ile Ala Asn Asp Gln Gly Asn Arg Ile Thr Pro Ser
   50 55 60
   Tyr Val Ala Phe Thr Pro Glu Gly Glu Arg Leu Ile Gly Asp Ala Ala
   65 70 75 80
   Lys Asn Gln Leu Thr Ser Asn Pro Glu Asn Thr Val Phe Asp Ala Lys
   85 90 95
   Arg Leu Ile Gly Arg Thr Trp Asn Asp Pro Ser Val Gln Gln Asp Ile
               100                 105                 110
   Lys Phe Leu Pro Phe Lys Val Val Glu Lys Lys Thr Lys Pro Tyr Ile
           115                 120                 125
   Gln Val Asp Ile Gly Gly Gly Gln Thr Lys Thr Phe Ala Pro Glu Glu
       130                 135                 140
   Ile Ser Ala Met Val Leu Thr Lys Met Lys Glu Thr Ala Glu Ala Tyr
   145                 150                 155                 160
   Leu Gly Lys Lys Val Thr His Ala Val Val Thr Val Pro Ala Tyr Phe
                   165                 170                 175
   Asn Asp Ala Gln Arg Gln Ala Thr Lys Asp Ala Gly Thr Ile Ala Gly
               180                 185                 190
   Leu Asn Val Met Arg Ile Ile Asn Glu Pro Thr Ala Ala Ala Ile Ala
           195                 200                 205
   Tyr Gly Leu Asp Lys Arg Glu Gly Glu Lys Asn Ile Leu Val Phe Asp
       210                 215                 220
   Leu Gly Gly Gly Thr Phe Asp Val Ser Leu Leu Thr Ile Asp Asn Gly
   225                 230                 235                 240
   Val Phe Glu Val Val Ala Thr Asn Gly Asp Thr His Leu Gly Gly Glu
                   245                 250                 255
   Asp Phe Asp Gln Arg Val Met Glu His Phe Ile Lys Leu Tyr Lys Lys
               260                 265                 270
   Lys Thr Gly Lys Asp Val Arg Lys Asp Asn Arg Ala Val Gln Lys Leu
           275                 280                 285
   Arg Arg Glu Val Glu Lys Ala Lys Arg Ala Leu Ser Ser Gln His Gln
       290                 295                 300
   Ala Arg Ile Glu Ile Glu Ser Phe Tyr Glu Gly Glu Asp Phe Ser Glu
   305                 310                 315                 320
   Thr Leu Thr Arg Ala Lys Phe Glu Glu Leu Asn Met Asp Leu Phe Arg
                   325                 330                 335
   Ser Thr Met Lys Pro Val Gln Lys Val Leu Glu Asp Ser Asp Leu Lys
               340                 345                 350
   Lys Ser Asp Ile Asp Glu Ile Val Leu Val Gly Gly Ser Thr Arg Ile
           355                 360                 365
   Pro Lys Ile Gln Gln Leu Val Lys Glu Phe Phe Asn Gly Lys Glu Pro
       370                 375                 380
   Ser Arg Gly Ile Asn Pro Asp Glu Ala Val Ala Tyr Gly Ala Ala Val
   385                 390                 395                 400
   Gln Ala Gly Val Leu Ser Gly Asp Gln Asp Thr Gly Asp Leu Val Leu
                   405                 410                 415
   Leu Asp Val Cys Pro Leu Thr Leu Gly Ile Glu Thr Val Gly Gly Val
               420                 425                 430
   Met Thr Lys Leu Ile Pro Arg Asn Thr Val Val Pro Thr Lys Lys Ser
           435                 440                 445
   Gln Ile Phe Ser Thr Ala Ser Asp Asn Gln Pro Thr Val Thr Ile Lys
       450                 455                 460
   Val Tyr Glu Gly Glu Arg Pro Leu Thr Lys Asp Asn His Leu Leu Gly
   465                 470                 475                 480
   Thr Phe Asp Leu Thr Gly Ile Pro Pro Ala Pro Arg Gly Val Pro Gln
                   485                 490                 495
   Ile Glu Val Thr Phe Glu Ile Asp Val Asn Gly Ile Leu Arg Val Thr
               500                 505                 510
   Ala Glu Asp Lys Gly Thr Gly Asn Lys Asn Lys Ile Thr Ile Thr Asn
           515                 520                 525
   Asp Gln Asn Arg Leu Thr Pro Glu Glu Ile Glu Arg Met Val Asn Asp
       530                 535                 540
   Ala Glu Lys Phe Ala Glu Glu Asp Lys Lys Leu Lys Glu Arg Ile Asp
   545                 550                 555                 560
   Thr Arg Asn Glu Leu Glu Ser Tyr Ala Tyr Ser Leu Lys Asn Gln Ile
                   565                 570                 575
   Gly Asp Lys Glu Lys Leu Gly Gly Lys Leu Ser Ser Glu Asp Lys Glu
               580                 585                 590
   Thr Met Glu Lys Ala Val Glu Glu Lys Ile Glu Trp Leu Glu Ser His
   595 600 605
   Gln Asp Ala Asp Ile Glu Asp Phe Lys Ala Lys Lys Lys Glu Leu Glu
   610 615 620
   Glu Ile Val Gln Pro Ile Ile Ser Lys Leu Tyr Gly Ser Ala Gly Pro
   625 630 635 640
   Pro Pro Thr Gly Glu Glu Asp Thr Ala Glu Lys Asp Glu Leu
   645 650
<210> 5
   <211> 646
   <212> PRT
   <213> Homo sapiens
<220>
   <221> MISC_FEATURE
   <223> Heat shock cognate 71 kDa protein
<400> 5
   Met Ser Lys Gly Pro Ala Val Gly Ile Asp Leu Gly Thr Thr Tyr Ser
   1 5 10 15
   Cys Val Gly Val Phe Gln His Gly Lys Val Glu Ile Ile Ala Asn Asp
   20 25 30
   Gln Gly Asn Arg Thr Thr Pro Ser Tyr Val Ala Phe Thr Asp Thr Glu
   35 40 45
   Arg Leu Ile Gly Asp Ala Ala Lys Asn Gln Val Ala Met Asn Pro Thr
   50 55 60
   Asn Thr Val Phe Asp Ala Lys Arg Leu Ile Gly Arg Arg Phe Asp Asp
   65 70 75 80
   Ala Val Val Gln Ser Asp Met Lys His Trp Pro Phe Met Val Val Asn
   85 90 95
   Asp Ala Gly Arg Pro Lys Val Gln Val Glu Tyr Lys Gly Glu Thr Lys
   100 105 110
   Ser Phe Tyr Pro Glu Glu Val Ser Ser Met Val Leu Thr Lys Met Lys
   115 120 125
   Glu Ile Ala Glu Ala Tyr Leu Gly Lys Thr Val Thr Asn Ala Val Val
   130 135 140
   Thr Val Pro Ala Tyr Phe Asn Asp Ser Gln Arg Gln Ala Thr Lys Asp
   145 150 155 160
   Ala Gly Thr Ile Ala Gly Leu Asn Val Leu Arg Ile Ile Asn Glu Pro
   165 170 175
   Thr Ala Ala Ala Ile Ala Tyr Gly Leu Asp Lys Lys Val Gly Ala Glu
   180 185 190
   Arg Asn Val Leu Ile Phe Asp Leu Gly Gly Gly Thr Phe Asp Val Ser
   195 200 205
   Ile Leu Thr Ile Glu Asp Gly Ile Phe Glu Val Lys Ser Thr Ala Gly
   210 215 220
   Asp Thr His Leu Gly Gly Glu Asp Phe Asp Asn Arg Met Val Asn His
   225 230 235 240
   Phe Ile Ala Glu Phe Lys Arg Lys His Lys Lys Asp Ile Ser Glu Asn
   245 250 255
   Lys Arg Ala Val Arg Arg Leu Arg Thr Ala Cys Glu Arg Ala Lys Arg
   260 265 270
   Thr Leu Ser Ser Ser Thr Gln Ala Ser Ile Glu Ile Asp Ser Leu Tyr
   275 280 285
   Glu Gly Ile Asp Phe Tyr Thr Ser Ile Thr Arg Ala Arg Phe Glu Glu
   290 295 300
   Leu Asn Ala Asp Leu Phe Arg Gly Thr Leu Asp Pro Val Glu Lys Ala
   305 310 315 320
   Leu Arg Asp Ala Lys Leu Asp Lys Ser Gln Ile His Asp Ile Val Leu
   325 330 335
   Val Gly Gly Ser Thr Arg Ile Pro Lys Ile Gln Lys Leu Leu Gln Asp
   340 345 350
   Phe Phe Asn Gly Lys Glu Leu Asn Lys Ser Ile Asn Pro Asp Glu Ala
   355 360 365
   Val Ala Tyr Gly Ala Ala Val Gln Ala Ala Ile Leu Ser Gly Asp Lys
   370 375 380
   Ser Glu Asn Val Gln Asp Leu Leu Leu Leu Asp Val Thr Pro Leu Ser
   385 390 395 400
   Leu Gly Ile Glu Thr Ala Gly Gly Val Met Thr Val Leu Ile Lys Arg
   405 410 415
   Asn Thr Thr Ile Pro Thr Lys Gln Thr Gln Thr Phe Thr Thr Tyr Ser
   420 425 430
   Asp Asn Gln Pro Gly Val Leu Ile Gln Val Tyr Glu Gly Glu Arg Ala
   435 440 445
   Met Thr Lys Asp Asn Asn Leu Leu Gly Lys Phe Glu Leu Thr Gly Ile
   450 455 460
   Pro Pro Ala Pro Arg Gly Val Pro Gln Ile Glu Val Thr Phe Asp Ile
   465 470 475 480
   Asp Ala Asn Gly Ile Leu Asn Val Ser Ala Val Asp Lys Ser Thr Gly
   485 490 495
   Lys Glu Asn Lys Ile Thr Ile Thr Asn Asp Lys Gly Arg Leu Ser Lys
   500 505 510
   Glu Asp Ile Glu Arg Met Val Gln Glu Ala Glu Lys Tyr Lys Ala Glu
   515 520 525
   Asp Glu Lys Gln Arg Asp Lys Val Ser Ser Lys Asn Ser Leu Glu Ser
   530 535 540
   Tyr Ala Phe Asn Met Lys Ala Thr Val Glu Asp Glu Lys Leu Gln Gly
   545 550 555 560
   Lys Ile Asn Asp Glu Asp Lys Gln Lys Ile Leu Asp Lys Cys Asn Glu
   565 570 575
   Ile Ile Asn Trp Leu Asp Lys Asn Gln Thr Ala Glu Lys Glu Glu Phe
   580 585 590
   Glu His Gln Gln Lys Glu Leu Glu Lys Val Cys Asn Pro Ile Ile Thr
   595 600 605
   Lys Leu Tyr Gln Ser Ala Gly Gly Met Pro Gly Gly Met Pro Gly Gly
   610 615 620
   Phe Pro Gly Gly Gly Ala Pro Pro Ser Gly Gly Ala Ser Ser Gly Pro
   625 630 635 640
   Thr Ile Glu Glu Val Asp
   645
<210> 6
   <211> 679
   <212> PRT
   <213> Homo sapiens
<220>
   <221> MISC_FEATURE
   <223> Précurseur mitochondrial de la protéine de Stress-70
<400> 6
<210> 7
   <211> 664
   <212> PRT
   <213> Homo sapiens
<220>
   <221> MISC_FEATURE
   <223> Lamine-A/C
<400> 7
   Met Glu Thr Pro Ser Gln Arg Arg Ala Thr Arg Ser Gly Ala Gln Ala
   1 5 10 15
   Ser Ser Thr Pro Leu Ser Pro Thr Arg Ile Thr Arg Leu Gln Glu Lys
   20 25 30
   Glu Asp Leu Gln Glu Leu Asn Asp Arg Leu Ala Val Tyr Ile Asp Arg
   35 40 45
   Val Arg Ser Leu Glu Thr Glu Asn Ala Gly Leu Arg Leu Arg Ile Thr
   50 55 60
   Glu Ser Glu Glu Val Val Ser Arg Glu Val Ser Gly Ile Lys Ala Ala
   65 70 75 80
   Tyr Glu Ala Glu Leu Gly Asp Ala Arg Lys Thr Leu Asp Ser Val Ala
   85 90 95
   Lys Glu Arg Ala Arg Leu Gln Leu Glu Leu Ser Lys Val Arg Glu Glu
   100 105 110
   Phe Lys Glu Leu Lys Ala Arg Asn Thr Lys Lys Glu Gly Asp Leu Ile
   115 120 125
   Ala Ala Gln Ala Arg Leu Lys Asp Leu Glu Ala Leu Leu Asn Ser Lys
   130 135 140
   Glu Ala Ala Leu Ser Thr Ala Leu Ser Glu Lys Arg Thr Leu Glu Gly
   145                 150                 155                 160
   Glu Leu His Asp Leu Arg Gly Gln Val Ala Lys Leu Glu Ala Ala Leu
                   165                 170                 175
   Gly Glu Ala Lys Lys Gln Leu Gln Asp Glu Met Leu Arg Arg Val Asp
               180                 185                 190
   Ala Glu Asn Arg Leu Gln Thr Met Lys Glu Glu Leu Asp Phe Gln Lys
           195                 200                 205
   Asn Ile Tyr Ser Glu Glu Leu Arg Glu Thr Lys Arg Arg His Glu Thr
       210                 215                 220
   Arg Leu Val Glu Ile Asp Asn Gly Lys Gln Arg Glu Phe Glu Ser Arg
   225                 230                 235                 240
   Leu Ala Asp Ala Leu Gln Glu Leu Arg Ala Gln His Glu Asp Gln Val
                   245                 250                 255
   Glu Gln Tyr Lys Lys Glu Leu Glu Lys Thr Tyr Ser Ala Lys Leu Asp
               260                 265                 270
   Asn Ala Arg Gln Ser Ala Glu Arg Asn Ser Asn Leu Val Gly Ala Ala
           275                 280                 285
   His Glu Glu Leu Gln Gln Ser Arg Ile Arg Ile Asp Ser Leu Ser Ala
       290                 295                 300
   Gln Leu Ser Gln Leu Gln Lys Gln Leu Ala Ala Lys Glu Ala Lys Leu
   305                 310                 315                 320
   Arg Asp Leu Glu Asp Ser Leu Ala Arg Glu Arg Asp Thr Ser Arg Arg
                   325                 330                 335
   Leu Leu Ala Glu Lys Glu Arg Glu Met Ala Glu Met Arg Ala Arg Met
               340                 345                 350
   Gln Gln Gln Leu Asp Glu Tyr Gln Glu Leu Leu Asp Ile Lys Leu Ala
           355                 360                 365
   Leu Asp Met Glu Ile His Ala Tyr Arg Lys Leu Leu Glu Gly Glu Glu
       370                 375                 380
   Glu Arg Leu Arg Leu Ser Pro Ser Pro Thr Ser Gln Arg Ser Arg Gly
   385                 390                 395                 400
   Arg Ala Ser Ser His Ser Ser Gln Thr Gln Gly Gly Gly Ser Val Thr
   405 410 415
   Lys Lys Arg Lys Leu Glu Ser Thr Glu Ser Arg Ser Ser Phe Ser Gln
   420 425 430
   His Ala Arg Thr Ser Gly Arg Val Ala Val Glu Glu Val Asp Glu Glu
   435 440 445
   Gly Lys Phe Val Arg Leu Arg Asn Lys Ser Asn Glu Asp Gln Ser Met
   450 455 460
   Gly Asn Trp Gln Ile Lys Arg Gln Asn Gly Asp Asp Pro Leu Leu Thr
   465 470 475 480
   Tyr Arg Phe Pro Pro Lys Phe Thr Leu Lys Ala Gly Gln Val Val Thr
   485 490 495
   Ile Trp Ala Ala Gly Ala Gly Ala Thr His Ser Pro Pro Thr Asp Leu
   500 505 510
   Val Trp Lys Ala Gln Asn Thr Trp Gly Cys Gly Asn Ser Leu Arg Thr
   515 520 525
   Ala Leu Ile Asn Ser Thr Gly Glu Glu Val Ala Met Arg Lys Leu Val
   530 535 540
   Arg Ser Val Thr Val Val Glu Asp Asp Glu Asp Glu Asp Gly Asp Asp
   545 550 555 560
   Leu Leu His His His His Gly Ser His Cys Ser Ser Ser Gly Asp Pro
   565 570 575
   Ala Glu Tyr Asn Leu Arg Ser Arg Thr Val Leu Cys Gly Thr Cys Gly
   580 585 590
   Gln Pro Ala Asp Lys Ala Ser Ala Ser Gly Ser Gly Ala Gln Val Gly
   595 600 605
   Gly Pro Ile Ser Ser Gly Ser Ser Ala Ser Ser Val Thr Val Thr Arg
   610 615 620
   Ser Tyr Arg Ser Val Gly Gly Ser Gly Gly Gly Ser Phe Gly Asp Asn
   625 630 635 640
   Leu Val Thr Arg Ser Tyr Leu Leu Gly Asn Ser Ser Pro Arg Thr Gln
   645 650 655
   Ser Pro Gln Asn Cys Ser Ile Met
   660
<210> 8
   <211> 463
   <212> PRT
   <213> Homo sapiens
<220>
   <221> MISC_FEATURE
   <223> ribonucleoproteine K nucléaire hétérogène
<400> 8
   Met Glu Thr Glu Gln Pro Glu Glu Thr Phe Pro Asn Thr Glu Thr Asn
   1 5 10 15
   Gly Glu Phe Gly Lys Arg Pro Ala Glu Asp Met Glu Glu Glu Gln Ala
   20 25 30
   Phe Lys Arg Ser Arg Asn Thr Asp Glu Met Val Glu Leu Arg Ile Leu
   35 40 45
   Leu Gln Ser Lys Asn Ala Gly Ala Val Ile Gly Lys Gly Gly Lys Asn
   50 55 60
   Ile Lys Ala Leu Arg Thr Asp Tyr Asn Ala Ser Val Ser Val Pro Asp
   65 70 75 80
   Ser Ser Gly Pro Glu Arg Ile Leu Ser Ile Ser Ala Asp Ile Glu Thr
   85 90 95
   Ile Gly Glu Ile Leu Lys Lys Ile Ile Pro Thr Leu Glu Glu Gly Leu
   100 105 110
   Gln Leu Pro Ser Pro Thr Ala Thr Ser Gln Leu Pro Leu Glu Ser Asp
   115 120 125
   Ala Val Glu Cys Leu Asn Tyr Gln His Tyr Lys Gly Ser Asp Phe Asp
   130 135 140
   Cys Glu Leu Arg Leu Leu Ile His Gln Ser Leu Ala Gly Gly Ile Ile
   145 150 155 160
   Gly Val Lys Gly Ala Lys Ile Lys Glu Leu Arg Glu Asn Thr Gln Thr
                   165                 170                 175
   Thr Ile Lys Leu Phe Gln Glu Cys Cys Pro His Ser Thr Asp Arg Val
               180                 185                 190
   Val Leu Ile Gly Gly Lys Pro Asp Arg Val Val Glu Cys Ile Lys Ile
           195                 200                 205
   Ile Leu Asp Leu Ile Ser Glu Ser Pro Ile Lys Gly Arg Ala Gln Pro
       210                 215                 220
   Tyr Asp Pro Asn Phe Tyr Asp Glu Thr Tyr Asp Tyr Gly Gly Phe Thr
   225                 230                 235                 240
   Met Met Phe Asp Asp Arg Arg Gly Arg Pro Val Gly Phe Pro Met Arg
                   245                 250                 255
   Gly Arg Gly Gly Phe Asp Arg Met Pro Pro Gly Arg Gly Gly Arg Pro
               260                 265                 270
   Met Pro Pro Ser Arg Arg Asp Tyr Asp Asp Met Ser Pro Arg Arg Gly
           275                 280                 285
   Pro Pro Pro Pro Pro Pro Gly Arg Gly Gly Arg Gly Gly Ser Arg Ala
       290                 295                 300
   Arg Asn Leu Pro Leu Pro Pro Pro Pro Pro Pro Arg Gly Gly Asp Leu
   305                 310                 315                 320
   Met Ala Tyr Asp Arg Arg Gly Arg Pro Gly Asp Arg Tyr Asp Gly Met
                   325                 330                 335
   Val Gly Phe Ser Ala Asp Glu Thr Trp Asp Ser Ala Ile Asp Thr Trp
               340                 345                 350
   Ser Pro Ser Glu Trp Gln Met Ala Tyr Glu Pro Gln Gly Gly Ser Gly
           355                 360                 365
   Tyr Asp Tyr Ser Tyr Ala Gly Gly Arg Gly Ser Tyr Gly Asp Leu Gly
       370                 375                 380
   Gly Pro Ile Ile Thr Thr Gln Val Thr Ile Pro Lys Asp Leu Ala Gly
   385                 390                 395                 400
   Ser Ile Ile Gly Lys Gly Gly Gln Arg Ile Lys Gln Ile Arg His Glu
   405 410 415
   Ser Gly Ala Ser Ile Lys Ile Asp Glu Pro Leu Glu Gly Ser Glu Asp
   420 425 430
   Arg Ile Ile Thr Ile Thr Gly Thr Gln Asp Gln Ile Gln Asn Ala Gln
   435 440 445
   Tyr Leu Leu Gln Asn Ser Val Lys Gln Tyr Ser Gly Lys Phe Phe
   450 455 460
<210> 9
   <211> 541
   <212> PRT
   <213> Homo sapiens
<220>
   <221> MISC_FEATURE
   <223> sous-unité epsilon de la protéine 1 du complexe T
<400> 9
<210> 10
   <211> 573
   <212> PRT
   <213> Homo sapiens
<220>
   <221> MISC_FEATURE
   <223> Précuseur mitochondrial de la protéine 60 kDa heat shock
<400> 10
<210> 11
   <211> 508
   <212> PRT
   <213> Homo sapiens
<220>
   <221> MISC_FEATURE
   <223> Précuseur protéique de la disulfure-isomerase A1
<400> 11
<210> 12
   <211> 505
   <212> PRT
   <213> Homo sapiens
<220>
   <221> MISC_FEATURE
   <223> Précurseur protéique de la disulfure-isomerase A3
<400> 12
   Met Arg Leu Arg Arg Leu Ala Leu Phe Pro Gly Val Ala Leu Leu Leu
   1 5 10 15
   Ala Ala Ala Arg Leu Ala Ala Ala Ser Asp Val Leu Glu Leu Thr Asp
   20 25 30
   Asp Asn Phe Glu Ser Arg Ile Ser Asp Thr Gly Ser Ala Gly Leu Met
   35 40 45
   Leu Val Glu Phe Phe Ala Pro Trp Cys Gly His Cys Lys Arg Leu Ala
   50 55 60
   Pro Glu Tyr Glu Ala Ala Ala Thr Arg Leu Lys Gly Ile Val Pro Leu
   65 70 75 80
   Ala Lys Val Asp Cys Thr Ala Asn Thr Asn Thr Cys Asn Lys Tyr Gly
   85 90 95
   Val Ser Gly Tyr Pro Thr Leu Lys Ile Phe Arg Asp Gly Glu Glu Ala
   100 105 110
   Gly Ala Tyr Asp Gly Pro Arg Thr Ala Asp Gly Ile Val Ser His Leu
   115 120 125
   Lys Lys Gln Ala Gly Pro Ala Ser Val Pro Leu Arg Thr Glu Glu Glu
   130 135 140
   Phe Lys Lys Phe Ile Ser Asp Lys Asp Ala Ser Ile Val Gly Phe Phe
   145 150 155 160
   Asp Asp Ser Phe Ser Glu Ala His Ser Glu Phe Leu Lys Ala Ala Ser
   165 170 175
   Asn Leu Arg Asp Asn Tyr Arg Phe Ala His Thr Asn Val Glu Ser Leu
   180 185 190
   Val Asn Glu Tyr Asp Asp Asn Gly Glu Gly Ile Ile Leu Phe Arg Pro
   195 200 205
   Ser His Leu Thr Asn Lys Phe Glu Asp Lys Thr Val Ala Tyr Thr Glu
   210 215 220
   Gln Lys Met Thr Ser Gly Lys Ile Lys Lys Phe Ile Gln Glu Asn Ile
   225 230 235 240
   Phe Gly Ile Cys Pro His Met Thr Glu Asp Asn Lys Asp Leu Ile Gln
   245 250 255
   Gly Lys Asp Leu Leu Ile Ala Tyr Tyr Asp Val Asp Tyr Glu Lys Asn
   260 265 270
   Ala Lys Gly Ser Asn Tyr Trp Arg Asn Arg Val Met Met Val Ala Lys
   275 280 285
   Lys Phe Leu Asp Ala Gly His Lys Leu Asn Phe Ala Val Ala Ser Arg
   290 295 300
   Lys Thr Phe Ser His Glu Leu Ser Asp Phe Gly Leu Glu Ser Thr Ala
   305 310 315 320
   Gly Glu Ile Pro Val Val Ala Ile Arg Thr Ala Lys Gly Glu Lys Phe
   325 330 335
   Val Met Gln Glu Glu Phe Ser Arg Asp Gly Lys Ala Leu Glu Arg Phe
   340 345 350
   Leu Gln Asp Tyr Phe Asp Gly Asn Leu Lys Arg Tyr Leu Lys Ser Glu
   355 360 365
   Pro Ile Pro Glu Ser Asn Asp Gly Pro Val Lys Val Val Val Ala Glu
   370 375 380
   Asn Phe Asp Glu Ile Val Asn Asn Glu Asn Lys Asp Val Leu Ile Glu
   385 390 395 400
   Phe Tyr Ala Pro Trp Cys Gly His Cys Lys Asn Leu Glu Pro Lys Tyr
   405 410 415
   Lys Glu Leu Gly Glu Lys Leu Ser Lys Asp Pro Asn Ile Val Ile Ala
   420 425 430
   Lys Met Asp Ala Thr Ala Asn Asp Val Pro Ser Pro Tyr Glu Val Arg
   435 440 445
   Gly Phe Pro Thr Ile Tyr Phe Ser Pro Ala Asn Lys Lys Leu Asn Pro
   450 455 460
   Lys Lys Tyr Glu Gly Gly Arg Glu Leu Ser Asp Phe Ile Ser Tyr Leu
   465 470 475 480
   Gln Arg Glu Ala Thr Asn Pro Pro Val Ile Gln Glu Glu Lys Pro Lys
   485 490 495
   Lys Lys Lys Lys Ala Gln Glu Asp Leu
   500 505
<210> 13
   <211> 548
   <212> PRT
   <213> Homo sapiens
<220>
   <221> MISC_FEATURE
   <223> sous-unité theta de la protéine 1 du complexe T
<400> 13
   Met Ala Leu His Val Pro Lys Ala Pro Gly Phe Ala Gln Met Leu Lys
   1               5                   10                  15
   Glu Gly Ala Lys His Phe Ser Gly Leu Glu Glu Ala Val Tyr Arg Asn
               20                  25                  30
   Ile Gln Ala Cys Lys Glu Leu Ala Gln Thr Thr Arg Thr Ala Tyr Gly
           35                  40                  45
   Pro Asn Gly Met Asn Lys Met Val Ile Asn His Leu Glu Lys Leu Phe
       50                  55                  60
   Val Thr Asn Asp Ala Ala Thr Ile Leu Arg Glu Leu Glu Val Gln His
   65                  70                  75                  80
   Pro Ala Ala Lys Met Ile Val Met Ala Ser His Met Gln Glu Gln Glu
                   85                  90                  95
   Val Gly Asp Gly Thr Asn Phe Val Leu Val Phe Ala Gly Ala Leu Leu
               100                 105                 110
   Glu Leu Ala Glu Glu Leu Leu Arg Ile Gly Leu Ser Val Ser Glu Val
           115                 120                 125
   Ile Glu Gly Tyr Glu Ile Ala Cys Arg Lys Ala His Glu Ile Leu Pro
       130                 135                 140
   Asn Leu Val Cys Cys Ser Ala Lys Asn Leu Arg Asp Ile Asp Glu Val
   145                 150                 155                 160
   Ser Ser Leu Leu Arg Thr Ser Ile Met Ser Lys Gln Tyr Gly Asn Glu
                   165                 170                 175
   Val Phe Leu Ala Lys Leu Ile Ala Gln Ala Cys Val Ser Ile Phe Pro
               180                 185                 190
   Asp Ser Gly His Phe Asn Val Asp Asn Ile Arg Val Cys Lys Ile Leu
           195                 200                 205
   Gly Ser Gly Ile Ser Ser Ser Ser Val Leu His Gly Met Val Phe Lys
       210                 215                 220
   Lys Glu Thr Glu Gly Asp Val Thr Ser Val Lys Asp Ala Lys Ile Ala
   225                 230                 235                 240
   Val Tyr Ser Cys Pro Phe Asp Gly Met Ile Thr Glu Thr Lys Gly Thr
                   245                 250                 255
   Val Leu Ile Lys Thr Ala Glu Glu Leu Met Asn Phe Ser Lys Gly Glu
               260                 265                 270
   Glu Asn Leu Met Asp Ala Gln Val Lys Ala Ile Ala Asp Thr Gly Ala
           275                 280                 285
   Asn Val Val Val Thr Gly Gly Lys Val Ala Asp Met Ala Leu His Tyr
       290                 295                 300
   Ala Asn Lys Tyr Asn Ile Met Leu Val Arg Leu Asn Ser Lys Trp Asp
   305                 310                 315                 320
   Leu Arg Arg Leu Cys Lys Thr Val Gly Ala Thr Ala Leu Pro Arg Leu
                   325                 330                 335
   Thr Pro Pro Val Leu Glu Glu Met Gly His Cys Asp Ser Val Tyr Leu
               340                 345                 350
   Ser Glu Val Gly Asp Thr Gln Val Val Val Phe Lys His Glu Lys Glu
           355                 360                 365
   Asp Gly Ala Ile Ser Thr Ile Val Leu Arg Gly Ser Thr Asp Asn Leu
       370                 375                 380
   Met Asp Asp Ile Glu Arg Ala Val Asp Asp Gly Val Asn Thr Phe Lys
   385                 390                 395                 400
   Val Leu Thr Arg Asp Lys Arg Leu Val Pro Gly Gly Gly Ala Thr Glu
                   405                 410                 415
   Ile Glu Leu Ala Lys Gln Ile Thr Ser Tyr Gly Glu Thr Cys Pro Gly
               420                 425                 430
   Leu Glu Gln Tyr Ala Ile Lys Lys Phe Ala Glu Ala Phe Glu Ala Ile
           435                 440                 445
   Pro Arg Ala Leu Ala Glu Asn Ser Gly Val Lys Ala Asn Glu Val Ile
       450                 455                 460
   Ser Lys Leu Tyr Ala Val His Gln Glu Gly Asn Lys Asn Val Gly Leu
   465                 470                 475                 480
   Asp Ile Glu Ala Glu Val Pro Ala Val Lys Asp Met Leu Glu Ala Gly
   485 490 495
   Ile Leu Asp Thr Tyr Leu Gly Lys Tyr Trp Ala Ile Lys Leu Ala Thr
   500 505 510
   Asn Ala Ala Val Thr Val Leu Arg Val Asp Gln Ile Ile Met Ala Lys
   515 520 525
   Pro Ala Gly Gly Pro Lys Pro Pro Ser Gly Lys Lys Asp Trp Asp Asp
   530 535 540
   Asp Gln Asn Asp
   545
<210> 14
   <211> 535
   <212> PRT
   <213> Homo sapiens
<220>
   <221> MISC_FEATURE
   <223> sous-unité beta de la protéine 1 du complexe T
<400> 14
   Met Ala Ser Leu Ser Leu Ala Pro Val Asn Ile Phe Lys Ala Gly Ala
   1 5 10 15
   Asp Glu Glu Arg Ala Glu Thr Ala Arg Leu Thr Ser Phe Ile Gly Ala
   20 25 30
   Ile Ala Ile Gly Asp Leu Val Lys Ser Thr Leu Gly Pro Lys Gly Met
   35 40 45
   Asp Lys Ile Leu Leu Ser Ser Gly Arg Asp Ala Ser Leu Met Val Thr
   50 55 60
   Asn Asp Gly Ala Thr Ile Leu Lys Asn Ile Gly Val Asp Asn Pro Ala
   65 70 75 80
   Ala Lys Val Leu Val Asp Met Ser Arg Val Gln Asp Asp Glu Val Gly
   85 90 95
   Asp Gly Thr Thr Ser Val Thr Val Leu Ala Ala Glu Leu Leu Arg Glu
   100 105 110
   Ala Glu Ser Leu Ile Ala Lys Lys Ile His Pro Gln Thr Ile Ile Ala
   115 120 125
   Gly Trp Arg Glu Ala Thr Lys Ala Ala Arg Glu Ala Leu Leu Ser Ser
   130 135 140
   Ala Val Asp His Gly Ser Asp Glu Val Lys Phe Arg Gln Asp Leu Met
   145 150 155 160
   Asn Ile Ala Gly Thr Thr Leu Ser Ser Lys Leu Leu Thr His His Lys
   165 170 175
   Asp His Phe Thr Lys Leu Ala Val Glu Ala Val Leu Arg Leu Lys Gly
   180 185 190
   Ser Gly Asn Leu Glu Ala Ile His Ile Ile Lys Lys Leu Gly Gly Ser
   195 200 205
   Leu Ala Asp Ser Tyr Leu Asp Glu Gly Phe Leu Leu Asp Lys Lys Ile
   210 215 220
   Gly Val Asn Gln Pro Lys Arg Ile Glu Asn Ala Lys Ile Leu Ile Ala
   225 230 235 240
   Asn Thr Gly Met Asp Thr Asp Lys Ile Lys Ile Phe Gly Ser Arg Val
   245 250 255
   Arg Val Asp Ser Thr Ala Lys Val Ala Glu Ile Glu His Ala Glu Lys
   260 265 270
   Glu Lys Met Lys Glu Lys Val Glu Arg Ile Leu Lys His Gly Ile Asn
   275 280 285
   Cys Phe Ile Asn Arg Gln Leu Ile Tyr Asn Tyr Pro Glu Gln Leu Phe
   290 295 300
   Gly Ala Ala Gly Val Met Ala Ile Glu His Ala Asp Phe Ala Gly Val
   305 310 315 320
   Glu Arg Leu Ala Leu Val Thr Gly Gly Glu Ile Ala Ser Thr Phe Asp
   325 330 335
   His Pro Glu Leu Val Lys Leu Gly Ser Cys Lys Leu Ile Glu Glu Val
   340 345 350
   Met Ile Gly Glu Asp Lys Leu Ile His Phe Ser Gly Val Ala Leu Gly
   355 360 365
   Glu Ala Cys Thr Ile Val Leu Arg Gly Ala Thr Gln Gln Ile Leu Asp
   370 375 380
   Glu Ala Glu Arg Ser Leu His Asp Ala Leu Cys Val Leu Ala Gln Thr
   385 390 395 400
   Val Lys Asp Ser Arg Thr Val Tyr Gly Gly Gly Cys Ser Glu Met Leu
   405 410 415
   Met Ala His Ala Val Thr Gln Leu Ala Asn Arg Thr Pro Gly Lys Glu
   420 425 430
   Ala Val Ala Met Glu Ser Tyr Ala Lys Ala Leu Arg Met Leu Pro Thr
   435 440 445
   Ile Ile Ala Asp Asn Ala Gly Tyr Asp Ser Ala Asp Leu Val Ala Gln
   450 455 460
   Leu Arg Ala Ala His Ser Glu Gly Asn Thr Thr Ala Gly Leu Asp Met
   465 470 475 480
   Arg Glu Gly Thr Ile Gly Asp Met Ala Ile Leu Gly Ile Thr Glu Ser
   485 490 495
   Phe Gln Val Lys Arg Gln Val Leu Leu Ser Ala Ala Glu Ala Ala Glu
   500 505 510
   Val Ile Leu Arg Val Asp Asn Ile Ile Lys Ala Ala Pro Arg Lys Arg
   515 520 525
   Val Pro Asp His His Pro Cys
   530 535
<210> 15
   <211> 553
   <212> PRT
   <213> Homo sapiens
<220>
   <221> MISC_FEATURE
   <223> Précurseur mitochondrial de la sous-unité alpha de l'ATP synthase
<400> 15
<210> 16
   <211> 449
   <212> PRT
   <213> Homo sapiens
<220>
   <221> MISC_FEATURE
   <223> ribonucleoprotéine H nucléaire hétérogène
<400> 16
   Met Met Leu Gly Thr Glu Gly Gly Glu Gly Phe Val Val Lys Val Arg
   1 5 10 15
   Gly Leu Pro Trp Ser Cys Ser Ala Asp Glu Val Gln Arg Phe Phe Ser
   20 25 30
   Asp Cys Lys Ile Gln Asn Gly Ala Gln Gly Ile Arg Phe Ile Tyr Thr
   35 40 45
   Arg Glu Gly Arg Pro Ser Gly Glu Ala Phe Val Glu Leu Glu Ser Glu
   50 55 60
   Asp Glu Val Lys Leu Ala Leu Lys Lys Asp Arg Glu Thr Met Gly His
   65 70 75 80
   Arg Tyr Val Glu Val Phe Lys Ser Asn Asn Val Glu Met Asp Trp Val
   85 90 95
   Leu Lys His Thr Gly Pro Asn Ser Pro Asp Thr Ala Asn Asp Gly Phe
   100 105 110
   Val Arg Leu Arg Gly Leu Pro Phe Gly Cys Ser Lys Glu Glu Ile Val
   115 120 125
   Gln Phe Phe Ser Gly Leu Glu Ile Val Pro Asn Gly Ile Thr Leu Pro
   130 135 140
   Val Asp Phe Gln Gly Arg Ser Thr Gly Glu Ala Phe Val Gln Phe Ala
   145 150 155 160
   Ser Gln Glu Ile Ala Glu Lys Ala Leu Lys Lys His Lys Glu Arg Ile
   165 170 175
   Gly His Arg Tyr Ile Glu Ile Phe Lys Ser Ser Arg Ala Glu Val Arg
   180 185 190
   Thr His Tyr Asp Pro Pro Arg Lys Leu Met Ala Met Gln Arg Pro Gly
   195 200 205
   Pro Tyr Asp Arg Pro Gly Ala Gly Arg Gly Tyr Asn Ser Ile Gly Arg
   210 215 220
   Gly Ala Gly Phe Glu Arg Met Arg Arg Gly Ala Tyr Gly Gly Gly Tyr
   225 230 235 240
   Gly Gly Tyr Asp Asp Tyr Asn Gly Tyr Asn Asp Gly Tyr Gly Phe Gly
   245 250 255
   Ser Asp Arg Phe Gly Arg Asp Leu Asn Tyr Cys Phe Ser Gly Met Ser
   260 265 270
   Asp His Arg Tyr Gly Asp Gly Gly Ser Thr Phe Gln Ser Thr Thr Gly
   275 280 285
   His Cys Val His Met Arg Gly Leu Pro Tyr Arg Ala Thr Glu Asn Asp
   290 295 300
   Ile Tyr Asn Phe Phe Ser Pro Leu Asn Pro Val Arg Val His Ile Glu
   305 310 315 320
   Ile Gly Pro Asp Gly Arg Val Thr Gly Glu Ala Asp Val Glu Phe Ala
   325 330 335
   Thr His Glu Asp Ala Val Ala Ala Met Ser Lys Asp Lys Ala Asn Met
   340 345 350
   Gln His Arg Tyr Val Glu Leu Phe Leu Asn Ser Thr Ala Gly Ala Ser
   355 360 365
   Gly Gly Ala Tyr Glu His Arg Tyr Val Glu Leu Phe Leu Asn Ser Thr
   370 375 380
   Ala Gly Ala Ser Gly Gly Ala Tyr Gly Ser Gln Met Met Gly Gly Met
   385 390 395 400
   Gly Leu Ser Asn Gln Ser Ser Tyr Gly Gly Pro Ala Ser Gln Gln Leu
   405 410 415
   Ser Gly Gly Tyr Gly Gly Gly Tyr Gly Gly Gln Ser Ser Met Ser Gly
   420 425 430
   Tyr Asp Gln Val Leu Gln Glu Asn Ser Ser Asp Phe Gln Ser Asn Ile
   435 440 445
   Ala
<210> 17
   <211> 444
   <212> PRT
   <213> Homo sapiens
<220>
   <221> MISC_FEATURE
   <223> Chaîne beta Tubuline
<400> 17
<210> 18
   <211> 364
   <212> PRT
   <213> Homo sapiens
<220>
   <221> MISC_FEATURE
   <223> Fructose-bisphosphate aldolase A
<400> 18
   Met Pro Tyr Gln Tyr Pro Ala Leu Thr Pro Glu Gln Lys Lys Glu Leu
   1               5                   10                  15
   Ser Asp Ile Ala His Arg Ile Val Ala Pro Gly Lys Gly Ile Leu Ala
               20                  25                  30
   Ala Asp Glu Ser Thr Gly Ser Ile Ala Lys Arg Leu Gln Ser Ile Gly
           35                  40                  45
   Thr Glu Asn Thr Glu Glu Asn Arg Arg Phe Tyr Arg Gln Leu Leu Leu
       50                  55                  60
   Thr Ala Asp Asp Arg Val Asn Pro Cys Ile Gly Gly Val Ile Leu Phe
   65                  70                  75                  80
   His Glu Thr Leu Tyr Gln Lys Ala Asp Asp Gly Arg Pro Phe Pro Gln
                   85                  90                  95
   Val Ile Lys Ser Lys Gly Gly Val Val Gly Ile Lys Val Asp Lys Gly
               100                 105                 110
   Val Val Pro Leu Ala Gly Thr Asn Gly Glu Thr Thr Thr Gln Gly Leu
           115                 120                 125
   Asp Gly Leu Ser Glu Arg Cys Ala Gln Tyr Lys Lys Asp Gly Ala Asp
       130                 135                 140
   Phe Ala Lys Trp Arg Cys Val Leu Lys Ile Gly Glu His Thr Pro Ser
   145                 150                 155                 160
   Ala Leu Ala Ile Met Glu Asn Ala Asn Val Leu Ala Arg Tyr Ala Ser
                   165                 170                 175
   Ile Cys Gln Gln Asn Gly Ile Val Pro Ile Val Glu Pro Glu Ile Leu
               180                 185                 190
   Pro Asp Gly Asp His Asp Leu Lys Arg Cys Gln Tyr Val Thr Glu Lys
           195                 200                 205
   Val Leu Ala Ala Val Tyr Lys Ala Leu Ser Asp His His Ile Tyr Leu
       210                 215                 220
   Glu Gly Thr Leu Leu Lys Pro Asn Met Val Thr Pro Gly His Ala Cys
   225                 230                 235                 240
   Thr Gln Lys Phe Ser His Glu Glu Ile Ala Met Ala Thr Val Thr Ala
                   245                 250                 255
   Leu Arg Arg Thr Val Pro Pro Ala Val Thr Gly Ile Thr Phe Leu Ser
               260                 265                 270
   Gly Gly Gln Ser Glu Glu Glu Ala Ser Ile Asn Leu Asn Ala Ile Asn
           275                 280                 285
   Lys Cys Pro Leu Leu Lys Pro Trp Ala Leu Thr Phe Ser Tyr Gly Arg
       290                 295                 300
   Ala Leu Gln Ala Ser Ala Leu Lys Ala Trp Gly Gly Lys Lys Glu Asn
   305                 310                 315                 320
   Leu Lys Ala Ala Gln Glu Glu Tyr Val Lys Arg Ala Leu Ala Asn Ser
                   325                 330                 335
   Leu Ala Cys Gln Gly Lys Tyr Thr Pro Ser Gly Gln Ala Gly Ala Ala
               340                 345                 350
   Ala Ser Glu Ser Leu Phe Val Ser Asn His Ala Tyr
           355                 360
<210> 19
   <211> 315
   <212> PRT
   <213> Homo sapiens
<220>
   <221> MISC_FEATURE
   <223> Précurseur de Calumenine
<400> 19
   Met Asp Leu Arg Gln Phe Leu Met Cys Leu Ser Leu Cys Thr Ala Phe
   1               5                   10                  15
   Ala Leu Ser Lys Pro Thr Glu Lys Lys Asp Arg Val His His Glu Pro
               20                  25                  30
   Gln Leu Ser Asp Lys Val His Asn Asp Ala Gln Ser Phe Asp Tyr Asp
           35                  40                  45
   His Asp Ala Phe Leu Gly Ala Glu Glu Ala Lys Thr Phe Asp Gln Leu
       50                  55                  60
   Thr Pro Glu Glu Ser Lys Glu Arg Leu Gly Lys Ile Val Ser Lys Ile
   65                  70                  75                  80
   Asp Gly Asp Lys Asp Gly Phe Val Thr Val Asp Glu Leu Lys Asp Trp
                   85                  90                  95
   Ile Lys Phe Ala Gln Lys Arg Trp Ile Tyr Glu Asp Val Glu Arg Gln
               100                 105                 110
   Trp Lys Gly His Asp Leu Asn Glu Asp Gly Leu Val Ser Trp Glu Glu
           115                 120                 125
   Tyr Lys Asn Ala Thr Tyr Gly Tyr Val Leu Asp Asp Pro Asp Pro Asp
       130                 135                 140
   Asp Gly Phe Asn Tyr Lys Gln Met Met Val Arg Asp Glu Arg Arg Phe
   145                 150                 155                 160
   Lys Met Ala Asp Lys Asp Gly Asp Leu Ile Ala Thr Lys Glu Glu Phe
                   165                 170                 175
   Thr Ala Phe Leu His Pro Glu Glu Tyr Asp Tyr Met Lys Asp Ile Val
   180 185 190
   Val Gln Glu Thr Met Glu Asp Ile Asp Lys Asn Ala Asp Gly Phe Ile
   195 200 205
   Asp Leu Glu Glu Tyr Ile Gly Asp Met Tyr Ser His Asp Gly Asn Thr
   210 215 220
   Asp Glu Pro Glu Trp Val Lys Thr Glu Arg Glu Gln Phe Val Glu Phe
   225 230 235 240
   Arg Asp Lys Asn Arg Asp Gly Lys Met Asp Lys Glu Glu Thr Lys Asp
   245 250 255
   Trp Ile Leu Pro Ser Asp Tyr Asp His Ala Glu Ala Glu Ala Arg His
   260 265 270
   Leu Val Tyr Glu Ser Asp Gln Asn Lys Asp Gly Lys Leu Thr Lys Glu
   275 280 285
   Glu Ile Val Asp Lys Tyr Asp Leu Phe Val Gly Ser Gln Ala Thr Asp
   290 295 300
   Phe Gly Glu Ala Leu Val Arg His Asp Glu Phe
   305 310 315
<210> 20
   <211> 328
   <212> PRT
   <213> Homo sapiens
<220>
   <221> MISC_FEATURE
   <223> Reticulocalbine-3
<400> 20
   Met Met Trp Arg Pro Ser Val Leu Leu Leu Leu Leu Leu Leu Arg His
   1 5 10 15
   Gly Ala Gln Gly Lys Pro Ser Pro Asp Ala Gly Pro His Gly Gln Gly
   20 25 30
   Arg Val His Gln Ala Ala Pro Leu Ser Asp Ala Pro His Asp Asp Ala
   35 40 45
   His Gly Asn Phe Gln Tyr Asp His Glu Ala Phe Leu Gly Arg Glu Val
   50 55 60
   Ala Lys Glu Phe Asp Gln Leu Thr Pro Glu Glu Ser Gln Ala Arg Leu
   65                  70                  75                  80
   Gly Arg Ile Val Asp Arg Met Asp Arg Ala Gly Asp Gly Asp Gly Trp
                   85                  90                  95
   Val Ser Leu Ala Glu Leu Arg Ala Trp Ile Ala His Thr Gln Gln Arg
               100                 105                 110
   His Ile Arg Asp Ser Val Ser Ala Ala Trp Asp Thr Tyr Asp Thr Asp
           115                 120                 125
   Arg Asp Gly Arg Val Gly Trp Glu Glu Leu Arg Asn Ala Thr Tyr Gly
       130                 135                 140
   His Tyr Ala Pro Gly Glu Glu Phe His Asp Val Glu Asp Ala Glu Thr
   145                 150                 155                 160
   Tyr Lys Lys Met Leu Ala Arg Asp Glu Arg Arg Phe Arg Val Ala Asp
                   165                 170                 175
   Gln Asp Gly Asp Ser Met Ala Thr Arg Glu Glu Leu Thr Ala Phe Leu
               180                 185                 190
   His Pro Glu Glu Phe Pro His Met Arg Asp Ile Val Ile Ala Glu Thr
           195                 200                 205
   Leu Glu Asp Leu Asp Arg Asn Lys Asp Gly Tyr Val Gln Val Glu Glu
       210                 215                 220
   Tyr Ile Ala Asp Leu Tyr Ser Ala Glu Pro Gly Glu Glu Glu Pro Ala
   225                 230                 235                 240
   Trp Val Gln Thr Glu Arg Gln Gln Phe Arg Asp Phe Arg Asp Leu Asn
                   245                 250                 255
   Lys Asp Gly His Leu Asp Gly Ser Glu Val Gly His Trp Val Leu Pro
               260                 265                 270
   Pro Ala Gln Asp Gln Pro Leu Val Glu Ala Asn His Leu Leu His Glu
           275                 280                 285
   Ser Asp Thr Asp Lys Asp Gly Arg Leu Ser Lys Ala Glu Ile Leu Gly
       290                 295                 300
   Asn Trp Asn Met Phe Val Gly Ser Gln Ala Thr Asn Tyr Gly Glu Asp
   305 310 315 320
   Leu Thr Arg His His Asp Glu Leu
   325
<210> 21
   <211> 376
   <212> PRT
   <213> Homo sapiens
<220>
   <221> MISC_FEATURE
   <223> Sous-unité 13 régulatrice non-ATPase du proteasome 26S
<400> 21
   Met Lys Asp Val Pro Gly Phe Leu Gln Gln Ser Gln Ser Ser Gly Pro
   1 5 10 15
   Gly Gln Pro Ala Val Trp His Arg Leu Glu Glu Leu Tyr Thr Lys Lys
   20 25 30
   Leu Trp His Gln Leu Thr Leu Gln Val Leu Asp Phe Val Gln Asp Pro
   35 40 45
   Cys Phe Ala Gln Gly Asp Gly Leu Ile Lys Leu Tyr Glu Asn Phe Ile
   50 55 60
   Ser Glu Phe Glu His Arg Val Asn Pro Leu Ser Leu Val Glu Ile Ile
   65 70 75 80
   Leu His Val Val Arg Gln Met Thr Asp Pro Asn Val Ala Leu Thr Phe
   85 90 95
   Leu Glu Lys Thr Arg Glu Lys Val Lys Ser Ser Asp Glu Ala Val Ile
   100 105 110
   Leu Cys Lys Thr Ala Ile Gly Ala Leu Lys Leu Asn Ile Gly Asp Leu
   115 120 125
   Gln Val Thr Lys Glu Thr Ile Glu Asp Val Glu Glu Met Leu Asn Asn
   130 135 140
   Leu Pro Gly Val Thr Ser Val His Ser Arg Phe Tyr Asp Leu Ser Ser
   145 150 155 160
   Lys Tyr Tyr Gln Thr Ile Gly Asn His Ala Ser Tyr Tyr Lys Asp Ala
   165 170 175
   Leu Arg Phe Leu Gly Cys Val Asp Ile Lys Asp Leu Pro Val Ser Glu
   180 185 190
   Gln Gln Glu Arg Ala Phe Thr Leu Gly Leu Ala Gly Leu Leu Gly Glu
   195 200 205
   Gly Val Phe Asn Phe Gly Glu Leu Leu Met His Pro Val Leu Glu Ser
   210 215 220
   Leu Arg Asn Thr Asp Arg Gln Trp Leu Ile Asp Thr Leu Tyr Ala Phe
   225 230 235 240
   Asn Ser Gly Asn Val Glu Arg Phe Gln Thr Leu Lys Thr Ala Trp Gly
   245 250 255
   Gln Gln Pro Asp Leu Ala Ala Asn Glu Ala Gln Leu Leu Arg Lys Ile
   260 265 270
   Gln Leu Leu Cys Leu Met Glu Met Thr Phe Thr Arg Pro Ala Asn His
   275 280 285
   Arg Gln Leu Thr Phe Glu Glu Ile Ala Lys Ser Ala Lys Ile Thr Val
   290 295 300
   Asn Glu Val Glu Leu Leu Val Met Lys Ala Leu Ser Val Gly Leu Val
   305 310 315 320
   Lys Gly Ser Ile Asp Glu Val Asp Lys Arg Val His Met Thr Trp Val
   325 330 335
   Gln Pro Arg Val Leu Asp Leu Gln Gln Ile Lys Gly Met Lys Asp Arg
   340 345 350
   Leu Glu Phe Trp Cys Thr Asp Val Lys Ser Met Glu Met Leu Val Glu
   355 360 365
   His Gln Ala His Asp Ile Leu Thr
   370 375
<210> 22
   <211> 289
   <212> PRT
   <213> Homo sapiens
<220>
   <221> MISC_FEATURE
   <223> pyrophosphatase inorganique
<400> 22
   Met Ser Gly Phe Ser Thr Glu Glu Arg Ala Ala Pro Phe Ser Leu Glu
   1               5                   10                  15
   Tyr Arg Val Phe Leu Lys Asn Glu Lys Gly Gln Tyr Ile Ser Pro Phe
               20                  25                  30
   His Asp Ile Pro Ile Tyr Ala Asp Lys Asp Val Phe His Met Val Val
           35                  40                  45
   Glu Val Pro Arg Trp Ser Asn Ala Lys Met Glu Ile Ala Thr Lys Asp
       50                  55                  60
   Pro Leu Asn Pro Ile Lys Gln Asp Val Lys Lys Gly Lys Leu Arg Tyr
   65                  70                  75                  80
   Val Ala Asn Leu Phe Pro Tyr Lys Gly Tyr Ile Trp Asn Tyr Gly Ala
                   85                  90                  95
   Ile Pro Gln Thr Trp Glu Asp Pro Gly His Asn Asp Lys His Thr Gly
               100                 105                 110
   Cys Cys Gly Asp Asn Asp Pro Ile Asp Val Cys Glu Ile Gly Ser Lys
           115                 120                 125
   Val Cys Ala Arg Gly Glu Ile Ile Gly Val Lys Val Leu Gly Ile Leu
       130                 135                 140
   Ala Met Ile Asp Glu Gly Glu Thr Asp Trp Lys Val Ile Ala Ile Asn
   145                 150                 155                 160
   Val Asp Asp Pro Asp Ala Ala Asn Tyr Asn Asp Ile Asn Asp Val Lys
                   165                 170                 175
   Arg Leu Lys Pro Gly Tyr Leu Glu Ala Thr Val Asp Trp Phe Arg Arg
               180                 185                 190
   Tyr Lys Val Pro Asp Gly Lys Pro Glu Asn Glu Phe Ala Phe Asn Ala
           195                 200                 205
   Glu Phe Lys Asp Lys Asp Phe Ala Ile Asp Ile Ile Lys Ser Thr His
       210                 215                 220
   Asp His Trp Lys Ala Leu Val Thr Lys Lys Thr Asn Gly Lys Gly Ile
   225 230 235 240
   Ser Cys Met Asn Thr Thr Leu Ser Glu Ser Pro Phe Lys Cys Asp Pro
   245 250 255
   Asp Ala Ala Arg Ala Ile Val Asp Ala Leu Pro Pro Pro Cys Glu Ser
   260 265 270
   Ala Cys Thr Val Pro Thr Asp Val Asp Lys Trp Phe His His Gln Lys
   275 280 285
   Asn
<210> 23
   <211> 320
   <212> PRT
   <213> Homo sapiens
<220>
   <221> MISC_FEATURE
   <223> Annexine A5
<400> 23
   Met Ala Gln Val Leu Arg Gly Thr Val Thr Asp Phe Pro Gly Phe Asp
   1 5 10 15
   Glu Arg Ala Asp Ala Glu Thr Leu Arg Lys Ala Met Lys Gly Leu Gly
   20 25 30
   Thr Asp Glu Glu Ser Ile Leu Thr Leu Leu Thr Ser Arg Ser Asn Ala
   35 40 45
   Gln Arg Gln Glu Ile Ser Ala Ala Phe Lys Thr Leu Phe Gly Arg Asp
   50 55 60
   Leu Leu Asp Asp Leu Lys Ser Glu Leu Thr Gly Lys Phe Glu Lys Leu
   65 70 75 80
   Ile Val Ala Leu Met Lys Pro Ser Arg Leu Tyr Asp Ala Tyr Glu Leu
   85 90 95
   Lys His Ala Leu Lys Gly Ala Gly Thr Asn Glu Lys Val Leu Thr Glu
   100 105 110
   Ile Ile Ala Ser Arg Thr Pro Glu Glu Leu Arg Ala Ile Lys Gln Val
   115 120 125
   Tyr Glu Glu Glu Tyr Gly Ser Ser Leu Glu Asp Asp Val Val Gly Asp
   130 135 140
   Thr Ser Gly Tyr Tyr Gln Arg Met Leu Val Val Leu Leu Gln Ala Asn
   145 150 155 160
   Arg Asp Pro Asp Ala Gly Ile Asp Glu Ala Gln Val Glu Gln Asp Ala
   165 170 175
   Gln Ala Leu Phe Gln Ala Gly Glu Leu Lys Trp Gly Thr Asp Glu Glu
   180 185 190
   Lys Phe Ile Thr Ile Phe Gly Thr Arg Ser Val Ser His Leu Arg Lys
   195 200 205
   Val Phe Asp Lys Tyr Met Thr Ile Ser Gly Phe Gln Ile Glu Glu Thr
   210 215 220
   Ile Asp Arg Glu Thr Ser Gly Asn Leu Glu Gln Leu Leu Leu Ala Val
   225 230 235 240
   Val Lys Ser Ile Arg Ser Ile Pro Ala Tyr Leu Ala Glu Thr Leu Tyr
   245 250 255
   Tyr Ala Met Lys Gly Ala Gly Thr Asp Asp His Thr Leu Ile Arg Val
   260 265 270
   Met Val Ser Arg Ser Glu Ile Asp Leu Phe Asn Ile Arg Lys Glu Phe
   275 280 285
   Arg Lys Asn Phe Ala Thr Ser Leu Tyr Ser Met Ile Lys Gly Asp Thr
   290 295 300
   Ser Gly Asp Tyr Lys Lys Ala Leu Leu Leu Leu Cys Gly Glu Asp Asp
   305 310 315 320
<210> 24
   <211> 255
   <212> PRT
   <213> Homo sapiens
<220>
   <221> MISC_FEATURE
   <223> 14-3-3 protéine epsilon
<400> 24
   Met Asp Asp Arg Glu Asp Leu Val Tyr Gln Ala Lys Leu Ala Glu Gln
   1               5                   10                  15
   Ala Glu Arg Tyr Asp Glu Met Val Glu Ser Met Lys Lys Val Ala Gly
               20                  25                  30
   Met Asp Val Glu Leu Thr Val Glu Glu Arg Asn Leu Leu Ser Val Ala
           35                  40                  45
   Tyr Lys Asn Val Ile Gly Ala Arg Arg Ala Ser Trp Arg Ile Ile Ser
       50                  55                  60
   Ser Ile Glu Gln Lys Glu Glu Asn Lys Gly Gly Glu Asp Lys Leu Lys
   65                  70                  75                  80
   Met Ile Arg Glu Tyr Arg Gln Met Val Glu Thr Glu Leu Lys Leu Ile
                   85                  90                  95
   Cys Cys Asp Ile Leu Asp Val Leu Asp Lys His Leu Ile Pro Ala Ala
               100                 105                 110
   Asn Thr Gly Glu Ser Lys Val Phe Tyr Tyr Lys Met Lys Gly Asp Tyr
           115                 120                 125
   His Arg Tyr Leu Ala Glu Phe Ala Thr Gly Asn Asp Arg Lys Glu Ala
       130                 135                 140
   Ala Glu Asn Ser Leu Val Ala Tyr Lys Ala Ala Ser Asp Ile Ala Met
   145                 150                 155                 160
   Thr Glu Leu Pro Pro Thr His Pro Ile Arg Leu Gly Leu Ala Leu Asn
                   165                 170                 175
   Phe Ser Val Phe Tyr Tyr Glu Ile Leu Asn Ser Pro Asp Arg Ala Cys
               180                 185                 190
   Arg Leu Ala Lys Ala Ala Phe Asp Asp Ala Ile Ala Glu Leu Asp Thr
           195                 200                 205
   Leu Ser Glu Glu Ser Tyr Lys Asp Ser Thr Leu Ile Met Gln Leu Leu
       210                 215                 220
   Arg Asp Asn Leu Thr Leu Trp Thr Ser Asp Met Gln Gly Asp Gly Glu
   225 230 235 240
   Glu Gln Asn Lys Glu Ala Leu Gln Asp Val Glu Asp Glu Asn Gln
   245 250 255
<210> 25
   <211> 258
   <212> PRT
   <213> Homo sapiens
<220>
   <221> MISC_FEATURE
   <223> 6-phosphogluconolactonase
<400> 25
   Met Ala Ala Pro Ala Pro Gly Leu Ile Ser Val Phe Ser Ser Ser Gln
   1 5 10 15
   Glu Leu Gly Ala Ala Leu Ala Gln Leu Val Ala Gln Arg Ala Ala Cys
   20 25 30
   Cys Leu Ala Gly Ala Arg Ala Arg Phe Ala Leu Gly Leu Ser Gly Gly
   35 40 45
   Ser Leu Val Ser Met Leu Ala Arg Glu Leu Pro Ala Ala Val Ala Pro
   50 55 60
   Ala Gly Pro Ala Ser Leu Ala Arg Trp Thr Leu Gly Phe Cys Asp Glu
   65 70 75 80
   Arg Leu Val Pro Phe Asp His Ala Glu Ser Thr Tyr Gly Leu Tyr Arg
   85 90 95
   Thr His Leu Leu Ser Arg Leu Pro Ile Pro Glu Ser Gln Val Ile Thr
   100 105 110
   Ile Asn Pro Glu Leu Pro Val Glu Glu Ala Ala Glu Asp Tyr Ala Lys
   115 120 125
   Lys Leu Arg Gln Ala Phe Gln Gly Asp Ser Ile Pro Val Phe Asp Leu
   130 135 140
   Leu Ile Leu Gly Val Gly Pro Asp Gly His Thr Cys Ser Leu Phe Pro
   145 150 155 160
   Asp His Pro Leu Leu Gln Glu Arg Glu Lys Ile Val Ala Pro Ile Ser
   165 170 175
   Asp Ser Pro Lys Pro Pro Pro Gln Arg Val Thr Leu Thr Leu Pro Val
   180 185 190
   Leu Asn Ala Ala Arg Thr Val Ile Phe Val Ala Thr Gly Glu Gly Lys
   195 200 205
   Ala Ala Val Leu Lys Arg Ile Leu Glu Asp Gln Glu Glu Asn Pro Leu
   210 215 220
   Pro Ala Ala Leu Val Gln Pro His Thr Gly Lys Leu Cys Trp Phe Leu
   225 230 235 240
   Asp Glu Ala Ala Ala Arg Leu Leu Thr Val Pro Phe Glu Lys His Ser
   245 250 255
   Thr Leu
<210> 26
   <211> 135
   <212> PRT
   <213> Homo sapiens
<220>
   <221> MISC_FEATURE
   <223> Galectine-1
<400> 26
   Met Ala Cys Gly Leu Val Ala Ser Asn Leu Asn Leu Lys Pro Gly Glu
   1 5 10 15
   Cys Leu Arg Val Arg Gly Glu Val Ala Pro Asp Ala Lys Ser Phe Val
   20 25 30
   Leu Asn Leu Gly Lys Asp Ser Asn Asn Leu Cys Leu His Phe Asn Pro
   35 40 45
   Arg Phe Asn Ala His Gly Asp Ala Asn Thr Ile Val Cys Asn Ser Lys
   50 55 60
   Asp Gly Gly Ala Trp Gly Thr Glu Gln Arg Glu Ala Val Phe Pro Phe
   65 70 75 80
   Gln Pro Gly Ser Val Ala Glu Val Cys Ile Thr Phe Asp Gln Ala Asn
   85 90 95
   Leu Thr Val Lys Leu Pro Asp Gly Tyr Glu Phe Lys Phe Pro Asn Arg
   100 105 110
   Leu Asn Leu Glu Ala Ile Asn Tyr Met Ala Ala Asp Gly Asp Phe Lys
   115 120 125
   Ile Lys Cys Val Ala Phe Asp
   130 135
<210> 27
   <211> 520
   <212> PRT
   <213> Homo sapiens
<220>
   <221> MISC_FEATURE
   <223> Précurseur mitochondrial de la Succinyl-CoA:3-ketoacid-coenzyme A transferase 1
<400> 27
<210> 28
   <211> 355
   <212> PRT
   <213> Homo sapiens
<220>
   <221> MISC_FEATURE
   <223> ribonucléoprotéine D0 nucléaire hétérogène
<400> 28
   Met Ser Glu Glu Gln Phe Gly Gly Asp Gly Ala Ala Ala Ala Ala Thr
   1 5 10 15
   Ala Ala Val Gly Gly Ser Ala Gly Glu Gln Glu Gly Ala Met Val Ala
   20 25 30
   Ala Thr Gln Gly Ala Ala Ala Ala Ala Gly Ser Gly Ala Gly Thr Gly
   35 40 45
   Gly Gly Thr Ala Ser Gly Gly Thr Glu Gly Gly Ser Ala Glu Ser Glu
   50 55 60
   Gly Ala Lys Ile Asp Ala Ser Lys Asn Glu Glu Asp Glu Gly His Ser
   65 70 75 80
   Asn Ser Ser Pro Arg His Ser Glu Ala Ala Thr Ala Gln Arg Glu Glu
   85 90 95
   Trp Lys Met Phe Ile Gly Gly Leu Ser Trp Asp Thr Thr Lys Lys Asp
   100 105 110
   Leu Lys Asp Tyr Phe Ser Lys Phe Gly Glu Val Val Asp Cys Thr Leu
   115 120 125
   Lys Leu Asp Pro Ile Thr Gly Arg Ser Arg Gly Phe Gly Phe Val Leu
   130 135 140
   Phe Lys Glu Ser Glu Ser Val Asp Lys Val Met Asp Gln Lys Glu His
   145 150 155 160
   Lys Leu Asn Gly Lys Val Ile Asp Pro Lys Arg Ala Lys Ala Met Lys
   165 170 175
   Thr Lys Glu Pro Val Lys Lys Ile Phe Val Gly Gly Leu Ser Pro Asp
   180 185 190
   Thr Pro Glu Glu Lys Ile Arg Glu Tyr Phe Gly Gly Phe Gly Glu Val
   195 200 205
   Glu Ser Ile Glu Leu Pro Met Asp Asn Lys Thr Asn Lys Arg Arg Gly
   210 215 220
   Phe Cys Phe Ile Thr Phe Lys Glu Glu Glu Pro Val Lys Lys Ile Met
   225 230 235 240
   Glu Lys Lys Tyr His Asn Val Gly Leu Ser Lys Cys Glu Ile Lys Val
   245 250 255
   Ala Met Ser Lys Glu Gln Tyr Gln Gln Gln Gln Gln Trp Gly Ser Arg
   260 265 270
   Gly Gly Phe Ala Gly Arg Ala Arg Gly Arg Gly Gly Gly Pro Ser Gln
   275 280 285
   Asn Trp Asn Gln Gly Tyr Ser Asn Tyr Trp Asn Gln Gly Tyr Gly Asn
   290 295 300
   Tyr Gly Tyr Asn Ser Gln Gly Tyr Gly Gly Tyr Gly Gly Tyr Asp Tyr
   305 310 315 320
   Thr Gly Tyr Asn Asn Tyr Tyr Gly Tyr Gly Asp Tyr Ser Asn Gln Gln
   325 330 335
   Ser Gly Tyr Gly Lys Val Ser Arg Arg Gly Gly His Gln Asn Ser Tyr
   340 345 350
   Lys Pro Tyr
   355
<210> 29
   <211> 433
   <212> PRT
   <213> Homo sapiens
<220>
   <221> MISC_FEATURE
   <223> Sous-unité 7 régulatrice de la protease 26S
<400> 29
   Met Pro Asp Tyr Leu Gly Ala Asp Gln Arg Lys Thr Lys Glu Asp Glu
   1 5 10 15
   Lys Asp Asp Lys Pro Ile Arg Ala Leu Asp Glu Gly Asp Ile Ala Leu
   20 25 30
   Leu Lys Thr Tyr Gly Gln Ser Thr Tyr Ser Arg Gln Ile Lys Gln Val
   35 40 45
   Glu Asp Asp Ile Gln Gln Leu Leu Lys Lys Ile Asn Glu Leu Thr Gly
   50 55 60
   Ile Lys Glu Ser Asp Thr Gly Leu Ala Pro Pro Ala Leu Trp Asp Leu
   65 70 75 80
   Ala Ala Asp Lys Gln Thr Leu Gln Ser Glu Gln Pro Leu Gln Val Ala
   85 90 95
   Arg Cys Thr Lys Ile Ile Asn Ala Asp Ser Glu Asp Pro Lys Tyr Ile
   100 105 110
   Ile Asn Val Lys Gln Phe Ala Lys Phe Val Val Asp Leu Ser Asp Gln
   115 120 125
   Val Ala Pro Thr Asp Ile Glu Glu Gly Met Arg Val Gly Val Asp Arg
   130 135 140
   Asn Lys Tyr Gln Ile His Ile Pro Leu Pro Pro Lys Ile Asp Pro Thr
   145 150 155 160
   Val Thr Met Met Gln Val Glu Glu Lys Pro Asp Val Thr Tyr Ser Asp
   165 170 175
   Val Gly Gly Cys Lys Glu Gln Ile Glu Lys Leu Arg Glu Val Val Glu
   180 185 190
   Thr Pro Leu Leu His Pro Glu Arg Phe Val Asn Leu Gly Ile Glu Pro
   195 200 205
   Pro Lys Gly Val Leu Leu Phe Gly Pro Pro Gly Thr Gly Lys Thr Leu
   210 215 220
   Cys Ala Arg Ala Val Ala Asn Arg Thr Asp Ala Cys Phe Ile Arg Val
   225 230 235 240
   Ile Gly Ser Glu Leu Val Gln Lys Tyr Val Gly Glu Gly Ala Arg Met
   245 250 255
   Val Arg Glu Leu Phe Glu Met Ala Arg Thr Lys Lys Ala Cys Leu Ile
   260 265 270
   Phe Phe Asp Glu Ile Asp Ala Ile Gly Gly Ala Arg Phe Asp Asp Gly
   275 280 285
   Ala Gly Gly Asp Asn Glu Val Gln Arg Thr Met Leu Glu Leu Ile Asn
   290 295 300
   Gln Leu Asp Gly Phe Asp Pro Arg Gly Asn Ile Lys Val Leu Met Ala
   305 310 315 320
   Thr Asn Arg Pro Asp Thr Leu Asp Pro Ala Leu Met Arg Pro Gly Arg
   325 330 335
   Leu Asp Arg Lys Ile Glu Phe Ser Leu Pro Asp Leu Glu Gly Arg Thr
   340 345 350
   His Ile Phe Lys Ile His Ala Arg Ser Met Ser Val Glu Arg Asp Ile
   355 360 365
   Arg Phe Glu Leu Leu Ala Arg Leu Cys Pro Asn Ser Thr Gly Ala Glu
       370                 375                 380
   Ile Arg Ser Val Cys Thr Glu Ala Gly Met Phe Ala Ile Arg Ala Arg
   385                 390                 395                 400
   Arg Lys Ile Ala Thr Glu Lys Asp Phe Leu Glu Ala Val Asn Lys Val
                   405                 410                 415
   Ile Lys Ser Tyr Ala Lys Phe Ser Ala Thr Pro Arg Tyr Met Thr Tyr
               420                 425                 430
   Asn
<210> 30
   <211> 316
   <212> PRT
   <213> Homo sapiens
<220>
   <221> MISC_FEATURE
   <223> Heme oxygénase 2
<400> 30
   Met Ser Ala Glu Val Glu Thr Ser Glu Gly Val Asp Glu Ser Glu Lys
   1               5                   10                  15
   Lys Asn Ser Gly Ala Leu Glu Lys Glu Asn Gln Met Arg Met Ala Asp
               20                  25                  30
   Leu Ser Glu Leu Leu Lys Glu Gly Thr Lys Glu Ala His Asp Arg Ala
           35                  40                  45
   Glu Asn Thr Gln Phe Val Lys Asp Phe Leu Lys Gly Asn Ile Lys Lys
       50                  55                  60
   Glu Leu Phe Lys Leu Ala Thr Thr Ala Leu Tyr Phe Thr Tyr Ser Ala
   65                  70                  75                  80
   Leu Glu Glu Glu Met Glu Arg Asn Lys Asp His Pro Ala Phe Ala Pro
                   85                  90                  95
   Leu Tyr Phe Pro Met Glu Leu His Arg Lys Glu Ala Leu Thr Lys Asp
               100                 105                 110
   Met Glu Tyr Phe Phe Gly Glu Asn Trp Glu Glu Gln Val Gln Cys Pro
           115                 120                 125
   Lys Ala Ala Gln Lys Tyr Val Glu Arg Ile His Tyr Ile Gly Gln Asn
       130                 135                 140
   Glu Pro Glu Leu Leu Val Ala His Ala Tyr Thr Arg Tyr Met Gly Asp
   145                 150                 155                 160
   Leu Ser Gly Gly Gln Val Leu Lys Lys Val Ala Gln Arg Ala Leu Lys
                   165                 170                 175
   Leu Pro Ser Thr Gly Glu Gly Thr Gln Phe Tyr Leu Phe Glu Asn Val
               180                 185                 190
   Asp Asn Ala Gln Gln Phe Lys Gln Leu Tyr Arg Ala Arg Met Asn Ala
           195                 200                 205
   Leu Asp Leu Asn Met Lys Thr Lys Glu Arg Ile Val Glu Glu Ala Asn
       210                 215                 220
   Lys Ala Phe Glu Tyr Asn Met Gln Ile Phe Asn Glu Leu Asp Gln Ala
   225                 230                 235                 240
   Gly Ser Thr Leu Ala Arg Glu Thr Leu Glu Asp Gly Phe Pro Val His
                   245                 250                 255
   Asp Gly Lys Gly Asp Met Arg Lys Cys Pro Phe Tyr Ala Ala Glu Gln
               260                 265                 270
   Asp Lys Gly Ala Leu Glu Gly Ser Ser Cys Pro Phe Arg Thr Ala Met
           275                 280                 285
   Ala Val Leu Arg Lys Pro Ser Leu Gln Phe Ile Leu Ala Ala Gly Val
       290                 295                 300
   Ala Leu Ala Ala Gly Leu Leu Ala Trp Tyr Tyr Met
   305                 310                 315
<210> 31
   <211> 126
   <212> PRT
   <213> Homo sapiens
<220>
   <221> MISC_FEATURE
   <223> Histone H2B type F-S
<400> 31
   Met Pro Glu Pro Ala Lys Ser Ala Pro Ala Pro Lys Lys Gly Ser Lys
   1 5 10 15
   Lys Ala Val Thr Lys Ala Gln Lys Lys Asp Gly Arg Lys Arg Lys Arg
   20 25 30
   Ser Arg Lys Glu Ser Tyr Ser Val Tyr Val Tyr Lys Val Leu Lys Gln
   35 40 45
   Val His Pro Asp Thr Gly Ile Ser Ser Lys Ala Met Gly Ile Met Asn
   50 55 60
   Ser Phe Val Asn Asp Ile Phe Glu Arg Ile Ala Gly Glu Ala Ser Arg
   65 70 75 80
   Leu Pro His Tyr Asn Lys Arg Ser Thr Ile Thr Ser Arg Glu Ile Gln
   85 90 95
   Thr Ala Val Arg Leu Leu Leu Pro Gly Glu Leu Ala Lys His Ala Val
   100 105 110
   Ser Glu Gly Thr Lys Ala Val Thr Lys Tyr Thr Ser Ala Lys
   115 120 125
<210> 32
   <211> 241
   <212> PRT
   <213> Homo sapiens
<220>
   <221> MISC_FEATURE
   <223> sous-unité alpha type-5 du Proteasome
<400> 32
   Met Phe Leu Thr Arg Ser Glu Tyr Asp Arg Gly Val Asn Thr Phe Ser
   1 5 10 15
   Pro Glu Gly Arg Leu Phe Gln Val Glu Tyr Ala Ile Glu Ala Ile Lys
   20 25 30
   Leu Gly Ser Thr Ala Ile Gly Ile Gln Thr Ser Glu Gly Val Cys Leu
   35 40 45
   Ala Val Glu Lys Arg Ile Thr Ser Pro Leu Met Glu Pro Ser Ser Ile
   50 55 60
   Glu Lys Ile Val Glu Ile Asp Ala His Ile Gly Cys Ala Met Ser Gly
   65 70 75 80
   Leu Ile Ala Asp Ala Lys Thr Leu Ile Asp Lys Ala Arg Val Glu Thr
   85 90 95
   Gln Asn His Trp Phe Thr Tyr Asn Glu Thr Met Thr Val Glu Ser Val
   100 105 110
   Thr Gln Ala Val Ser Asn Leu Ala Leu Gln Phe Gly Glu Glu Asp Ala
   115 120 125
   Asp Pro Gly Ala Met Ser Arg Pro Phe Gly Val Ala Leu Leu Phe Gly
   130 135 140
   Gly Val Asp Glu Lys Gly Pro Gln Leu Phe His Met Asp Pro Ser Gly
   145 150 155 160
   Thr Phe Val Gln Cys Asp Ala Arg Ala Ile Gly Ser Ala Ser Glu Gly
   165 170 175
   Ala Gln Ser Ser Leu Gln Glu Val Tyr His Lys Ser Met Thr Leu Lys
   180 185 190
   Glu Ala Ile Lys Ser Ser Leu Ile Ile Leu Lys Gln Val Met Glu Glu
   195 200 205
   Lys Leu Asn Ala Thr Asn Ile Glu Leu Ala Thr Val Gln Pro Gly Gln
   210 215 220
   Asn Phe His Met Phe Thr Lys Glu Glu Leu Glu Glu Val Ile Lys Asp
   225 230 235 240
   Ile
<210> 33
   <211> 201
   <212> PRT
   <213> Homo sapiens
<220>
   <221> MISC_FEATURE
   <223> sous-unité beta type-2 du Proteasome
<400> 33
   Met Glu Tyr Leu Ile Gly Ile Gln Gly Pro Asp Tyr Val Leu Val Ala
   1               5                   10                  15
   Ser Asp Arg Val Ala Ala Ser Asn Ile Val Gln Met Lys Asp Asp His
               20                  25                  30
   Asp Lys Met Phe Lys Met Ser Glu Lys Ile Leu Leu Leu Cys Val Gly
           35                  40                  45
   Glu Ala Gly Asp Thr Val Gln Phe Ala Glu Tyr Ile Gln Lys Asn Val
       50                  55                  60
   Gln Leu Tyr Lys Met Arg Asn Gly Tyr Glu Leu Ser Pro Thr Ala Ala
   65                  70                  75                  80
   Ala Asn Phe Thr Arg Arg Asn Leu Ala Asp Cys Leu Arg Ser Arg Thr
                   85                  90                  95
   Pro Tyr His Val Asn Leu Leu Leu Ala Gly Tyr Asp Glu His Glu Gly
               100                 105                 110
   Pro Ala Leu Tyr Tyr Met Asp Tyr Leu Ala Ala Leu Ala Lys Ala Pro
           115                 120                 125
   Phe Ala Ala His Gly Tyr Gly Ala Phe Leu Thr Leu Ser Ile Leu Asp
       130                 135                 140
   Arg Tyr Tyr Thr Pro Thr Ile Ser Arg Glu Arg Ala Val Glu Leu Leu
   145                 150                 155                 160
   Arg Lys Cys Leu Glu Glu Leu Gln Lys Arg Phe Ile Leu Asn Leu Pro
                   165                 170                 175
   Thr Phe Ser Val Arg Ile Ile Asp Lys Asn Gly Ile His Asp Leu Asp
               180                 185                 190
   Asn Ile Ser Phe Pro Lys Gln Gly Ser
           195                 200
<210> 34
   <211> 602
   <212> PRT
   <213> Homo sapiens
<220>
   <221> MISC_FEATURE
   <223> Protéine 4 associée au cytosquelette
<400> 34
   Met Pro Ser Ala Lys Gln Arg Gly Ser Lys Gly Gly His Gly Ala Ala
   1               5                   10                  15
   Ser Pro Ser Glu Lys Gly Ala His Pro Ser Gly Gly Ala Asp Asp Val
               20                  25                  30
   Ala Lys Lys Pro Pro Pro Ala Pro Gln Gln Pro Pro Pro Pro Pro Ala
           35                  40                  45
   Pro His Pro Gln Gln His Pro Gln Gln His Pro Gln Asn Gln Ala His
       50                  55                  60
   Gly Lys Gly Gly His Arg Gly Gly Gly Gly Gly Gly Gly Lys Ser Ser
   65                  70                  75                  80
   Ser Ser Ser Ser Ala Ser Ala Ala Ala Ala Ala Ala Ala Ala Ser Ser
                   85                  90                  95
   Ser Ala Ser Cys Ser Arg Arg Leu Gly Arg Ala Leu Asn Phe Leu Phe
               100                 105                 110
   Tyr Leu Ala Leu Val Ala Ala Ala Ala Phe Ser Gly Trp Cys Val His
           115                 120                 125
   His Val Leu Glu Glu Val Gln Gln Val Arg Arg Ser His Gln Asp Phe
       130                 135                 140
   Ser Arg Gln Arg Glu Glu Leu Gly Gln Gly Leu Gln Gly Val Glu Gln
   145                 150                 155                 160
   Lys Val Gln Ser Leu Gln Ala Thr Phe Gly Thr Phe Glu Ser Ile Leu
                   165                 170                 175
   Arg Ser Ser Gln His Lys Gln Asp Leu Thr Glu Lys Ala Val Lys Gln
               180                 185                 190
   Gly Glu Ser Glu Val Ser Arg Ile Ser Glu Val Leu Gln Lys Leu Gln
           195                 200                 205
   Asn Glu Ile Leu Lys Asp Leu Ser Asp Gly Ile His Val Val Lys Asp
       210                 215                 220
   Ala Arg Glu Arg Asp Phe Thr Ser Leu Glu Asn Thr Val Glu Glu Arg
   225                 230                 235                 240
   Leu Thr Glu Leu Thr Lys Ser Ile Asn Asp Asn Ile Ala Ile Phe Thr
   245 250 255
   Glu Val Gln Lys Arg Ser Gln Lys Glu Ile Asn Asp Met Lys Ala Lys
   260 265 270
   Val Ala Ser Leu Glu Glu Ser Glu Gly Asn Lys Gln Asp Leu Lys Ala
   275 280 285
   Leu Lys Glu Ala Val Lys Glu Ile Gln Thr Ser Ala Lys Ser Arg Glu
   290 295 300
   Trp Asp Met Glu Ala Leu Arg Ser Thr Leu Gln Thr Met Glu Ser Asp
   305 310 315 320
   Ile Tyr Thr Glu Val Arg Glu Leu Val Ser Leu Lys Gln Glu Gln Gln
   325 330 335
   Ala Phe Lys Glu Ala Ala Asp Thr Glu Arg Leu Ala Leu Gln Ala Leu
   340 345 350
   Thr Glu Lys Leu Leu Arg Ser Glu Glu Ser Val Ser Arg Leu Pro Glu
   355 360 365
   Glu Ile Arg Arg Leu Glu Glu Glu Leu Arg Gln Leu Lys Ser Asp Ser
   370 375 380
   His Gly Pro Lys Glu Asp Gly Gly Phe Arg His Ser Glu Ala Phe Glu
   385 390 395 400
   Ala Leu Gln Gln Lys Ser Gln Gly Leu Asp Ser Arg Leu Gln His Val
   405 410 415
   Glu Asp Gly Val Leu Ser Met Gln Val Ala Ser Ala Arg Gln Thr Glu
   420 425 430
   Ser Leu Glu Ser Leu Leu Ser Lys Ser Gln Glu His Glu Gln Arg Leu
   435 440 445
   Ala Ala Leu Gln Gly Arg Leu Glu Gly Leu Gly Ser Ser Glu Ala Asp
   450 455 460
   Gln Asp Gly Leu Ala Ser Thr Val Arg Ser Leu Gly Glu Thr Gln Leu
   465 470 475 480
   Val Leu Tyr Gly Asp Val Glu Glu Leu Lys Arg Ser Val Gly Glu Leu
   485 490 495
   Pro Ser Thr Val Glu Ser Leu Gln Lys Val Gln Glu Gln Val His Thr
   500 505 510
   Leu Leu Ser Gln Asp Gln Ala Gln Ala Ala Arg Leu Pro Pro Gln Asp
   515 520 525
   Phe Leu Asp Arg Leu Ser Ser Leu Asp Asn Leu Lys Ala Ser Val Ser
   530 535 540
   Gln Val Glu Ala Asp Leu Lys Met Leu Arg Thr Ala Val Asp Ser Leu
   545 550 555 560
   Val Ala Tyr Ser Val Lys Ile Glu Thr Asn Glu Asn Asn Leu Glu Ser
   565 570 575
   Ala Lys Gly Leu Leu Asp Asp Leu Arg Asn Asp Leu Asp Arg Leu Phe
   580 585 590
   Val Lys Val Glu Lys Ile His Glu Lys Val
   595 600
<210> 35
   <211> 367
   <212> PRT
   <213> Homo sapiens
<220>
   <221> MISC_FEATURE
   <223> Uroporphyrinogen decarboxylase
<400> 35
<210> 36
   <211> 180
   <212> PRT
   <213> Homo sapiens
<220>
   <221> MISC_FEATURE
   <223> Adenine phosphoribosyltransferase
<400> 36
<210> 37
   <211> 140
   <212> PRT
   <213> Homo sapiens
<220>
   <221> MISC_FEATURE
   <223> Profiline-1
<400> 37
<210> 38
   <211> 627
   <212> PRT
   <213> Homo sapiens
<220>
   <221> MISC_FEATURE
   <223> Plastine-3
<400> 38
<210> 39
   <211> 217
   <212> PRT
   <213> Homo sapiens
<220>
   <221> MISC_FEATURE
   <223> protéine 2 liée au récepteur du facteur de croissance
<400> 39
<210> 40
   <211> 589
   <212> PRT
   <213> Homo sapiens
<220>
   <221> MISC_FEATURE
   <223> ribonucléoprotéine L nucléaire hétérogène
<400> 40
   Met Ser Arg Arg Leu Leu Pro Arg Ala Glu Lys Arg Arg Arg Arg Leu
   1               5                   10                  15
   Glu Gln Arg Gln Gln Pro Asp Glu Gln Arg Arg Arg Ser Gly Ala Met
               20                  25                  30
   Val Lys Met Ala Ala Ala Gly Gly Gly Gly Gly Gly Gly Arg Tyr Tyr
           35                  40                  45
   Gly Gly Gly Ser Glu Gly Gly Arg Ala Pro Lys Arg Leu Lys Thr Asp
       50                  55                  60
   Asn Ala Gly Asp Gln His Gly Gly Gly Gly Gly Gly Gly Gly Gly Ala
   65                  70                  75                  80
   Gly Ala Ala Gly Gly Gly Gly Gly Gly Glu Asn Tyr Asp Asp Pro His
                   85                  90                  95
   Lys Thr Pro Ala Ser Pro Val Val His Ile Arg Gly Leu Ile Asp Gly
               100                 105                 110
   Val Val Glu Ala Asp Leu Val Glu Ala Leu Gln Glu Phe Gly Pro Ile
           115                 120                 125
   Ser Tyr Val Val Val Met Pro Lys Lys Arg Gln Ala Leu Val Glu Phe
       130                 135                 140
   Glu Asp Val Leu Gly Ala Cys Asn Ala Val Asn Tyr Ala Ala Asp Asn
   145                 150                 155                 160
   Gln Ile Tyr Ile Ala Gly His Pro Ala Phe Val Asn Tyr Ser Thr Ser
                   165                 170                 175
   Gln Lys Ile Ser Arg Pro Gly Asp Ser Asp Asp Ser Arg Ser Val Asn
               180                 185                 190
   Ser Val Leu Leu Phe Thr Ile Leu Asn Pro Ile Tyr Ser Ile Thr Thr
           195                 200                 205
   Asp Val Leu Tyr Thr Ile Cys Asn Pro Cys Gly Pro Val Gln Arg Ile
       210                 215                 220
   Val Ile Phe Arg Lys Asn Gly Val Gln Ala Met Val Glu Phe Asp Ser
   225                 230                 235                 240
   Val Gln Ser Ala Gln Arg Ala Lys Ala Ser Leu Asn Gly Ala Asp Ile
                   245                 250                 255
   Tyr Ser Gly Cys Cys Thr Leu Lys Ile Glu Tyr Ala Lys Pro Thr Arg
               260                 265                 270
   Leu Asn Val Phe Lys Asn Asp Gln Asp Thr Trp Asp Tyr Thr Asn Pro
           275                 280                 285
   Asn Leu Ser Gly Gln Gly Asp Pro Gly Ser Asn Pro Asn Lys Arg Gln
       290                 295                 300
   Arg Gln Pro Pro Leu Leu Gly Asp His Pro Ala Glu Tyr Gly Gly Pro
   305                 310                 315                 320
   His Gly Gly Tyr His Ser His Tyr His Asp Glu Gly Tyr Gly Pro Pro
                   325                 330                 335
   Pro Pro His Tyr Glu Gly Arg Arg Met Gly Pro Pro Val Gly Gly His
               340                 345                 350
   Arg Arg Gly Pro Ser Arg Tyr Gly Pro Gln Tyr Gly His Pro Pro Pro
           355                 360                 365
   Pro Pro Pro Pro Pro Glu Tyr Gly Pro His Ala Asp Ser Pro Val Leu
       370                 375                 380
   Met Val Tyr Gly Leu Asp Gln Ser Lys Met Asn Cys Asp Arg Val Phe
   385                 390                 395                 400
   Asn Val Phe Cys Leu Tyr Gly Asn Val Glu Lys Val Lys Phe Met Lys
                   405                 410                 415
   Ser Lys Pro Gly Ala Ala Met Val Glu Met Ala Asp Gly Tyr Ala Val
               420                 425                 430
   Asp Arg Ala Ile Thr His Leu Asn Asn Asn Phe Met Phe Gly Gln Lys
           435                 440                 445
   Leu Asn Val Cys Val Ser Lys Gln Pro Ala Ile Met Pro Gly Gln Ser
   450 455 460
   Tyr Gly Leu Glu Asp Gly Ser Cys Ser Tyr Lys Asp Phe Ser Glu Ser
   465 470 475 480
   Arg Asn Asn Arg Phe Ser Thr Pro Glu Gln Ala Ala Lys Asn Arg Ile
   485 490 495
   Gln His Pro Ser Asn Val Leu His Phe Phe Asn Ala Pro Leu Glu Val
   500 505 510
   Thr Glu Glu Asn Phe Phe Glu Ile Cys Asp Glu Leu Gly Val Lys Arg
   515 520 525
   Pro Ser Ser Val Lys Val Phe Ser Gly Lys Ser Glu Arg Ser Ser Ser
   530 535 540
   Gly Leu Leu Glu Trp Glu Ser Lys Ser Asp Ala Leu Glu Thr Leu Gly
   545 550 555 560
   Phe Leu Asn His Tyr Gln Met Lys Asn Pro Asn Gly Pro Tyr Pro Tyr
   565 570 575
   Thr Leu Lys Leu Cys Phe Ser Thr Ala Gln His Ala Ser
   580 585
<210> 41
   <211> 331
   <212> PRT
   <213> Homo sapiens
<220>
   <221> MISC_FEATURE
   <223> Précurseur de la Reticulocalbin-1
<400> 41
   Met Ala Arg Gly Gly Arg Gly Arg Arg Leu Gly Leu Ala Leu Gly Leu
   1 5 10 15
   Leu Leu Ala Leu Val Leu Ala Pro Arg Val Leu Arg Ala Lys Pro Thr
   20 25 30
   Val Arg Lys Glu Arg Val Val Arg Pro Asp Ser Glu Leu Gly Glu Arg
   35 40 45
   Pro Pro Glu Asp Asn Gln Ser Phe Gln Tyr Asp His Glu Ala Phe Leu
   50 55 60
   Gly Lys Glu Asp Ser Lys Thr Phe Asp Gln Leu Thr Pro Asp Glu Ser
   65                  70                  75                  80
   Lys Glu Arg Leu Gly Lys Ile Val Asp Arg Ile Asp Asn Asp Gly Asp
                   85                  90                  95
   Gly Phe Val Thr Thr Glu Glu Leu Lys Thr Trp Ile Lys Arg Val Gln
               100                 105                 110
   Lys Arg Tyr Ile Phe Asp Asn Val Ala Lys Val Trp Lys Asp Tyr Asp
           115                 120                 125
   Arg Asp Lys Asp Asp Lys Ile Ser Trp Glu Glu Tyr Lys Gln Ala Thr
       130                 135                 140
   Tyr Gly Tyr Tyr Leu Gly Asn Pro Ala Glu Phe His Asp Ser Ser Asp
   145                 150                 155                 160
   His His Thr Phe Lys Lys Met Leu Pro Arg Asp Glu Arg Arg Phe Lys
                   165                 170                 175
   Ala Ala Asp Leu Asn Gly Asp Leu Thr Ala Thr Arg Glu Glu Phe Thr
               180                 185                 190
   Ala Phe Leu His Pro Glu Glu Phe Glu His Met Lys Glu Ile Val Val
           195                 200                 205
   Leu Glu Thr Leu Glu Asp Ile Asp Lys Asn Gly Asp Gly Phe Val Asp
       210                 215                 220
   Gln Asp Glu Tyr Ile Ala Asp Met Phe Ser His Glu Glu Asn Gly Pro
   225                 230                 235                 240
   Glu Pro Asp Trp Val Leu Ser Glu Arg Glu Gln Phe Asn Glu Phe Arg
                   245                 250                 255
   Asp Leu Asn Lys Asp Gly Lys Leu Asp Lys Asp Glu Ile Arg His Trp
               260                 265                 270
   Ile Leu Pro Gln Asp Tyr Asp His Ala Gln Ala Glu Ala Arg His Leu
           275                 280                 285
   Val Tyr Glu Ser Asp Lys Asn Lys Asp Glu Lys Leu Thr Lys Glu Glu
       290                 295                 300
   Ile Leu Glu Asn Trp Asn Met Phe Val Gly Ser Gln Ala Thr Asn Tyr
   305 310 315 320
   Gly Glu Asp Leu Thr Lys Asn His Asp Glu Leu
   325 330
<210> 42
   <211> 376
   <212> PRT
   <213> Homo sapiens
<220>
   <221> MISC_FEATURE
   <223> Serpine B9
<400> 42
   Met Glu Thr Leu Ser Asn Ala Ser Gly Thr Phe Ala Ile Arg Leu Leu
   1 5 10 15
   Lys Ile Leu Cys Gln Asp Asn Pro Ser His Asn Val Phe Cys Ser Pro
   20 25 30
   Val Ser Ile Ser Ser Ala Leu Ala Met Val Leu Leu Gly Ala Lys Gly
   35 40 45
   Asn Thr Ala Thr Gln Met Ala Gln Ala Leu Ser Leu Asn Thr Glu Glu
   50 55 60
   Asp Ile His Arg Ala Phe Gln Ser Leu Leu Thr Glu Val Asn Lys Ala
   65 70 75 80
   Gly Thr Gln Tyr Leu Leu Arg Thr Ala Asn Arg Leu Phe Gly Glu Lys
   85 90 95
   Thr Cys Gln Phe Leu Ser Thr Phe Lys Glu Ser Cys Leu Gln Phe Tyr
   100 105 110
   His Ala Glu Leu Lys Glu Leu Ser Phe Ile Arg Ala Ala Glu Glu Ser
   115 120 125
   Arg Lys His Ile Asn Thr Trp Val Ser Lys Lys Thr Glu Gly Lys Ile
   130 135 140
   Glu Glu Leu Leu Pro Gly Ser Ser Ile Asp Ala Glu Thr Arg Leu Val
   145 150 155 160
   Leu Val Asn Ala Ile Tyr Phe Lys Gly Lys Trp Asn Glu Pro Phe Asp
                   165                 170                 175
   Glu Thr Tyr Thr Arg Glu Met Pro Phe Lys Ile Asn Gln Glu Glu Gln
               180                 185                 190
   Arg Pro Val Gln Met Met Tyr Gln Glu Ala Thr Phe Lys Leu Ala His
           195                 200                 205
   Val Gly Glu Val Arg Ala Gln Leu Leu Glu Leu Pro Tyr Ala Arg Lys
       210                 215                 220
   Glu Leu Ser Leu Leu Val Leu Leu Pro Asp Asp Gly Val Glu Leu Ser
   225                 230                 235                 240
   Thr Val Glu Lys Ser Leu Thr Phe Glu Lys Leu Thr Ala Trp Thr Lys
                   245                 250                 255
   Pro Asp Cys Met Lys Ser Thr Glu Val Glu Val Leu Leu Pro Lys Phe
               260                 265                 270
   Lys Leu Gln Glu Asp Tyr Asp Met Glu Ser Val Leu Arg His Leu Gly
           275                 280                 285
   Ile Val Asp Ala Phe Gln Gln Gly Lys Ala Asp Leu Ser Ala Met Ser
       290                 295                 300
   Ala Glu Arg Asp Leu Cys Leu Ser Lys Phe Val His Lys Ser Phe Val
   305                 310                 315                 320
   Glu Val Asn Glu Glu Gly Thr Glu Ala Ala Ala Ala Ser Ser Cys Phe
                   325                 330                 335
   Val Val Ala Glu Cys Cys Met Glu Ser Gly Pro Arg Phe Cys Ala Asp
               340                 345                 350
   His Pro Phe Leu Phe Phe Ile Arg His Asn Arg Ala Asn Ser Ile Leu
           355                 360                 365
   Phe Cys Gly Arg Phe Ser Ser Pro
       370                 375
<210> 43
   <211> 366
   <212> PRT
   <213> Homo sapiens
<220>
   <221> MISC_FEATURE
   <223> Précurseur mitochondrial de la sous-unité alpha de la Isocitrate dehydrogenase [NAD]
<400> 43
<210> 44
   <211> 693
   <212> PRT
   <213> Homo sapiens
<220>
   <221> MISC_FEATURE
   <223> GMP synthase [hydrolysant la glutamine]
<400> 44
   Met Ala Leu Cys Asn Gly Asp Ser Lys Leu Glu Asn Ala Gly Gly Asp
   1 5 10 15
   Leu Lys Asp Gly His His His Tyr Glu Gly Ala Val Val Ile Leu Asp
   20 25 30
   Ala Gly Ala Gln Tyr Gly Lys Val Ile Asp Arg Arg Val Arg Glu Leu
   35 40 45
   Phe Val Gln Ser Glu Ile Phe Pro Leu Glu Thr Pro Ala Phe Ala Ile
   50 55 60
   Lys Glu Gln Gly Phe Arg Ala Ile Ile Ile Ser Gly Gly Pro Asn Ser
   65 70 75 80
   Val Tyr Ala Glu Asp Ala Pro Trp Phe Asp Pro Ala Ile Phe Thr Ile
   85 90 95
   Gly Lys Pro Val Leu Gly Ile Cys Tyr Gly Met Gln Met Met Asn Lys
   100 105 110
   Val Phe Gly Gly Thr Val His Lys Lys Ser Val Arg Glu Asp Gly Val
   115 120 125
   Phe Asn Ile Ser Val Asp Asn Thr Cys Ser Leu Phe Arg Gly Leu Gln
   130 135 140
   Lys Glu Glu Val Val Leu Leu Thr His Gly Asp Ser Val Asp Lys Val
   145 150 155 160
   Ala Asp Gly Phe Lys Val Val Ala Arg Ser Gly Asn Ile Val Ala Gly
   165 170 175
   Ile Ala Asn Glu Ser Lys Lys Leu Tyr Gly Ala Gln Phe His Pro Glu
   180 185 190
   Val Gly Leu Thr Glu Asn Gly Lys Val Ile Leu Lys Asn Phe Leu Tyr
   195 200 205
   Asp Ile Ala Gly Cys Ser Gly Thr Phe Thr Val Gln Asn Arg Glu Leu
   210 215 220
   Glu Cys Ile Arg Glu Ile Lys Glu Arg Val Gly Thr Ser Lys Val Leu
   225 230 235 240
   Val Leu Leu Ser Gly Gly Val Asp Ser Thr Val Cys Thr Ala Leu Leu
   245 250 255
   Asn Arg Ala Leu Asn Gln Glu Gln Val Ile Ala Val His Ile Asp Asn
   260 265 270
   Gly Phe Met Arg Lys Arg Glu Ser Gln Ser Val Glu Glu Ala Leu Lys
   275 280 285
   Lys Leu Gly Ile Gln Val Lys Val Ile Asn Ala Ala His Ser Phe Tyr
       290                 295                 300
   Asn Gly Thr Thr Thr Leu Pro Ile Ser Asp Glu Asp Arg Thr Pro Arg
   305                 310                 315                 320
   Lys Arg Ile Ser Lys Thr Leu Asn Met Thr Thr Ser Pro Glu Glu Lys
                   325                 330                 335
   Arg Lys Ile Ile Gly Asp Thr Phe Val Lys Ile Ala Asn Glu Val Ile
               340                 345                 350
   Gly Glu Met Asn Leu Lys Pro Glu Glu Val Phe Leu Ala Gln Gly Thr
           355                 360                 365
   Leu Arg Pro Asp Leu Ile Glu Ser Ala Ser Leu Val Ala Ser Gly Lys
       370                 375                 380
   Ala Glu Leu Ile Lys Thr His His Asn Asp Thr Glu Leu Ile Arg Lys
   385                 390                 395                 400
   Leu Arg Glu Glu Gly Lys Val Ile Glu Pro Leu Lys Asp Phe His Lys
                   405                 410                 415
   Asp Glu Val Arg Ile Leu Gly Arg Glu Leu Gly Leu Pro Glu Glu Leu
               420                 425                 430
   Val Ser Arg His Pro Phe Pro Gly Pro Gly Leu Ala Ile Arg Val Ile
           435                 440                 445
   Cys Ala Glu Glu Pro Tyr Ile Cys Lys Asp Phe Pro Glu Thr Asn Asn
       450                 455                 460
   Ile Leu Lys Ile Val Ala Asp Phe Ser Ala Ser Val Lys Lys Pro His
   465                 470                 475                 480
   Thr Leu Leu Gln Arg Val Lys Ala Cys Thr Thr Glu Glu Asp Gln Glu
                   485                 490                 495
   Lys Leu Met Gln Ile Thr Ser Leu His Ser Leu Asn Ala Phe Leu Leu
               500                 505                 510
   Pro Ile Lys Thr Val Gly Val Gln Gly Asp Cys Arg Ser Tyr Ser Tyr
           515                 520                 525
   Val Cys Gly Ile Ser Ser Lys Asp Glu Pro Asp Trp Glu Ser Leu Ile
   530 535 540
   Phe Leu Ala Arg Leu Ile Pro Arg Met Cys His Asn Val Asn Arg Val
   545 550 555 560
   Val Tyr Ile Phe Gly Pro Pro Val Lys Glu Pro Pro Thr Asp Val Thr
   565 570 575
   Pro Thr Phe Leu Thr Thr Gly Val Leu Ser Thr Leu Arg Gln Ala Asp
   580 585 590
   Phe Glu Ala His Asn Ile Leu Arg Glu Ser Gly Tyr Ala Gly Lys Ile
   595 600 605
   Ser Gln Met Pro Val Ile Leu Thr Pro Leu His Phe Asp Arg Asp Pro
   610 615 620
   Leu Gln Lys Gln Pro Ser Cys Gln Arg Ser Val Val Ile Arg Thr Phe
   625 630 635 640
   Ile Thr Ser Asp Phe Met Thr Gly Ile Pro Ala Thr Pro Gly Asn Glu
   645 650 655
   Ile Pro Val Glu Val Val Leu Lys Met Val Thr Glu Ile Lys Lys Ile
   660 665 670
   Pro Gly Ile Ser Arg Ile Met Tyr Asp Leu Thr Ser Lys Pro Pro Gly
   675 680 685
   Thr Thr Glu Trp Glu
   690
<210> 45
   <211> 531
   <212> PRT
   <213> Homo sapiens
<220>
   <221> MISC_FEATURE
   <223> Sous-unité zeta de la protein 1 du complexe T
<400> 45
   Met Ala Ala Val Lys Thr Leu Asn Pro Lys Ala Glu Val Ala Arg Ala
   1 5 10 15
   Gln Ala Ala Leu Ala Val Asn Ile Ser Ala Ala Arg Gly Leu Gln Asp
   20 25 30
   Val Leu Arg Thr Asn Leu Gly Pro Lys Gly Thr Met Lys Met Leu Val
   35 40 45
   Ser Gly Ala Gly Asp Ile Lys Leu Thr Lys Asp Gly Asn Val Leu Leu
   50 55 60
   His Glu Met Gln Ile Gln His Pro Thr Ala Ser Leu Ile Ala Lys Val
   65 70 75 80
   Ala Thr Ala Gln Asp Asp Ile Thr Gly Asp Gly Thr Thr Ser Asn Val
   85 90 95
   Leu Ile Ile Gly Glu Leu Leu Lys Gln Ala Asp Leu Tyr Ile Ser Glu
   100 105 110
   Gly Leu His Pro Arg Ile Ile Thr Glu Gly Phe Glu Ala Ala Lys Glu
   115 120 125
   Lys Ala Leu Gln Phe Leu Glu Glu Val Lys Val Ser Arg Glu Met Asp
   130 135 140
   Arg Glu Thr Leu Ile Asp Val Ala Arg Thr Ser Leu Arg Thr Lys Val
   145 150 155 160
   His Ala Glu Leu Ala Asp Val Leu Thr Glu Ala Val Val Asp Ser Ile
   165 170 175
   Leu Ala Ile Lys Lys Gln Asp Glu Pro Ile Asp Leu Phe Met Ile Glu
   180 185 190
   Ile Met Glu Met Lys His Lys Ser Glu Thr Asp Thr Ser Leu Ile Arg
   195 200 205
   Gly Leu Val Leu Asp His Gly Ala Arg His Pro Asp Met Lys Lys Arg
   210 215 220
   Val Glu Asp Ala Tyr Ile Leu Thr Cys Asn Val Ser Leu Glu Tyr Glu
   225 230 235 240
   Lys Thr Glu Val Asn Ser Gly Phe Phe Tyr Lys Ser Ala Glu Glu Arg
   245 250 255
   Glu Lys Leu Val Lys Ala Glu Arg Lys Phe Ile Glu Asp Arg Val Lys
   260 265 270
   Lys Ile Ile Glu Leu Lys Arg Lys Val Cys Gly Asp Ser Asp Lys Gly
   275 280 285
   Phe Val Val Ile Asn Gln Lys Gly Ile Asp Pro Phe Ser Leu Asp Ala
   290 295 300
   Leu Ser Lys Glu Gly Ile Val Ala Leu Arg Arg Ala Lys Arg Arg Asn
   305 310 315 320
   Met Glu Arg Leu Thr Leu Ala Cys Gly Gly Val Ala Leu Asn Ser Phe
   325 330 335
   Asp Asp Leu Ser Pro Asp Cys Leu Gly His Ala Gly Leu Val Tyr Glu
   340 345 350
   Tyr Thr Leu Gly Glu Glu Lys Phe Thr Phe Ile Glu Lys Cys Asn Asn
   355 360 365
   Pro Arg Ser Val Thr Leu Leu Ile Lys Gly Pro Asn Lys His Thr Leu
   370 375 380
   Thr Gln Ile Lys Asp Ala Val Arg Asp Gly Leu Arg Ala Val Lys Asn
   385 390 395 400
   Ala Ile Asp Asp Gly Cys Val Val Pro Gly Ala Gly Ala Val Glu Val
   405 410 415
   Ala Met Ala Glu Ala Leu Ile Lys His Lys Pro Ser Val Lys Gly Arg
   420 425 430
   Ala Gln Leu Gly Val Gln Ala Phe Ala Asp Ala Leu Leu Ile Ile Pro
   435 440 445
   Lys Val Leu Ala Gln Asn Ser Gly Phe Asp Leu Gln Glu Thr Leu Val
   450 455 460
   Lys Ile Gln Ala Glu His Ser Glu Ser Gly Gln Leu Val Gly Val Asp
   465 470 475 480
   Leu Asn Thr Gly Glu Pro Met Val Ala Ala Glu Val Gly Val Trp Asp
   485 490 495
   Asn Tyr Cys Val Lys Lys Gln Leu Leu His Ser Cys Thr Val Ile Ala
   500 505 510
   Thr Asn Ile Leu Leu Val Asp Glu Ile Met Arg Ala Gly Met Ser Ser
   515 520 525
   Leu Lys Gly
   530
<210> 46
   <211> 166
   <212> PRT
   <213> Homo sapiens
<220>
   <221> MISC_FEATURE
   <223> Cofiline-1
<400> 46
   Met Ala Ser Gly Val Ala Val Ser Asp Gly Val Ile Lys Val Phe Asn
   1 5 10 15
   Asp Met Lys Val Arg Lys Ser Ser Thr Pro Glu Glu Val Lys Lys Arg
   20 25 30
   Lys Lys Ala Val Leu Phe Cys Leu Ser Glu Asp Lys Lys Asn Ile Ile
   35 40 45
   Leu Glu Glu Gly Lys Glu Ile Leu Val Gly Asp Val Gly Gln Thr Val
   50 55 60
   Asp Asp Pro Tyr Ala Thr Phe Val Lys Met Leu Pro Asp Lys Asp Cys
   65 70 75 80
   Arg Tyr Ala Leu Tyr Asp Ala Thr Tyr Glu Thr Lys Glu Ser Lys Lys
   85 90 95
   Glu Asp Leu Val Phe Ile Phe Trp Ala Pro Glu Ser Ala Pro Leu Lys
   100 105 110
   Ser Lys Met Ile Tyr Ala Ser Ser Lys Asp Ala Ile Lys Lys Lys Leu
   115 120 125
   Thr Gly Ile Lys His Glu Leu Gln Ala Asn Cys Tyr Glu Glu Val Lys
   130 135 140
   Asp Arg Cys Thr Leu Ala Glu Lys Leu Gly Gly Ser Ala Val Ile Ser
   145 150 155 160
   Leu Glu Gly Lys Pro Leu
   165
<210> 47
   <211> 780
   <212> PRT
   <213> Homo sapiens
<220>
   <221> MISC_FEATURE
   <223> Précurseur mitochondrial de l'aconitate hydratase
<400> 47
<210> 48
   <211> 758
   <212> PRT
   <213> Homo sapiens
<220>
   <221> MISC_FEATURE
   <223> Protéine de la membrane interne des mitochondries
<400> 48
   Met Leu Arg Ala Cys Gln Leu Ser Gly Val Thr Ala Ala Ala Gln Ser
   1 5 10 15
   Cys Leu Cys Gly Lys Phe Val Leu Arg Pro Leu Arg Pro Cys Arg Arg
   20 25 30
   Tyr Ser Thr Ser Gly Ser Ser Gly Leu Thr Thr Gly Lys Ile Ala Gly
   35 40 45
   Ala Gly Leu Leu Phe Val Gly Gly Gly Ile Gly Gly Thr Ile Leu Tyr
   50 55 60
   Ala Lys Trp Asp Ser His Phe Arg Glu Ser Val Glu Lys Thr Ile Pro
   65 70 75 80
   Tyr Ser Asp Lys Leu Phe Glu Met Val Leu Gly Pro Ala Ala Tyr Asn
   85 90 95
   Val Pro Leu Pro Lys Lys Ser Ile Gln Ser Gly Pro Leu Lys Ile Ser
   100 105 110
   Ser Val Ser Glu Val Met Lys Glu Ser Lys Gln Pro Ala Ser Gln Leu
   115 120 125
   Gln Lys Gln Lys Gly Asp Thr Pro Ala Ser Ala Thr Ala Pro Thr Glu
   130 135 140
   Ala Ala Gln Ile Ile Ser Ala Ala Gly Asp Thr Leu Ser Val Pro Ala
   145 150 155 160
   Pro Ala Val Gln Pro Glu Glu Ser Leu Lys Thr Asp His Pro Glu Ile
   165 170 175
   Gly Glu Gly Lys Pro Thr Pro Ala Leu Ser Glu Glu Ala Ser Ser Ser
   180 185 190
   Ser Ile Arg Glu Arg Pro Pro Glu Glu Val Ala Ala Arg Leu Ala Gln
   195 200 205
   Gln Glu Lys Gln Glu Gln Val Lys Ile Glu Ser Leu Ala Lys Ser Leu
   210 215 220
   Glu Asp Ala Leu Arg Gln Thr Ala Ser Val Thr Leu Gln Ala Ile Ala
   225 230 235 240
   Ala Gln Asn Ala Ala Val Gln Ala Val Asn Ala His Ser Asn Ile Leu
   245 250 255
   Lys Ala Ala Met Asp Asn Ser Glu Ile Ala Gly Glu Lys Lys Ser Ala
   260 265 270
   Gln Trp Arg Thr Val Glu Gly Ala Leu Lys Glu Arg Arg Lys Ala Val
   275 280 285
   Asp Glu Ala Ala Asp Ala Leu Leu Lys Ala Lys Glu Glu Leu Glu Lys
   290 295 300
   Met Lys Ser Val Ile Glu Asn Ala Lys Lys Lys Glu Val Ala Gly Ala
   305 310 315 320
   Lys Pro His Ile Thr Ala Ala Glu Gly Lys Leu His Asn Met Ile Val
                   325                 330                 335
   Asp Leu Asp Asn Val Val Lys Lys Val Gln Ala Ala Gln Ser Glu Ala
               340                 345                 350
   Lys Val Val Ser Gln Tyr His Glu Leu Val Val Gln Ala Arg Asp Asp
           355                 360                 365
   Phe Lys Arg Glu Leu Asp Ser Ile Thr Pro Glu Val Leu Pro Gly Trp
       370                 375                 380
   Lys Gly Met Ser Val Ser Asp Leu Ala Asp Lys Leu Ser Thr Asp Asp
   385                 390                 395                 400
   Leu Asn Ser Leu Ile Ala His Ala His Arg Arg Ile Asp Gln Leu Asn
                   405                 410                 415
   Arg Glu Leu Ala Glu Gln Lys Ala Thr Glu Lys Gln His Ile Thr Leu
               420                 425                 430
   Ala Leu Glu Lys Gln Lys Leu Glu Glu Lys Arg Ala Phe Asp Ser Ala
           435                 440                 445
   Val Ala Lys Ala Leu Glu His His Arg Ser Glu Ile Gln Ala Glu Gln
       450                 455                 460
   Asp Arg Lys Ile Glu Glu Val Arg Asp Ala Met Glu Asn Glu Met Arg
   465                 470                 475                 480
   Thr Gln Leu Arg Arg Gln Ala Ala Ala His Thr Asp His Leu Arg Asp
                   485                 490                 495
   Val Leu Arg Val Gln Glu Gln Glu Leu Lys Ser Glu Phe Glu Gln Asn
               500                 505                 510
   Leu Ser Glu Lys Leu Ser Glu Gln Glu Leu Gln Phe Arg Arg Leu Ser
           515                 520                 525
   Gln Glu Gln Val Asp Asn Phe Thr Leu Asp Ile Asn Thr Ala Tyr Ala
       530                 535                 540
   Arg Leu Arg Gly Ile Glu Gln Ala Val Gln Ser His Ala Val Ala Glu
   545                 550                 555                 560
   Glu Glu Ala Arg Lys Ala His Gln Leu Trp Leu Ser Val Glu Ala Leu
                   565                 570                 575
   Lys Tyr Ser Met Lys Thr Ser Ser Ala Glu Thr Pro Thr Ile Pro Leu
                   580                 585                 590
   Gly Ser Ala Val Glu Ala Ile Lys Ala Asn Cys Ser Asp Asn Glu Phe
           595                 600                 605
   Thr Gln Ala Leu Thr Ala Ala Ile Pro Pro Glu Ser Leu Thr Arg Gly
       610                 615                 620
   Val Tyr Ser Glu Glu Thr Leu Arg Ala Arg Phe Tyr Ala Val Gln Lys
   625                 630                 635                 640
   Leu Ala Arg Arg Val Ala Met Ile Asp Glu Thr Arg Asn Ser Leu Tyr
                   645                 650                 655
   Gln Tyr Phe Leu Ser Tyr Leu Gln Ser Leu Leu Leu Phe Pro Pro Gln
               660                 665                 670
   Gln Leu Lys Pro Pro Pro Glu Leu Cys Pro Glu Asp Ile Asn Thr Phe
           675                 680                 685
   Lys Leu Leu Ser Tyr Ala Ser Tyr Cys Ile Glu His Gly Asp Leu Glu
       690                 695                 700
   Leu Ala Ala Lys Phe Val Asn Gln Leu Lys Gly Glu Ser Arg Arg Val
   705                 710                 715                 720
   Ala Gln Asp Trp Leu Lys Glu Ala Arg Met Thr Leu Glu Thr Lys Gln
                   725                 730                 735
   Ile Val Glu Ile Leu Thr Ala Tyr Ala Ser Ala Val Gly Ile Gly Thr
               740                 745                 750
   Thr Gln Val Gln Pro Glu
           755
<210> 49
   <211> 463
   <212> PRT
   <213> Homo sapiens
<220>
   <221> MISC_FEATURE
   <223> ribonucleoprotéine K nucléaire hétérogène
<400> 49
   Met Glu Thr Glu Gln Pro Glu Glu Thr Phe Pro Asn Thr Glu Thr Asn
   1               5                   10                  15
   Gly Glu Phe Gly Lys Arg Pro Ala Glu Asp Met Glu Glu Glu Gln Ala
               20                  25                  30
   Phe Lys Arg Ser Arg Asn Thr Asp Glu Met Val Glu Leu Arg Ile Leu
           35                  40                  45
   Leu Gln Ser Lys Asn Ala Gly Ala Val Ile Gly Lys Gly Gly Lys Asn
       50                  55                  60
   Ile Lys Ala Leu Arg Thr Asp Tyr Asn Ala Ser Val Ser Val Pro Asp
   65                  70                  75                  80
   Ser Ser Gly Pro Glu Arg Ile Leu Ser Ile Ser Ala Asp Ile Glu Thr
                   85                  90                  95
   Ile Gly Glu Ile Leu Lys Lys Ile Ile Pro Thr Leu Glu Glu Gly Leu
               100                 105                 110
   Gln Leu Pro Ser Pro Thr Ala Thr Ser Gln Leu Pro Leu Glu Ser Asp
           115                 120                 125
   Ala Val Glu Cys Leu Asn Tyr Gln His Tyr Lys Gly Ser Asp Phe Asp
       130                 135                 140
   Cys Glu Leu Arg Leu Leu Ile His Gln Ser Leu Ala Gly Gly Ile Ile
   145                 150                 155                 160
   Gly Val Lys Gly Ala Lys Ile Lys Glu Leu Arg Glu Asn Thr Gln Thr
                   165                 170                 175
   Thr Ile Lys Leu Phe Gln Glu Cys Cys Pro His Ser Thr Asp Arg Val
               180                 185                 190
   Val Leu Ile Gly Gly Lys Pro Asp Arg Val Val Glu Cys Ile Lys Ile
           195                 200                 205
   Ile Leu Asp Leu Ile Ser Glu Ser Pro Ile Lys Gly Arg Ala Gln Pro
       210                 215                 220
   Tyr Asp Pro Asn Phe Tyr Asp Glu Thr Tyr Asp Tyr Gly Gly Phe Thr
   225                 230                 235                 240
   Met Met Phe Asp Asp Arg Arg Gly Arg Pro Val Gly Phe Pro Met Arg
                   245                 250                 255
   Gly Arg Gly Gly Phe Asp Arg Met Pro Pro Gly Arg Gly Gly Arg Pro
               260                 265                 270
   Met Pro Pro Ser Arg Arg Asp Tyr Asp Asp Met Ser Pro Arg Arg Gly
           275                 280                 285
   Pro Pro Pro Pro Pro Pro Gly Arg Gly Gly Arg Gly Gly Ser Arg Ala
       290                 295                 300
   Arg Asn Leu Pro Leu Pro Pro Pro Pro Pro Pro Arg Gly Gly Asp Leu
   305                 310                 315                 320
   Met Ala Tyr Asp Arg Arg Gly Arg Pro Gly Asp Arg Tyr Asp Gly Met
                   325                 330                 335
   Val Gly Phe Ser Ala Asp Glu Thr Trp Asp Ser Ala Ile Asp Thr Trp
               340                 345                 350
   Ser Pro Ser Glu Trp Gln Met Ala Tyr Glu Pro Gln Gly Gly Ser Gly
           355                 360                 365
   Tyr Asp Tyr Ser Tyr Ala Gly Gly Arg Gly Ser Tyr Gly Asp Leu Gly
       370                 375                 380
   Gly Pro Ile Ile Thr Thr Gln Val Thr Ile Pro Lys Asp Leu Ala Gly
   385                 390                 395                 400
   Ser Ile Ile Gly Lys Gly Gly Gln Arg Ile Lys Gln Ile Arg His Glu
                   405                 410                 415
   Ser Gly Ala Ser Ile Lys Ile Asp Glu Pro Leu Glu Gly Ser Glu Asp
               420                 425                 430
   Arg Ile Ile Thr Ile Thr Gly Thr Gln Asp Gln Ile Gln Asn Ala Gln
           435                 440                 445
   Tyr Leu Leu Gln Asn Ser Val Lys Gln Tyr Ser Gly Lys Phe Phe
       450                 455                 460
<210> 50
   <211> 452
   <212> PRT
   <213> Homo sapiens
<220>
   <221> MISC_FEATURE
   <223> Précurseur mitochondrial du facteur d'élongation Tu
<400> 50
   Met Ala Ala Ala Thr Leu Leu Arg Ala Thr Pro His Phe Ser Gly Leu
   1               5                   10                  15
   Ala Ala Gly Arg Thr Phe Leu Leu Gln Gly Leu Leu Arg Leu Leu Lys
               20                  25                  30
   Ala Pro Ala Leu Pro Leu Leu Cys Arg Gly Leu Ala Val Glu Ala Lys
           35                  40                  45
   Lys Thr Tyr Val Arg Asp Lys Pro His Val Asn Val Gly Thr Ile Gly
       50                  55                  60
   His Val Asp His Gly Lys Thr Thr Leu Thr Ala Ala Ile Thr Lys Ile
   65                  70                  75                  80
   Leu Ala Glu Gly Gly Gly Ala Lys Phe Lys Lys Tyr Glu Glu Ile Asp
                   85                  90                  95
   Asn Ala Pro Glu Glu Arg Ala Arg Gly Ile Thr Ile Asn Ala Ala His
               100                 105                 110
   Val Glu Tyr Ser Thr Ala Ala Arg His Tyr Ala His Thr Asp Cys Pro
           115                 120                 125
   Gly His Ala Asp Tyr Val Lys Asn Met Ile Thr Gly Thr Ala Pro Leu
       130                 135                 140
   Asp Gly Cys Ile Leu Val Val Ala Ala Asn Asp Gly Pro Met Pro Gln
   145                 150                 155                 160
   Thr Arg Glu His Leu Leu Leu Ala Arg Gln Ile Gly Val Glu His Val
                   165                 170                 175
   Val Val Tyr Val Asn Lys Ala Asp Ala Val Gln Asp Ser Glu Met Val
               180                 185                 190
   Glu Leu Val Glu Leu Glu Ile Arg Glu Leu Leu Thr Glu Phe Gly Tyr
           195                 200                 205
   Lys Gly Glu Glu Thr Pro Val Ile Val Gly Ser Ala Leu Cys Ala Leu
       210                 215                 220
   Glu Gly Arg Asp Pro Glu Leu Gly Leu Lys Ser Val Gln Lys Leu Leu
   225 230 235 240
   Asp Ala Val Asp Thr Tyr Ile Pro Val Pro Ala Arg Asp Leu Glu Lys
   245 250 255
   Pro Phe Leu Leu Pro Val Glu Ala Val Tyr Ser Val Pro Gly Arg Gly
   260 265 270
   Thr Val Val Thr Gly Thr Leu Glu Arg Gly Ile Leu Lys Lys Gly Asp
   275 280 285
   Glu Cys Glu Leu Leu Gly His Ser Lys Asn Ile Arg Thr Val Val Thr
   290 295 300
   Gly Ile Glu Met Phe His Lys Ser Leu Glu Arg Ala Glu Ala Gly Asp
   305 310 315 320
   Asn Leu Gly Ala Leu Val Arg Gly Leu Lys Arg Glu Asp Leu Arg Arg
   325 330 335
   Gly Leu Val Met Val Lys Pro Gly Ser Ile Lys Pro His Gln Lys Val
   340 345 350
   Glu Ala Gln Val Tyr Ile Leu Ser Lys Glu Glu Gly Gly Arg His Lys
   355 360 365
   Pro Phe Val Ser His Phe Met Pro Val Met Phe Ser Leu Thr Trp Asp
   370 375 380
   Met Ala Cys Arg Ile Ile Leu Pro Pro Glu Lys Glu Leu Ala Met Pro
   385 390 395 400
   Gly Glu Asp Leu Lys Phe Asn Leu Ile Leu Arg Gln Pro Met Ile Leu
   405 410 415
   Glu Lys Gly Gln Arg Phe Thr Leu Arg Asp Gly Asn Arg Thr Ile Gly
   420 425 430
   Thr Gly Leu Val Thr Asn Thr Leu Ala Met Thr Glu Glu Glu Lys Asn
   435 440 445
   Ile Lys Trp Gly
   450
<210> 51
   <211> 325
   <212> PRT
   <213> Homo sapiens
<220>
   <221> MISC_FEATURE
   <223> Alcohol dehydrogenase [NADP+]
<400> 51
   Met Ala Ala Ser Cys Val Leu Leu His Thr Gly Gln Lys Met Pro Leu
   1               5                   10                  15
   Ile Gly Leu Gly Thr Trp Lys Ser Glu Pro Gly Gln Val Lys Ala Ala
               20                  25                  30
   Val Lys Tyr Ala Leu Ser Val Gly Tyr Arg His Ile Asp Cys Ala Ala
           35                  40                  45
   Ile Tyr Gly Asn Glu Pro Glu Ile Gly Glu Ala Leu Lys Glu Asp Val
       50                  55                  60
   Gly Pro Gly Lys Ala Val Pro Arg Glu Glu Leu Phe Val Thr Ser Lys
   65                  70                  75                  80
   Leu Trp Asn Thr Lys His His Pro Glu Asp Val Glu Pro Ala Leu Arg
                   85                  90                  95
   Lys Thr Leu Ala Asp Leu Gln Leu Glu Tyr Leu Asp Leu Tyr Leu Met
               100                 105                 110
   His Trp Pro Tyr Ala Phe Glu Arg Gly Asp Asn Pro Phe Pro Lys Asn
           115                 120                 125
   Ala Asp Gly Thr Ile Cys Tyr Asp Ser Thr His Tyr Lys Glu Thr Trp
       130                 135                 140
   Lys Ala Leu Glu Ala Leu Val Ala Lys Gly Leu Val Gln Ala Leu Gly
   145                 150                 155                 160
   Leu Ser Asn Phe Asn Ser Arg Gln Ile Asp Asp Ile Leu Ser Val Ala
                   165                 170                 175
   Ser Val Arg Pro Ala Val Leu Gln Val Glu Cys His Pro Tyr Leu Ala
               180                 185                 190
   Gln Asn Glu Leu Ile Ala His Cys Gln Ala Arg Gly Leu Glu Val Thr
   195 200 205
   Ala Tyr Ser Pro Leu Gly Ser Ser Asp Arg Ala Trp Arg Asp Pro Asp
   210 215 220
   Glu Pro Val Leu Leu Glu Glu Pro Val Val Leu Ala Leu Ala Glu Lys
   225 230 235 240
   Tyr Gly Arg Ser Pro Ala Gln Ile Leu Leu Arg Trp Gln Val Gln Arg
   245 250 255
   Lys Val Ile Cys Ile Pro Lys Ser Ile Thr Pro Ser Arg Ile Leu Gln
   260 265 270
   Asn Ile Lys Val Phe Asp Phe Thr Phe Ser Pro Glu Glu Met Lys Gln
   275 280 285
   Leu Asn Ala Leu Asn Lys Asn Trp Arg Tyr Ile Val Pro Met Leu Thr
   290 295 300
   Val Asp Gly Lys Arg Val Pro Arg Asp Ala Gly His Pro Leu Tyr Pro
   305 310 315 320
   Phe Asn Asp Pro Tyr
   325
<210> 52
   <211> 359
   <212> PRT
   <213> Homo sapiens
<220>
   <221> MISC_FEATURE
   <223> Sialic acid synthase
<400> 52
   Met Pro Leu Glu Leu Glu Leu Cys Pro Gly Arg Trp Val Gly Gly Gln
   1 5 10 15
   His Pro Cys Phe Ile Ile Ala Glu Ile Gly Gln Asn His Gln Gly Asp
   20 25 30
   Leu Asp Val Ala Lys Arg Met Ile Arg Met Ala Lys Glu Cys Gly Ala
   35 40 45
   Asp Cys Ala Lys Phe Gln Lys Ser Glu Leu Glu Phe Lys Phe Asn Arg
   50 55 60
   Lys Ala Leu Glu Arg Pro Tyr Thr Ser Lys His Ser Trp Gly Lys Thr
   65 70 75 80
   Tyr Gly Glu His Lys Arg His Leu Glu Phe Ser His Asp Gln Tyr Arg
   85 90 95
   Glu Leu Gln Arg Tyr Ala Glu Glu Val Gly Ile Phe Phe Thr Ala Ser
   100 105 110
   Gly Met Asp Glu Met Ala Val Glu Phe Leu His Glu Leu Asn Val Pro
   115 120 125
   Phe Phe Lys Val Gly Ser Gly Asp Thr Asn Asn Phe Pro Tyr Leu Glu
   130 135 140
   Lys Thr Ala Lys Lys Gly Arg Pro Met Val Ile Ser Ser Gly Met Gln
   145 150 155 160
   Ser Met Asp Thr Met Lys Gln Val Tyr Gln Ile Val Lys Pro Leu Asn
   165 170 175
   Pro Asn Phe Cys Phe Leu Gln Cys Thr Ser Ala Tyr Pro Leu Gln Pro
   180 185 190
   Glu Asp Val Asn Leu Arg Val Ile Ser Glu Tyr Gln Lys Leu Phe Pro
   195 200 205
   Asp Ile Pro Ile Gly Tyr Ser Gly His Glu Thr Gly Ile Ala Ile Ser
   210 215 220
   Val Ala Ala Val Ala Leu Gly Ala Lys Val Leu Glu Arg His Ile Thr
   225 230 235 240
   Leu Asp Lys Thr Trp Lys Gly Ser Asp His Ser Ala Ser Leu Glu Pro
   245 250 255
   Gly Glu Leu Ala Glu Leu Val Arg Ser Val Arg Leu Val Glu Arg Ala
   260 265 270
   Leu Gly Ser Pro Thr Lys Gln Leu Leu Pro Cys Glu Met Ala Cys Asn
   275 280 285
   Glu Lys Leu Gly Lys Ser Val Val Ala Lys Val Lys Ile Pro Glu Gly
   290 295 300
   Thr Ile Leu Thr Met Asp Met Leu Thr Val Lys Val Gly Glu Pro Lys
   305 310 315 320
   Gly Tyr Pro Pro Glu Asp Ile Phe Asn Leu Val Gly Lys Lys Val Leu
   325 330 335
   Val Thr Val Glu Glu Asp Asp Thr Ile Met Glu Glu Leu Val Asp Asn
   340 345 350
   His Gly Lys Lys Ile Lys Ser
   355
<210> 53
   <211> 282
   <212> PRT
   <213> Homo sapiens
<220>
   <221> MISC_FEATURE
   <223> S-formylglutathione hydrolase
<400> 53
   Met Ala Leu Lys Gln Ile Ser Ser Asn Lys Cys Phe Gly Gly Leu Gln
   1 5 10 15
   Lys Val Phe Glu His Asp Ser Val Glu Leu Asn Cys Lys Met Lys Phe
   20 25 30
   Ala Val Tyr Leu Pro Pro Lys Ala Glu Thr Gly Lys Cys Pro Ala Leu
   35 40 45
   Tyr Trp Leu Ser Gly Leu Thr Cys Thr Glu Gln Asn Phe Ile Ser Lys
   50 55 60
   Ser Gly Tyr His Gln Ser Ala Ser Glu His Gly Leu Val Val Ile Ala
   65 70 75 80
   Pro Asp Thr Ser Pro Arg Gly Cys Asn Ile Lys Gly Glu Asp Glu Ser
   85 90 95
   Trp Asp Phe Gly Thr Gly Ala Gly Phe Tyr Val Asp Ala Thr Glu Asp
   100 105 110
   Pro Trp Lys Thr Asn Tyr Arg Met Tyr Ser Tyr Val Thr Glu Glu Leu
   115 120 125
   Pro Gln Leu Ile Asn Ala Asn Phe Pro Val Asp Pro Gln Arg Met Ser
   130 135 140
   Ile Phe Gly His Ser Met Gly Gly His Gly Ala Leu Ile Cys Ala Leu
   145 150 155 160
   Lys Asn Pro Gly Lys Tyr Lys Ser Val Ser Ala Phe Ala Pro Ile Cys
   165 170 175
   Asn Pro Val Leu Cys Pro Trp Gly Lys Lys Ala Phe Ser Gly Tyr Leu
   180 185 190
   Gly Thr Asp Gln Ser Lys Trp Lys Ala Tyr Asp Ala Thr His Leu Val
   195 200 205
   Lys Ser Tyr Pro Gly Ser Gln Leu Asp Ile Leu Ile Asp Gln Gly Lys
   210 215 220
   Asp Asp Gln Phe Leu Leu Asp Gly Gln Leu Leu Pro Asp Asn Phe Ile
   225 230 235 240
   Ala Ala Cys Thr Glu Lys Lys Ile Pro Val Val Phe Arg Leu Gln Glu
   245 250 255
   Gly Tyr Asp His Ser Tyr Tyr Phe Ile Ala Thr Phe Ile Thr Asp His
   260 265 270
   Ile Arg His His Ala Lys Tyr Leu Asn Ala
   275 280
<210> 54
   <211> 317
   <212> PRT
   <213> Homo sapiens
<220>
   <221> MISC_FEATURE
   <223> sous-unité beta-2-like 1 de la protéine de liaison au nucleotide guanine
<400> 54
<210> 55
   <211> 289
   <212> PRT
   <213> Homo sapiens
<220>
   <221> MISC_FEATURE
   <223> Purine nucleoside phosphorylase
<400> 55
<210> 56
   <211> 272
   <212> PRT
   <213> Homo sapiens
<220>
   <221> MISC_FEATURE
   <223> Prohibitine
<400> 56
   Met Ala Ala Lys Val Phe Glu Ser Ile Gly Lys Phe Gly Leu Ala Leu
   1 5 10 15
   Ala Val Ala Gly Gly Val Val Asn Ser Ala Leu Tyr Asn Val Asp Ala
   20 25 30
   Gly His Arg Ala Val Ile Phe Asp Arg Phe Arg Gly Val Gln Asp Ile
   35 40 45
   Val Val Gly Glu Gly Thr His Phe Leu Ile Pro Trp Val Gln Lys Pro
   50 55 60
   Ile Ile Phe Asp Cys Arg Ser Arg Pro Arg Asn Val Pro Val Ile Thr
   65 70 75 80
   Gly Ser Lys Asp Leu Gln Asn Val Asn Ile Thr Leu Arg Ile Leu Phe
   85 90 95
   Arg Pro Val Ala Ser Gln Leu Pro Arg Ile Phe Thr Ser Ile Gly Glu
   100 105 110
   Asp Tyr Asp Glu Arg Val Leu Pro Ser Ile Thr Thr Glu Ile Leu Lys
   115 120 125
   Ser Val Val Ala Arg Phe Asp Ala Gly Glu Leu Ile Thr Gln Arg Glu
   130 135 140
   Leu Val Ser Arg Gln Val Ser Asp Asp Leu Thr Glu Arg Ala Ala Thr
   145 150 155 160
   Phe Gly Leu Ile Leu Asp Asp Val Ser Leu Thr His Leu Thr Phe Gly
   165 170 175
   Lys Glu Phe Thr Glu Ala Val Glu Ala Lys Gln Val Ala Gln Gln Glu
   180 185 190
   Ala Glu Arg Ala Arg Phe Val Val Glu Lys Ala Glu Gln Gln Lys Lys
   195 200 205
   Ala Ala Ile Ile Ser Ala Glu Gly Asp Ser Lys Ala Ala Glu Leu Ile
   210 215 220
   Ala Asn Ser Leu Ala Thr Ala Gly Asp Gly Leu Ile Glu Leu Arg Lys
   225 230 235 240
   Leu Glu Ala Ala Glu Asp Ile Ala Tyr Gln Leu Ser Arg Ser Arg Asn
   245 250 255
   Ile Thr Tyr Leu Pro Ala Gly Gln Ser Val Leu Leu Gln Leu Pro Gln
   260 265 270
<210> 57
   <211> 282
   <212> PRT
   <213> Homo sapiens
<220>
   <221> MISC_FEATURE
   <223> Précurseur mitochondrial de la protéine de liaison au sous composant Q du composant 1 du complément
<400> 57
   Met Leu Pro Leu Leu Arg Cys Val Pro Arg Val Leu Gly Ser Ser Val
   1               5                   10                  15
   Ala Gly Leu Arg Ala Ala Ala Pro Ala Ser Pro Phe Arg Gln Leu Leu
               20                  25                  30
   Gln Pro Ala Pro Arg Leu Cys Thr Arg Pro Phe Gly Leu Leu Ser Val
           35                  40                  45
   Arg Ala Gly Ser Glu Arg Arg Pro Gly Leu Leu Arg Pro Arg Gly Pro
       50                  55                  60
   Cys Ala Cys Gly Cys Gly Cys Gly Ser Leu His Thr Asp Gly Asp Lys
   65                  70                  75                  80
   Ala Phe Val Asp Phe Leu Ser Asp Glu Ile Lys Glu Glu Arg Lys Ile
                   85                  90                  95
   Gln Lys His Lys Thr Leu Pro Lys Met Ser Gly Gly Trp Glu Leu Glu
               100                 105                 110
   Leu Asn Gly Thr Glu Ala Lys Leu Val Arg Lys Val Ala Gly Glu Lys
           115                 120                 125
   Ile Thr Val Thr Phe Asn Ile Asn Asn Ser Ile Pro Pro Thr Phe Asp
       130                 135                 140
   Gly Glu Glu Glu Pro Ser Gln Gly Gln Lys Val Glu Glu Gln Glu Pro
   145                 150                 155                 160
   Glu Leu Thr Ser Thr Pro Asn Phe Val Val Glu Val Ile Lys Asn Asp
                   165                 170                 175
   Asp Gly Lys Lys Ala Leu Val Leu Asp Cys His Tyr Pro Glu Asp Glu
               180                 185                 190
   Val Gly Gln Glu Asp Glu Ala Glu Ser Asp Ile Phe Ser Ile Arg Glu
           195                 200                 205
   Val Ser Phe Gln Ser Thr Gly Glu Ser Glu Trp Lys Asp Thr Asn Tyr
   210 215 220
   Thr Leu Asn Thr Asp Ser Leu Asp Trp Ala Leu Tyr Asp His Leu Met
   225 230 235 240
   Asp Phe Leu Ala Asp Arg Gly Val Asp Asn Thr Phe Ala Asp Glu Leu
   245 250 255
   Val Glu Leu Ser Thr Ala Leu Glu His Gln Glu Tyr Ile Thr Phe Leu
   260 265 270
   Glu Asp Leu Lys Ser Phe Val Lys Ser Gln
   275 280
<210> 58
   <211> 806
   <212> PRT
   <213> Homo sapiens
<220>
   <221> MISC_FEATURE
   <223> ATPase du réticulum endoplasmique
<400> 58
<210> 59
   <211> 152
   <212> PRT
   <213> Homo sapiens
<220>
   <221> MISC_FEATURE
   <223> Nucleoside diphosphate kinase A
<400> 59
   Met Ala Asn Cys Glu Arg Thr Phe Ile Ala Ile Lys Pro Asp Gly Val
   1               5                   10                  15
   Gln Arg Gly Leu Val Gly Glu Ile Ile Lys Arg Phe Glu Gln Lys Gly
               20                  25                  30
   Phe Arg Leu Val Gly Leu Lys Phe Met Gln Ala Ser Glu Asp Leu Leu
           35                  40                  45
   Lys Glu His Tyr Val Asp Leu Lys Asp Arg Pro Phe Phe Ala Gly Leu
       50                  55                  60
   Val Lys Tyr Met His Ser Gly Pro Val Val Ala Met Val Trp Glu Gly
   65                  70                  75                  80
   Leu Asn Val Val Lys Thr Gly Arg Val Met Leu Gly Glu Thr Asn Pro
                   85                  90                  95
   Ala Asp Ser Lys Pro Gly Thr Ile Arg Gly Asp Phe Cys Ile Gln Val
               100                 105                 110
   Gly Arg Asn Ile Ile His Gly Ser Asp Ser Val Glu Ser Ala Glu Lys
           115                 120                 125
   Glu Ile Gly Leu Trp Phe His Pro Glu Glu Leu Val Asp Tyr Thr Ser
       130                 135                 140
   Cys Ala Gln Asn Trp Ile Tyr Glu
   145                 150
<210> 60
   <211> 339
   <212> PRT
   <213> Homo sapiens
<400> 60
   Met Ser Thr Val His Glu Ile Leu Cys Lys Leu Ser Leu Glu Gly Asp
   1               5                   10                  15
   His Ser Thr Pro Pro Ser Ala Tyr Gly Ser Val Lys Ala Tyr Thr Asn
               20                  25                  30
   Phe Asp Ala Glu Arg Asp Ala Leu Asn Ile Glu Thr Ala Ile Lys Thr
   35 40 45
   Lys Gly Val Asp Glu Val Thr Ile Val Asn Ile Leu Thr Asn Arg Ser
   50 55 60
   Asn Ala Gln Arg Gln Asp Ile Ala Phe Ala Tyr Gln Arg Arg Thr Lys
   65 70 75 80
   Lys Glu Leu Ala Ser Ala Leu Lys Ser Ala Leu Ser Gly His Leu Glu
   85 90 95
   Thr Val Ile Leu Gly Leu Leu Lys Thr Pro Ala Gln Tyr Asp Ala Ser
   100 105 110
   Glu Leu Lys Ala Ser Met Lys Gly Leu Gly Thr Asp Glu Asp Ser Leu
   115 120 125
   Ile Glu Ile Ile Cys Ser Arg Thr Asn Gln Glu Leu Gln Glu Ile Asn
   130 135 140
   Arg Val Tyr Lys Glu Met Tyr Lys Thr Asp Leu Glu Lys Asp Ile Ile
   145 150 155 160
   Ser Asp Thr Ser Gly Asp Phe Arg Lys Leu Met Val Ala Leu Ala Lys
   165 170 175
   Gly Arg Arg Ala Glu Asp Gly Ser Val Ile Asp Tyr Glu Leu Ile Asp
   180 185 190
   Gln Asp Ala Arg Asp Leu Tyr Asp Ala Gly Val Lys Arg Lys Gly Thr
   195 200 205
   Asp Val Pro Lys Trp Ile Ser Ile Met Thr Glu Arg Ser Val Pro His
   210 215 220
   Leu Gln Lys Val Phe Asp Arg Tyr Lys Ser Tyr Ser Pro Tyr Asp Met
   225 230 235 240
   Leu Glu Ser Ile Arg Lys Glu Val Lys Gly Asp Leu Glu Asn Ala Phe
   245 250 255
   Leu Asn Leu Val Gln Cys Ile Gln Asn Lys Pro Leu Tyr Phe Ala Asp
   260 265 270
   Arg Leu Tyr Asp Ser Met Lys Gly Lys Gly Thr Arg Asp Lys Val Leu
   275 280 285
   Ile Arg Ile Met Val Ser Arg Ser Glu Val Asp Met Leu Lys Ile Arg
   290 295 300
   Ser Glu Phe Lys Arg Lys Tyr Gly Lys Ser Leu Tyr Tyr Tyr Ile Gln
   305 310 315 320
   Gln Asp Thr Lys Gly Asp Tyr Gln Lys Ala Leu Leu Tyr Leu Cys Gly
   325 330 335
   Gly Asp Asp
<210> 61
   <211> 434
   <212> PRT
   <213> Homo sapiens
<400> 61
   Met Ser Ile Leu Lys Ile His Ala Arg Glu Ile Phe Asp Ser Arg Gly
   1 5 10 15
   Asn Pro Thr Val Glu Val Asp Leu Phe Thr Ser Lys Gly Leu Phe Arg
   20 25 30
   Ala Ala Val Pro Ser Gly Ala Ser Thr Gly Ile Tyr Glu Ala Leu Glu
   35 40 45
   Leu Arg Asp Asn Asp Lys Thr Arg Tyr Met Gly Lys Gly Val Ser Lys
   50 55 60
   Ala Val Glu His Ile Asn Lys Thr Ile Ala Pro Ala Leu Val Ser Lys
   65 70 75 80
   Lys Leu Asn Val Thr Glu Gln Glu Lys Ile Asp Lys Leu Met Ile Glu
   85 90 95
   Met Asp Gly Thr Glu Asn Lys Ser Lys Phe Gly Ala Asn Ala Ile Leu
   100 105 110
   Gly Val Ser Leu Ala Val Cys Lys Ala Gly Ala Val Glu Lys Gly Val
   115 120 125
   Pro Leu Tyr Arg His Ile Ala Asp Leu Ala Gly Asn Ser Glu Val Ile
   130 135 140
   Leu Pro Val Pro Ala Phe Asn Val Ile Asn Gly Gly Ser His Ala Gly
   145 150 155 160
   Asn Lys Leu Ala Met Gln Glu Phe Met Ile Leu Pro Val Gly Ala Ala
   165 170 175
   Asn Phe Arg Glu Ala Met Arg Ile Gly Ala Glu Val Tyr His Asn Leu
   180 185 190
   Lys Asn Val Ile Lys Glu Lys Tyr Gly Lys Asp Ala Thr Asn Val Gly
   195 200 205
   Asp Glu Gly Gly Phe Ala Pro Asn Ile Leu Glu Asn Lys Glu Gly Leu
   210 215 220
   Glu Leu Leu Lys Thr Ala Ile Gly Lys Ala Gly Tyr Thr Asp Lys Val
   225 230 235 240
   Val Ile Gly Met Asp Val Ala Ala Ser Glu Phe Phe Arg Ser Gly Lys
   245 250 255
   Tyr Asp Leu Asp Phe Lys Ser Pro Asp Asp Pro Ser Arg Tyr Ile Ser
   260 265 270
   Pro Asp Gln Leu Ala Asp Leu Tyr Lys Ser Phe Ile Lys Asp Tyr Pro
   275 280 285
   Val Val Ser Ile Glu Asp Pro Phe Asp Gln Asp Asp Trp Gly Ala Trp
   290 295 300
   Gln Lys Phe Thr Ala Ser Ala Gly Ile Gln Val Val Gly Asp Asp Leu
   305 310 315 320
   Thr Val Thr Asn Pro Lys Arg Ile Ala Lys Ala Val Asn Glu Lys Ser
   325 330 335
   Cys Asn Cys Leu Leu Leu Lys Val Asn Gln Ile Gly Ser Val Thr Glu
   340 345 350
   Ser Leu Gln Ala Cys Lys Leu Ala Gln Ala Asn Gly Trp Gly Val Met
   355 360 365
   Val Ser His Arg Ser Gly Glu Thr Glu Asp Thr Phe Ile Ala Asp Leu
   370 375 380
   Val Val Gly Leu Cys Thr Gly Gln Ile Lys Thr Gly Ala Pro Cys Arg
   385 390 395 400
   Ser Glu Arg Leu Ala Lys Tyr Asn Gln Leu Leu Arg Ile Glu Glu Glu
   405 410 415
   Leu Gly Ser Lys Ala Lys Phe Ala Gly Arg Asn Phe Arg Asn Pro Leu
   420 425 430
   Ala Lys
<210> 62
   <211> 644
   <212> PRT
   <213> Homo sapiens
<400> 62
   Met Ala Asp Tyr Ser Thr Val Pro Pro Pro Ser Ser Gly Ser Ala Gly
   1 5 10 15
   Gly Gly Gly Gly Gly Gly Gly Gly Gly Gly Val Asn Asp Ala Phe Lys
   20 25 30
   Asp Ala Leu Gln Arg Ala Arg Gln Ile Ala Ala Lys Ile Gly Gly Asp
   35 40 45
   Ala Gly Thr Ser Leu Asn Ser Asn Asp Tyr Gly Tyr Gly Gly Gln Lys
   50 55 60
   Arg Pro Leu Glu Asp Gly Asp Gln Pro Asp Ala Lys Lys Val Ala Pro
   65 70 75 80
   Gln Asn Asp Ser Phe Gly Thr Gln Leu Pro Pro Met His Gln Gln Gln
   85 90 95
   Ser Arg Ser Val Met Thr Glu Glu Tyr Lys Val Pro Asp Gly Met Val
   100 105 110
   Gly Phe Ile Ile Gly Arg Gly Gly Glu Gln Ile Ser Arg Ile Gln Gln
   115 120 125
   Glu Ser Gly Cys Lys Ile Gln Ile Ala Pro Asp Ser Gly Gly Leu Pro
   130 135 140
   Glu Arg Ser Cys Met Leu Thr Gly Thr Pro Glu Ser Val Gln Ser Ala
   145 150 155 160
   Lys Arg Leu Leu Asp Gln Ile Val Glu Lys Gly Arg Pro Ala Pro Gly
   165 170 175
   Phe His His Gly Asp Gly Pro Gly Asn Ala Val Gln Glu Ile Met Ile
   180 185 190
   Pro Ala Ser Lys Ala Gly Leu Val Ile Gly Lys Gly Gly Glu Thr Ile
   195 200 205
   Lys Gln Leu Gln Glu Arg Ala Gly Val Lys Met Val Met Ile Gln Asp
   210 215 220
   Gly Pro Gln Asn Thr Gly Ala Asp Lys Pro Leu Arg Ile Thr Gly Asp
   225 230 235 240
   Pro Tyr Lys Val Gln Gln Ala Lys Glu Met Val Leu Glu Leu Ile Arg
   245 250 255
   Asp Gln Gly Gly Phe Arg Glu Val Arg Asn Glu Tyr Gly Ser Arg Ile
   260 265 270
   Gly Gly Asn Glu Gly Ile Asp Val Pro Ile Pro Arg Phe Ala Val Gly
   275 280 285
   Ile Val Ile Gly Arg Asn Gly Glu Met Ile Lys Lys Ile Gln Asn Asp
   290 295 300
   Ala Gly Val Arg Ile Gln Phe Lys Pro Asp Asp Gly Thr Thr Pro Glu
   305 310 315 320
   Arg Ile Ala Gln Ile Thr Gly Pro Pro Asp Arg Cys Gln His Ala Ala
   325 330 335
   Glu Ile Ile Thr Asp Leu Leu Arg Ser Val Gln Ala Gly Asn Pro Gly
   340 345 350
   Gly Pro Gly Pro Gly Gly Arg Gly Arg Gly Arg Gly Gln Gly Asn Trp
   355 360 365
   Asn Met Gly Pro Pro Gly Gly Leu Gln Glu Phe Asn Phe Ile Val Pro
   370 375 380
   Thr Gly Lys Thr Gly Leu Ile Ile Gly Lys Gly Gly Glu Thr Ile Lys
   385 390 395 400
   Ser Ile Ser Gln Gln Ser Gly Ala Arg Ile Glu Leu Gln Arg Asn Pro
   405 410 415
   Pro Pro Asn Ala Asp Pro Asn Met Lys Leu Phe Thr Ile Arg Gly Thr
   420 425 430
   Pro Gln Gln Ile Asp Tyr Ala Arg Gln Leu Ile Glu Glu Lys Ile Gly
   435 440 445
   Gly Pro Val Asn Pro Leu Gly Pro Pro Val Pro His Gly Pro His Gly
   450 455 460
   Val Pro Gly Pro His Gly Pro Pro Gly Pro Pro Gly Pro Gly Thr Pro
   465 470 475 480
   Met Gly Pro Tyr Asn Pro Ala Pro Tyr Asn Pro Gly Pro Pro Gly Pro
   485 490 495
   Ala Pro His Gly Pro Pro Ala Pro Tyr Ala Pro Gln Gly Trp Gly Asn
   500 505 510
   Ala Tyr Pro His Trp Gln Gln Gln Ala Pro Pro Asp Pro Ala Lys Ala
   515 520 525
   Gly Thr Asp Pro Asn Ser Ala Ala Trp Ala Ala Tyr Tyr Ala His Tyr
   530 535 540
   Tyr Gln Gln Gln Ala Gln Pro Pro Pro Ala Ala Pro Ala Gly Ala Pro
   545 550 555 560
   Thr Thr Thr Gln Thr Asn Gly Gln Gly Asp Gln Gln Asn Pro Ala Pro
   565 570 575
   Ala Gly Gln Val Asp Tyr Thr Lys Ala Trp Glu Glu Tyr Tyr Lys Lys
   580 585 590
   Met Gly Gln Ala Val Pro Ala Pro Thr Gly Ala Pro Pro Gly Gly Gln
   595 600 605
   Pro Asp Tyr Ser Ala Ala Trp Ala Glu Tyr Tyr Arg Gln Gln Ala Ala
   610 615 620
   Tyr Tyr Ala Gln Thr Ser Pro Gln Gly Met Pro Gln His Pro Pro Ala
   625 630 635 640
   Pro Gln Gly Gln
<210> 63
   <211> 509
   <212> PRT
   <213> Homo sapiens
<400> 63
<210> 64
   <211> 514
   <212> PRT
   <213> Homo sapiens
<400> 64
   Met Ala Asp Tyr Leu Ile Ser Gly Gly Thr Ser Tyr Val Pro Asp Asp
   1               5                   10                  15
   Gly Leu Thr Ala Gln Gln Leu Phe Asn Cys Gly Asp Gly Leu Thr Tyr
               20                  25                  30
   Asn Asp Phe Leu Ile Leu Pro Gly Tyr Ile Asp Phe Thr Ala Asp Gln
           35                  40                  45
   Val Asp Leu Thr Ser Ala Leu Thr Lys Lys Ile Thr Leu Lys Thr Pro
       50                  55                  60
   Leu Val Ser Ser Pro Met Asp Thr Val Thr Glu Ala Gly Met Ala Ile
   65                  70                  75                  80
   Ala Met Ala Leu Thr Gly Gly Ile Gly Phe Ile His His Asn Cys Thr
                   85                  90                  95
   Pro Glu Phe Gln Ala Asn Glu Val Arg Lys Val Lys Lys Tyr Glu Gln
               100                 105                 110
   Gly Phe Ile Thr Asp Pro Val Val Leu Ser Pro Lys Asp Arg Val Arg
           115                 120                 125
   Asp Val Phe Glu Ala Lys Ala Arg His Gly Phe Cys Gly Ile Pro Ile
       130                 135                 140
   Thr Asp Thr Gly Arg Met Gly Ser Arg Leu Val Gly Ile Ile Ser Ser
   145                 150                 155                 160
   Arg Asp Ile Asp Phe Leu Lys Glu Glu Glu His Asp Cys Phe Leu Glu
   165 170 175
   Glu Ile Met Thr Lys Arg Glu Asp Leu Val Val Ala Pro Ala Gly Ile
   180 185 190
   Thr Leu Lys Glu Ala Asn Glu Ile Leu Gln Arg Ser Lys Lys Gly Lys
   195 200 205
   Leu Pro Ile Val Asn Glu Asp Asp Glu Leu Val Ala Ile Ile Ala Arg
   210 215 220
   Thr Asp Leu Lys Lys Asn Arg Asp Tyr Pro Leu Ala Ser Lys Asp Ala
   225 230 235 240
   Lys Lys Gln Leu Leu Cys Gly Ala Ala Ile Gly Thr His Glu Asp Asp
   245 250 255
   Lys Tyr Arg Leu Asp Leu Leu Ala Gln Ala Gly Val Asp Val Val Val
   260 265 270
   Leu Asp Ser Ser Gln Gly Asn Ser Ile Phe Gln Ile Asn Met Ile Lys
   275 280 285
   Tyr Ile Lys Asp Lys Tyr Pro Asn Leu Gln Val Ile Gly Gly Asn Val
   290 295 300
   Val Thr Ala Ala Gln Ala Lys Asn Leu Ile Asp Ala Gly Val Asp Ala
   305 310 315 320
   Leu Arg Val Gly Met Gly Ser Gly Ser Ile Cys Ile Thr Gln Glu Val
   325 330 335
   Leu Ala Cys Gly Arg Pro Gln Ala Thr Ala Val Tyr Lys Val Ser Glu
   340 345 350
   Tyr Ala Arg Arg Phe Gly Val Pro Val Ile Ala Asp Gly Gly Ile Gln
   355 360 365
   Asn Val Gly His Ile Ala Lys Ala Leu Ala Leu Gly Ala Ser Thr Val
   370 375 380
   Met Met Gly Ser Leu Leu Ala Ala Thr Thr Glu Ala Pro Gly Glu Tyr
   385 390 395 400
   Phe Phe Ser Asp Gly Ile Arg Leu Lys Lys Tyr Arg Gly Met Gly Ser
   405 410 415
   Leu Asp Ala Met Asp Lys His Leu Ser Ser Gln Asn Arg Tyr Phe Ser
   420 425 430
   Glu Ala Asp Lys Ile Lys Val Ala Gln Gly Val Ser Gly Ala Val Gln
   435 440 445
   Asp Lys Gly Ser Ile His Lys Phe Val Pro Tyr Leu Ile Ala Gly Ile
   450 455 460
   Gln His Ser Cys Gln Asp Ile Gly Ala Lys Ser Leu Thr Gln Val Arg
   465 470 475 480
   Ala Met Met Tyr Ser Gly Glu Leu Lys Phe Glu Lys Arg Thr Ser Ser
   485 490 495
   Ala Gln Val Glu Gly Gly Val His Ser Leu His Ser Tyr Glu Lys Arg
   500 505 510
   Leu Phe
<210> 65
   <211> 510
   <212> PRT
   <213> Homo sapiens
<400> 65
<210> 66
   <211> 355
   <212> PRT
   <213> Homo sapiens
<400> 66
   Met Ser Glu Glu Gln Phe Gly Gly Asp Gly Ala Ala Ala Ala Ala Thr
   1 5 10 15
   Ala Ala Val Gly Gly Ser Ala Gly Glu Gln Glu Gly Ala Met Val Ala
   20 25 30
   Ala Thr Gln Gly Ala Ala Ala Ala Ala Gly Ser Gly Ala Gly Thr Gly
   35 40 45
   Gly Gly Thr Ala Ser Gly Gly Thr Glu Gly Gly Ser Ala Glu Ser Glu
   50 55 60
   Gly Ala Lys Ile Asp Ala Ser Lys Asn Glu Glu Asp Glu Gly His Ser
   65 70 75 80
   Asn Ser Ser Pro Arg His Ser Glu Ala Ala Thr Ala Gln Arg Glu Glu
   85 90 95
   Trp Lys Met Phe Ile Gly Gly Leu Ser Trp Asp Thr Thr Lys Lys Asp
   100 105 110
   Leu Lys Asp Tyr Phe Ser Lys Phe Gly Glu Val Val Asp Cys Thr Leu
   115 120 125
   Lys Leu Asp Pro Ile Thr Gly Arg Ser Arg Gly Phe Gly Phe Val Leu
   130 135 140
   Phe Lys Glu Ser Glu Ser Val Asp Lys Val Met Asp Gln Lys Glu His
   145 150 155 160
   Lys Leu Asn Gly Lys Val Ile Asp Pro Lys Arg Ala Lys Ala Met Lys
   165 170 175
   Thr Lys Glu Pro Val Lys Lys Ile Phe Val Gly Gly Leu Ser Pro Asp
   180 185 190
   Thr Pro Glu Glu Lys Ile Arg Glu Tyr Phe Gly Gly Phe Gly Glu Val
   195 200 205
   Glu Ser Ile Glu Leu Pro Met Asp Asn Lys Thr Asn Lys Arg Arg Gly
   210 215 220
   Phe Cys Phe Ile Thr Phe Lys Glu Glu Glu Pro Val Lys Lys Ile Met
   225 230 235 240
   Glu Lys Lys Tyr His Asn Val Gly Leu Ser Lys Cys Glu Ile Lys Val
   245 250 255
   Ala Met Ser Lys Glu Gln Tyr Gln Gln Gln Gln Gln Trp Gly Ser Arg
   260 265 270
   Gly Gly Phe Ala Gly Arg Ala Arg Gly Arg Gly Gly Gly Pro Ser Gln
   275 280 285
   Asn Trp Asn Gln Gly Tyr Ser Asn Tyr Trp Asn Gln Gly Tyr Gly Asn
   290 295 300
   Tyr Gly Tyr Asn Ser Gln Gly Tyr Gly Gly Tyr Gly Gly Tyr Asp Tyr
   305 310 315 320
   Thr Gly Tyr Asn Asn Tyr Tyr Gly Tyr Gly Asp Tyr Ser Asn Gln Gln
   325 330 335
   Ser Gly Tyr Gly Lys Val Ser Arg Arg Gly Gly His Gln Asn Ser Tyr
   340 345 350
   Lys Pro Tyr
   355
<210> 67
   <211> 329
   <212> PRT
   <213> Homo sapiens
<400> 67
   Met Thr Thr Gln Gln Ile Asp Leu Gln Gly Pro Gly Pro Trp Gly Phe
   1 5 10 15
   Arg Leu Val Gly Gly Lys Asp Phe Glu Gln Pro Leu Ala Ile Ser Arg
   20 25 30
   Val Thr Pro Gly Ser Lys Ala Ala Leu Ala Asn Leu Cys Ile Gly Asp
   35 40 45
   Val Ile Thr Ala Ile Asp Gly Glu Asn Thr Ser Asn Met Thr His Leu
   50 55 60
   Glu Ala Gln Asn Arg Ile Lys Gly Cys Thr Asp Asn Leu Thr Leu Thr
   65 70 75 80
   Val Ala Arg Ser Glu His Lys Val Trp Ser Pro Leu Val Thr Glu Glu
   85 90 95
   Gly Lys Arg His Pro Tyr Lys Met Asn Leu Ala Ser Glu Pro Gln Glu
   100 105 110
   Val Leu His Ile Gly Ser Ala His Asn Arg Ser Ala Met Pro Phe Thr
   115 120 125
   Ala Ser Pro Ala Ser Ser Thr Thr Ala Arg Val Ile Thr Asn Gln Tyr
   130 135 140
   Asn Asn Pro Ala Gly Leu Tyr Ser Ser Glu Asn Ile Ser Asn Phe Asn
   145 150 155 160
   Asn Ala Leu Glu Ser Lys Thr Ala Ala Ser Gly Val Glu Ala Asn Ser
   165 170 175
   Arg Pro Leu Asp His Ala Gln Pro Pro Ser Ser Leu Val Ile Asp Lys
   180 185 190
   Glu Ser Glu Val Tyr Lys Met Leu Gln Glu Lys Gln Glu Leu Asn Glu
   195 200 205
   Pro Pro Lys Gln Ser Thr Ser Phe Leu Val Leu Gln Glu Ile Leu Glu
   210 215 220
   Ser Glu Glu Lys Gly Asp Pro Asn Lys Pro Ser Gly Phe Arg Ser Val
   225 230 235 240
   Lys Ala Pro Val Thr Lys Val Ala Ala Ser Ile Gly Asn Ala Gln Lys
   245 250 255
   Leu Pro Met Cys Asp Lys Cys Gly Thr Gly Ile Val Gly Val Phe Val
   260 265 270
   Lys Leu Arg Asp Arg His Arg His Pro Glu Cys Tyr Val Cys Thr Asp
   275 280 285
   Cys Gly Thr Asn Leu Lys Gln Lys Gly His Phe Phe Val Glu Asp Gln
   290 295 300
   Ile Tyr Cys Glu Lys His Ala Arg Glu Arg Val Thr Pro Pro Glu Gly
   305 310 315 320
   Tyr Glu Val Val Thr Val Phe Pro Lys
   325
<210> 68
   <211> 317
   <212> PRT
   <213> Homo sapiens
<400> 68
   Met Pro Arg Glu Asp Arg Ala Thr Trp Lys Ser Asn Tyr Phe Leu Lys
   1               5                   10                  15
   Ile Ile Gln Leu Leu Asp Asp Tyr Pro Lys Cys Phe Ile Val Gly Ala
               20                  25                  30
   Asp Asn Val Gly Ser Lys Gln Met Gln Gln Ile Arg Met Ser Leu Arg
           35                  40                  45
   Gly Lys Ala Val Val Leu Met Gly Lys Asn Thr Met Met Arg Lys Ala
       50                  55                  60
   Ile Arg Gly His Leu Glu Asn Asn Pro Ala Leu Glu Lys Leu Leu Pro
   65                  70                  75                  80
   His Ile Arg Gly Asn Val Gly Phe Val Phe Thr Lys Glu Asp Leu Thr
                   85                  90                  95
   Glu Ile Arg Asp Met Leu Leu Ala Asn Lys Val Pro Ala Ala Ala Arg
               100                 105                 110
   Ala Gly Ala Ile Ala Pro Cys Glu Val Thr Val Pro Ala Gln Asn Thr
           115                 120                 125
   Gly Leu Gly Pro Glu Lys Thr Ser Phe Phe Gln Ala Leu Gly Ile Thr
       130                 135                 140
   Thr Lys Ile Ser Arg Gly Thr Ile Glu Ile Leu Ser Asp Val Gln Leu
   145                 150                 155                 160
   Ile Lys Thr Gly Asp Lys Val Gly Ala Ser Glu Ala Thr Leu Leu Asn
                   165                 170                 175
   Met Leu Asn Ile Ser Pro Phe Ser Phe Gly Leu Val Ile Gln Gln Val
               180                 185                 190
   Phe Asp Asn Gly Ser Ile Tyr Asn Pro Glu Val Leu Asp Ile Thr Glu
           195                 200                 205
   Glu Thr Leu His Ser Arg Phe Leu Glu Gly Val Arg Asn Val Ala Ser
       210                 215                 220
   Val Cys Leu Gln Ile Gly Tyr Pro Thr Val Ala Ser Val Pro His Ser
   225                 230                 235                 240
   Ile Ile Asn Gly Tyr Lys Arg Val Leu Ala Leu Ser Val Glu Thr Asp
                   245                 250                 255
   Tyr Thr Phe Pro Leu Ala Glu Lys Val Lys Ala Phe Leu Ala Asp Pro
   260 265 270
   Ser Ala Phe Val Ala Ala Ala Pro Val Ala Ala Ala Thr Thr Ala Ala
   275 280 285
   Pro Ala Ala Ala Ala Ala Pro Ala Lys Val Glu Ala Lys Glu Glu Ser
   290 295 300
   Glu Glu Ser Asp Glu Asp Met Gly Phe Gly Leu Phe Asp
   305 310 315
<210> 69
   <211> 294
   <212> PRT
   <213> Homo sapiens
<400> 69
   Met Ala Thr His Gly Gln Thr Cys Ala Arg Pro Met Cys Ile Pro Pro
   1 5 10 15
   Ser Tyr Ala Asp Leu Gly Lys Ala Ala Arg Asp Ile Phe Asn Lys Gly
   20 25 30
   Phe Gly Phe Gly Leu Val Lys Leu Asp Val Lys Thr Lys Ser Cys Ser
   35 40 45
   Gly Val Glu Phe Ser Thr Ser Gly Ser Ser Asn Thr Asp Thr Gly Lys
   50 55 60
   Val Thr Gly Thr Leu Glu Thr Lys Tyr Lys Trp Cys Glu Tyr Gly Leu
   65 70 75 80
   Thr Phe Thr Glu Lys Trp Asn Thr Asp Asn Thr Leu Gly Thr Glu Ile
   85 90 95
   Ala Ile Glu Asp Gln Ile Cys Gln Gly Leu Lys Leu Thr Phe Asp Thr
   100 105 110
   Thr Phe Ser Pro Asn Thr Gly Lys Lys Ser Gly Lys Ile Lys Ser Ser
   115 120 125
   Tyr Lys Arg Glu Cys Ile Asn Leu Gly Cys Asp Val Asp Phe Asp Phe
   130 135 140
   Ala Gly Pro Ala Ile His Gly Ser Ala Val Phe Gly Tyr Glu Gly Trp
   145 150 155 160
   Leu Ala Gly Tyr Gln Met Thr Phe Asp Ser Ala Lys Ser Lys Leu Thr
   165 170 175
   Arg Asn Asn Phe Ala Val Gly Tyr Arg Thr Gly Asp Phe Gln Leu His
   180 185 190
   Thr Asn Val Asn Asp Gly Thr Glu Phe Gly Gly Ser Ile Tyr Gln Lys
   195 200 205
   Val Cys Glu Asp Leu Asp Thr Ser Val Asn Leu Ala Trp Thr Ser Gly
   210 215 220
   Thr Asn Cys Thr Arg Phe Gly Ile Ala Ala Lys Tyr Gln Leu Asp Pro
   225 230 235 240
   Thr Ala Ser Ile Ser Ala Lys Val Asn Asn Ser Ser Leu Ile Gly Val
   245 250 255
   Gly Tyr Thr Gln Thr Leu Arg Pro Gly Val Lys Leu Thr Leu Ser Ala
   260 265 270
   Leu Val Asp Gly Lys Ser Ile Asn Ala Gly Gly His Lys Val Gly Leu
   275 280 285
   Ala Leu Glu Leu Glu Ala
   290
<210> 70
   <211> 189
   <212> PRT
   <213> Homo sapiens
<400> 70
<210> 71
   <211> 165
   <212> PRT
   <213> Homo sapiens
<400> 71
   Met Val Asn Pro Thr Val Phe Phe Asp Ile Ala Val Asp Gly Glu Pro
   1 5 10 15
   Leu Gly Arg Val Ser Phe Glu Leu Phe Ala Asp Lys Val Pro Lys Thr
   20 25 30
   Ala Glu Asn Phe Arg Ala Leu Ser Thr Gly Glu Lys Gly Phe Gly Tyr
   35 40 45
   Lys Gly Ser Cys Phe His Arg Ile Ile Pro Gly Phe Met Cys Gln Gly
   50 55 60
   Gly Asp Phe Thr Arg His Asn Gly Thr Gly Gly Lys Ser Ile Tyr Gly
   65 70 75 80
   Glu Lys Phe Glu Asp Glu Asn Phe Ile Leu Lys His Thr Gly Pro Gly
   85 90 95
   Ile Leu Ser Met Ala Asn Ala Gly Pro Asn Thr Asn Gly Ser Gln Phe
   100 105 110
   Phe Ile Cys Thr Ala Lys Thr Glu Trp Leu Asp Gly Lys His Val Val
   115 120 125
   Phe Gly Lys Val Lys Glu Gly Met Asn Ile Val Glu Ala Met Glu Arg
   130 135 140
   Phe Gly Ser Arg Asn Gly Lys Thr Ser Lys Lys Ile Thr Ile Ala Asp
   145 150 155 160
   Cys Gly Gln Leu Glu
   165
<210> 72
   <211> 256
   <212> PRT
   <213> Homo sapiens
<400> 72
   Met Ala Ala Ala Val Gly Arg Leu Leu Arg Ala Ser Val Ala Arg His
   1 5 10 15
   Val Ser Ala Ile Pro Trp Gly Ile Ser Ala Thr Ala Ala Leu Arg Pro
   20 25 30
   Ala Ala Cys Gly Arg Thr Ser Leu Thr Asn Leu Leu Cys Ser Gly Ser
   35 40 45
   Ser Gln Ala Lys Leu Phe Ser Thr Ser Ser Ser Cys His Ala Pro Ala
   50 55 60
   Val Thr Gln His Ala Pro Tyr Phe Lys Gly Thr Ala Val Val Asn Gly
   65 70 75 80
   Glu Phe Lys Asp Leu Ser Leu Asp Asp Phe Lys Gly Lys Tyr Leu Val
   85 90 95
   Leu Phe Phe Tyr Pro Leu Asp Phe Thr Phe Val Cys Pro Thr Glu Ile
   100 105 110
   Val Ala Phe Ser Asp Lys Ala Asn Glu Phe His Asp Val Asn Cys Glu
   115 120 125
   Val Val Ala Val Ser Val Asp Ser His Phe Ser His Leu Ala Trp Ile
   130 135 140
   Asn Thr Pro Arg Lys Asn Gly Gly Leu Gly His Met Asn Ile Ala Leu
   145 150 155 160
   Leu Ser Asp Leu Thr Lys Gln Ile Ser Arg Asp Tyr Gly Val Leu Leu
   165 170 175
   Glu Gly Ser Gly Leu Ala Leu Arg Gly Leu Phe Ile Ile Asp Pro Asn
   180 185 190
   Gly Val Ile Lys His Leu Ser Val Asn Asp Leu Pro Val Gly Arg Ser
   195 200 205
   Val Glu Glu Thr Leu Arg Leu Val Lys Ala Phe Gln Tyr Val Glu Thr
   210 215 220
   His Gly Glu Val Cys Pro Ala Asn Trp Thr Pro Asp Ser Pro Thr Ile
   225 230 235 240
   Lys Pro Ser Pro Ala Ala Ser Lys Glu Tyr Phe Gln Lys Val Asn Gln
   245 250 255
<210> 73
   <211> 358
   <212> PRT
   <213> Homo sapiens
<400> 73
<210> 74
   <211> 335
   <212> PRT
   <213> Homo sapiens
<400> 74
   Met Ala Ala Gly Ala Ala Glu Ala Ala Val Ala Ala Val Glu Glu Val
   1 5 10 15
   Gly Ser Ala Gly Gln Phe Glu Glu Leu Leu Arg Leu Lys Ala Lys Ser
   20 25 30
   Leu Leu Val Val His Phe Trp Ala Pro Trp Ala Pro Gln Cys Ala Gln
   35 40 45
   Met Asn Glu Val Met Ala Glu Leu Ala Lys Glu Leu Pro Gln Val Ser
   50 55 60
   Phe Val Lys Leu Glu Ala Glu Gly Val Pro Glu Val Ser Glu Lys Tyr
   65 70 75 80
   Glu Ile Ser Ser Val Pro Thr Phe Leu Phe Phe Lys Asn Ser Gln Lys
   85 90 95
   Ile Asp Arg Leu Asp Gly Ala His Ala Pro Glu Leu Thr Lys Lys Val
   100 105 110
   Gln Arg His Ala Ser Ser Gly Ser Phe Leu Pro Ser Ala Asn Glu His
   115 120 125
   Leu Lys Glu Asp Leu Asn Leu Arg Leu Lys Lys Leu Thr His Ala Ala
   130 135 140
   Pro Cys Met Leu Phe Met Lys Gly Thr Pro Gln Glu Pro Arg Cys Gly
   145 150 155 160
   Phe Ser Lys Gln Met Val Glu Ile Leu His Lys His Asn Ile Gln Phe
   165 170 175
   Ser Ser Phe Asp Ile Phe Ser Asp Glu Glu Val Arg Gln Gly Leu Lys
   180 185 190
   Ala Tyr Ser Ser Trp Pro Thr Tyr Pro Gln Leu Tyr Val Ser Gly Glu
   195 200 205
   Leu Ile Gly Gly Leu Asp Ile Ile Lys Glu Leu Glu Ala Ser Glu Glu
   210 215 220
   Leu Asp Thr Ile Cys Pro Lys Ala Pro Lys Leu Glu Glu Arg Leu Lys
   225 230 235 240
   Val Leu Thr Asn Lys Ala Ser Val Met Leu Phe Met Lys Gly Asn Lys
   245 250 255
   Gln Glu Ala Lys Cys Gly Phe Ser Lys Gln Ile Leu Glu Ile Leu Asn
   260 265 270
   Ser Thr Gly Val Glu Tyr Glu Thr Phe Asp Ile Leu Glu Asp Glu Glu
   275 280 285
   Val Arg Gln Gly Leu Lys Ala Tyr Ser Asn Trp Pro Thr Tyr Pro Gln
   290 295 300
   Leu Tyr Val Lys Gly Glu Leu Val Gly Gly Leu Asp Ile Val Lys Glu
   305 310 315 320
   Leu Lys Glu Asn Gly Glu Leu Leu Pro Ile Leu Arg Gly Glu Asn
   325 330 335
<210> 75
   <211> 201
   <212> PRT
   <213> Homo sapiens
<400> 75
   Met Thr Glu Lys Ala Pro Glu Pro His Val Glu Glu Asp Asp Asp Asp
   1 5 10 15
   Glu Leu Asp Ser Lys Leu Asn Tyr Lys Pro Pro Pro Gln Lys Ser Leu
   20 25 30
   Lys Glu Leu Gln Glu Met Asp Lys Asp Asp Glu Ser Leu Ile Lys Tyr
   35 40 45
   Lys Lys Thr Leu Leu Gly Asp Gly Pro Val Val Thr Asp Pro Lys Ala
   50 55 60
   Pro Asn Val Val Val Thr Arg Leu Thr Leu Val Cys Glu Ser Ala Pro
   65 70 75 80
   Gly Pro Ile Thr Met Asp Leu Thr Gly Asp Leu Glu Ala Leu Lys Lys
   85 90 95
   Glu Thr Ile Val Leu Lys Glu Gly Ser Glu Tyr Arg Val Lys Ile His
   100 105 110
   Phe Lys Val Asn Arg Asp Ile Val Ser Gly Leu Lys Tyr Val Gln His
   115 120 125
   Thr Tyr Arg Thr Gly Val Lys Val Asp Lys Ala Thr Phe Met Val Gly
   130 135 140
   Ser Tyr Gly Pro Arg Pro Glu Glu Tyr Glu Phe Leu Thr Pro Val Glu
   145 150 155 160
   Glu Ala Pro Lys Gly Met Leu Ala Arg Gly Thr Tyr His Asn Lys Ser
   165 170 175
   Phe Phe Thr Asp Asp Asp Lys Gln Asp His Leu Ser Trp Glu Trp Asn
   180 185 190
   Leu Ser Ile Lys Lys Glu Trp Thr Glu
   195 200
<210> 76
   <211> 210
   <212> PRT
   <213> Homo sapiens
<400> 76
   Met Pro Pro Tyr Thr Val Val Tyr Phe Pro Val Arg Gly Arg Cys Ala
   1 5 10 15
   Ala Leu Arg Met Leu Leu Ala Asp Gln Gly Gln Ser Trp Lys Glu Glu
   20 25 30
   Val Val Thr Val Glu Thr Trp Gln Glu Gly Ser Leu Lys Ala Ser Cys
   35 40 45
   Leu Tyr Gly Gln Leu Pro Lys Phe Gln Asp Gly Asp Leu Thr Leu Tyr
   50 55 60
   Gln Ser Asn Thr Ile Leu Arg His Leu Gly Arg Thr Leu Gly Leu Tyr
   65 70 75 80
   Gly Lys Asp Gln Gln Glu Ala Ala Leu Val Asp Met Val Asn Asp Gly
   85 90 95
   Val Glu Asp Leu Arg Cys Lys Tyr Ile Ser Leu Ile Tyr Thr Asn Tyr
   100 105 110
   Glu Ala Gly Lys Asp Asp Tyr Val Lys Ala Leu Pro Gly Gln Leu Lys
   115 120 125
   Pro Phe Glu Thr Leu Leu Ser Gln Asn Gln Gly Gly Lys Thr Phe Ile
   130 135 140
   Val Gly Asp Gln Ile Ser Phe Ala Asp Tyr Asn Leu Leu Asp Leu Leu
   145 150 155 160
   Leu Ile His Glu Val Leu Ala Pro Gly Cys Leu Asp Ala Phe Pro Leu
   165 170 175
   Leu Ser Ala Tyr Val Gly Arg Leu Ser Ala Arg Pro Lys Leu Lys Ala
   180 185 190
   Phe Leu Ala Ser Pro Glu Tyr Val Asn Leu Pro Ile Asn Gly Asn Gly
   195 200 205
   Lys Gln
   210
<210> 77
   <211> 198
   <212> PRT
   <213> Homo sapiens
<400> 77
   Met Ala Ser Gly Asn Ala Arg Ile Gly Lys Pro Ala Pro Asp Phe Lys
   1 5 10 15
   Ala Thr Ala Val Val Asp Gly Ala Phe Lys Glu Val Lys Leu Ser Asp
   20 25 30
   Tyr Lys Gly Lys Tyr Val Val Leu Phe Phe Tyr Pro Leu Asp Phe Thr
   35 40 45
   Phe Val Cys Pro Thr Glu Ile Ile Ala Phe Ser Asn Arg Ala Glu Asp
   50 55 60
   Phe Arg Lys Leu Gly Cys Glu Val Leu Gly Val Ser Val Asp Ser Gln
   65 70 75 80
   Phe Thr His Leu Ala Trp Ile Asn Thr Pro Arg Lys Glu Gly Gly Leu
   85 90 95
   Gly Pro Leu Asn Ile Pro Leu Leu Ala Asp Val Thr Arg Arg Leu Ser
   100 105 110
   Glu Asp Tyr Gly Val Leu Lys Thr Asp Glu Gly Ile Ala Tyr Arg Gly
   115 120 125
   Leu Phe Ile Ile Asp Gly Lys Gly Val Leu Arg Gln Ile Thr Val Asn
   130 135 140
   Asp Leu Pro Val Gly Arg Ser Val Asp Glu Ala Leu Arg Leu Val Gln
   145 150 155 160
   Ala Phe Gln Tyr Thr Asp Glu His Gly Glu Val Cys Pro Ala Gly Trp
   165 170 175
   Lys Pro Gly Ser Asp Thr Ile Lys Pro Asn Val Asp Asp Ser Lys Glu
   180 185 190
   Tyr Phe Ser Lys His Asn
   195
<210> 78
   <211> 343
   <212> PRT
   <213> Homo sapiens
<400> 78
   Met Pro Glu Glu Thr Gln Thr Gln Asp Gln Pro Met Glu Glu Glu Glu
   1 5 10 15
   Val Glu Thr Phe Ala Phe Gln Ala Glu Ile Ala Gln Leu Met Ser Leu
   20 25 30
   Ile Ile Asn Thr Phe Tyr Ser Asn Lys Glu Ile Phe Leu Arg Glu Leu
   35 40 45
   Ile Ser Asn Ser Ser Asp Ala Leu Asp Lys Ile Trp Tyr Glu Ser Leu
   50 55 60
   Thr Asp Pro Ser Lys Leu Asp Ser Gly Lys Glu Leu His Ile Asn Leu
   65 70 75 80
   Ile Pro Asn Lys Gln Asp Gln Thr Leu Thr Ile Val Asp Thr Gly Ile
   85 90 95
   Gly Met Thr Lys Ala Asp Leu Ile Asn Asn Leu Gly Thr Ile Ala Lys
   100 105 110
   Ser Gly Thr Lys Ala Phe Met Glu Ala Leu Gln Ala Gly Ala Asp Ile
   115 120 125
   Ser Met Ile Gly Gln Phe Gly Val Ser Phe Tyr Ser Ala Tyr Leu Val
   130 135 140
   Ala Glu Lys Val Thr Val Ile Thr Lys His Asn Asp Asp Glu Gln Tyr
   145 150 155 160
   Ala Trp Glu Ser Ser Ala Gly Gly Ser Phe Thr Val Arg Thr Asp Thr
   165 170 175
   Gly Glu Arg Met Gly Arg Gly Thr Lys Val Ile Leu His Leu Lys Glu
   180 185 190
   Asp Gln Thr Glu Tyr Leu Glu Glu Gln Arg Ile Lys Glu Ile Val Lys
   195 200 205
   Lys His Ser Gln Leu Ile Gly Tyr Pro Ile Thr Leu Phe Val Glu Lys
   210 215 220
   Glu Cys Asp Lys Glu Val Ser Asp Asp Glu Thr Glu Glu Lys Glu Asp
   225 230 235 240
   Lys Glu Glu Glu Lys Glu Lys Glu Glu Lys Glu Ser Lys Asp Lys Pro
   245 250 255
   Glu Ile Glu Asp Val Gly Ser Asp Glu Glu Glu Glu Lys Lys Asp Gly
   260 265 270
   Asp Lys Lys Lys Lys Lys Thr Lys Glu Lys Tyr Ile Asp Gln Glu Glu
   275 280 285
   Leu Asn Lys Thr Lys Pro Ile Trp Thr Arg Asn Pro Asp Asp Ile Thr
   290 295 300
   Asn Glu Glu Tyr Gly Glu Phe Cys Lys Asn Leu Thr Asn Asp Trp Glu
   305 310 315 320
   Asp His Leu Ala Val Lys His Phe Ser Val Glu Gly Gln Leu Glu Phe
   325 330 335
   Arg Ala Leu Leu Phe Val Pro
   340
<210> 79
   <211> 1224
   <212> PRT
   <213> Homo sapiens
<400> 79
<210> 80
   <211> 494
   <212> PRT
   <213> Homo sapiens
<400> 80
<210> 81
   <211> 375
   <212> PRT
   <213> Homo sapiens
<400> 81
   Met Asp Asp Asp Ile Ala Ala Leu Val Val Asp Asn Gly Ser Gly Met
   1               5                   10                  15
   Cys Lys Ala Gly Phe Ala Gly Asp Asp Ala Pro Arg Ala Val Phe Pro
               20                  25                  30
   Ser Ile Val Gly Arg Pro Arg His Gln Gly Val Met Val Gly Met Gly
           35                  40                  45
   Gln Lys Asp Ser Tyr Val Gly Asp Glu Ala Gln Ser Lys Arg Gly Ile
       50                  55                  60
   Leu Thr Leu Lys Tyr Pro Ile Glu His Gly Ile Val Thr Asn Trp Asp
   65                  70                  75                  80
   Asp Met Glu Lys Ile Trp His His Thr Phe Tyr Asn Glu Leu Arg Val
   85 90 95
   Ala Pro Glu Glu His Pro Val Leu Leu Thr Glu Ala Pro Leu Asn Pro
   100 105 110
   Lys Ala Asn Arg Glu Lys Met Thr Gln Ile Met Phe Glu Thr Phe Asn
   115 120 125
   Thr Pro Ala Met Tyr Val Ala Ile Gln Ala Val Leu Ser Leu Tyr Ala
   130 135 140
   Ser Gly Arg Thr Thr Gly Ile Val Met Asp Ser Gly Asp Gly Val Thr
   145 150 155 160
   His Thr Val Pro Ile Tyr Glu Gly Tyr Ala Leu Pro His Ala Ile Leu
   165 170 175
   Arg Leu Asp Leu Ala Gly Arg Asp Leu Thr Asp Tyr Leu Met Lys Ile
   180 185 190
   Leu Thr Glu Arg Gly Tyr Ser Phe Thr Thr Thr Ala Glu Arg Glu Ile
   195 200 205
   Val Arg Asp Ile Lys Glu Lys Leu Cys Tyr Val Ala Leu Asp Phe Glu
   210 215 220
   Gln Glu Met Ala Thr Ala Ala Ser Ser Ser Ser Leu Glu Lys Ser Tyr
   225 230 235 240
   Glu Leu Pro Asp Gly Gln Val Ile Thr Ile Gly Asn Glu Arg Phe Arg
   245 250 255
   Cys Pro Glu Ala Leu Phe Gln Pro Ser Phe Leu Gly Met Glu Ser Cys
   260 265 270
   Gly Ile His Glu Thr Thr Phe Asn Ser Ile Met Lys Cys Asp Val Asp
   275 280 285
   Ile Arg Lys Asp Leu Tyr Ala Asn Thr Val Leu Ser Gly Gly Thr Thr
   290 295 300
   Met Tyr Pro Gly Ile Ala Asp Arg Met Gln Lys Glu Ile Thr Ala Leu
   305 310 315 320
   Ala Pro Ser Thr Met Lys Ile Lys Ile Ile Ala Pro Pro Glu Arg Lys
                   325                 330                 335
   Tyr Ser Val Trp Ile Gly Gly Ser Ile Leu Ala Ser Leu Ser Thr Phe
               340                 345                 350
   Gln Gln Met Trp Ile Ser Lys Gln Glu Tyr Asp Glu Ser Gly Pro Ser
           355                 360                 365
   Ile Val His Arg Lys Cys Phe
       370                 375
<210> 82
   <211> 1075
   <212> PRT
   <213> Homo sapiens
<400> 82
   Met Val Val Glu Val Asp Ser Met Pro Ala Ala Ser Ser Val Lys Lys
   1               5                   10                  15
   Pro Phe Gly Leu Arg Ser Lys Met Gly Lys Trp Cys Cys Arg Cys Phe
               20                  25                  30
   Pro Cys Tyr Arg Glu Ser Gly Lys Ser Asn Val Gly Thr Ser Gly Asp
           35                  40                  45
   His Asp Asp Ser Ala Met Lys Thr Leu Arg Ser Lys Met Gly Lys Trp
       50                  55                  60
   Cys His His Cys Phe Pro Cys Cys Arg Gly Ser Gly Lys Ser Asn Val
   65                  70                  75                  80
   Gly Ala Ser Gly Asp His Asp Asp Ser Ala Met Lys Thr Leu Arg Asn
                   85                  90                  95
   Lys Met Gly Lys Trp Cys Cys His Cys Phe Pro Cys Cys Arg Gly Ser
               100                 105                 110
   Gly Lys Ser Lys Val Gly Ala Trp Gly Asp Tyr Asp Asp Ser Ala Phe
           115                 120                 125
   Met Glu Pro Arg Tyr His Val Arg Gly Glu Asp Leu Asp Lys Leu His
       130                 135                 140
   Arg Ala Ala Trp Trp Gly Lys Val Pro Arg Lys Asp Leu Ile Val Met
   145                 150                 155                 160
   Leu Arg Asp Thr Asp Val Asn Lys Lys Asp Lys Gln Lys Arg Thr Ala
                   165                 170                 175
   Leu His Leu Ala Ser Ala Asn Gly Asn Ser Glu Val Val Lys Leu Leu
               180                 185                 190
   Leu Asp Arg Arg Cys Gln Leu Asn Val Leu Asp Asn Lys Lys Arg Thr
           195                 200                 205
   Ala Leu Ile Lys Ala Val Gln Cys Gln Glu Asp Glu Cys Ala Leu Met
       210                 215                 220
   Leu Leu Glu His Gly Thr Asp Pro Asn Ile Pro Asp Glu Tyr Gly Asn
   225                 230                 235                 240
   Thr Thr Leu His Tyr Ala Ile Tyr Asn Glu Asp Lys Leu Met Ala Lys
                   245                 250                 255
   Ala Leu Leu Leu Tyr Gly Ala Asp Ile Glu Ser Lys Asn Lys His Gly
               260                 265                 270
   Leu Thr Pro Leu Leu Leu Gly Val His Glu Gln Lys Gln Gln Val Val
           275                 280                 285
   Lys Phe Leu Ile Lys Lys Lys Ala Asn Leu Asn Ala Leu Asp Arg Tyr
       290                 295                 300
   Gly Arg Thr Ala Leu Ile Leu Ala Val Cys Cys Gly Ser Ala Ser Ile
   305                 310                 315                 320
   Val Ser Leu Leu Leu Glu Gln Asn Ile Asp Val Ser Ser Gln Asp Leu
                   325                 330                 335
   Ser Gly Gln Thr Ala Arg Glu Tyr Ala Val Ser Ser His His His Val
               340                 345                 350
   Ile Cys Gln Leu Leu Ser Asp Tyr Lys Glu Lys Gln Met Leu Lys Ile
           355                 360                 365
   Ser Ser Glu Asn Ser Asn Pro Glu Gln Glu Leu Lys Leu Thr Ser Glu
       370                 375                 380
   Glu Glu Ser Gln Arg Phe Lys Gly Ser Glu Asn Ser Gln Pro Glu Lys
   385                 390                 395                 400
   Met Ser Gln Glu Leu Glu Ile Asn Lys Asp Gly Asp Arg Glu Val Glu
   405 410 415
   Glu Glu Met Lys Lys His Glu Ser Asn Asn Val Gly Leu Leu Glu Asn
   420 425 430
   Leu Thr Asn Gly Val Thr Ala Gly Asn Gly Asp Asn Gly Leu Ile Pro
   435 440 445
   Gln Arg Lys Ser Arg Thr Pro Glu Asn Gln Gln Phe Pro Asp Asn Glu
   450 455 460
   Ser Glu Glu Tyr His Arg Ile Cys Glu Leu Leu Ser Asp Tyr Lys Glu
   465 470 475 480
   Lys Gln Met Pro Lys Tyr Ser Ser Glu Asn Ser Asn Pro Glu Gln Asp
   485 490 495
   Leu Lys Leu Thr Ser Glu Glu Glu Ser Gln Arg Leu Lys Gly Ser Glu
   500 505 510
   Asn Gly Gln Pro Glu Lys Arg Ser Gln Glu Pro Glu Ile Asn Lys Asp
   515 520 525
   Gly Asp Arg Glu Leu Glu Asn Phe Met Ala Ile Glu Glu Met Lys Lys
   530 535 540
   His Gly Ser Thr His Val Gly Phe Pro Glu Asn Leu Thr Asn Gly Ala
   545 550 555 560
   Thr Ala Gly Asn Gly Asp Asp Gly Leu Ile Pro Pro Arg Lys Ser Arg
   565 570 575
   Thr Pro Glu Ser Gln Gln Phe Pro Asp Thr Glu Asn Glu Glu Tyr His
   580 585 590
   Ser Asp Glu Gln Asn Asp Thr Gln Lys Gln Phe Cys Glu Glu Gln Asn
   595 600 605
   Thr Gly Ile Leu His Asp Glu Ile Leu Ile His Glu Glu Lys Gln Ile
   610 615 620
   Glu Val Val Glu Lys Met Asn Ser Glu Leu Ser Leu Ser Cys Lys Lys
   625 630 635 640
   Glu Lys Asp Val Leu His Glu Asn Ser Thr Leu Arg Glu Glu Ile Ala
   645 650 655
   Met Leu Arg Leu Glu Leu Asp Thr Met Lys His Gln Ser Gln Leu Arg
   660 665 670
   Glu Lys Lys Tyr Leu Glu Asp Ile Glu Ser Val Lys Lys Lys Asn Asp
   675 680 685
   Asn Leu Leu Lys Ala Leu Gln Leu Asn Glu Leu Thr Met Asp Asp Asp
   690 695 700
   Thr Ala Val Leu Val Ile Asp Asn Gly Ser Gly Met Cys Lys Ala Gly
   705 710 715 720
   Phe Ala Gly Asp Asp Ala Pro Arg Ala Val Phe Pro Ser Ile Val Gly
   725 730 735
   Arg Pro Arg Gln Gln Gly Met Met Gly Gly Met His Gln Lys Glu Ser
   740 745 750
   Tyr Val Gly Lys Glu Ala Gln Ser Lys Arg Gly Ile Leu Thr Leu Lys
   755 760 765
   Tyr Pro Met Glu His Gly Ile Ile Thr Asn Trp Asp Asp Met Glu Lys
   770 775 780
   Ile Trp His His Thr Phe Tyr Asn Glu Leu Arg Val Ala Pro Glu Glu
   785 790 795 800
   His Pro Ile Leu Leu Thr Glu Ala Pro Leu Asn Pro Lys Ala Asn Arg
   805 810 815
   Glu Lys Met Thr Gln Ile Met Phe Glu Thr Phe Asn Thr Pro Ala Met
   820 825 830
   Tyr Val Ala Ile Gln Ala Val Pro Ser Leu Tyr Thr Ser Gly Arg Thr
   835 840 845
   Thr Gly Ile Val Met Asp Ser Gly Asp Gly Val Thr His Thr Val Pro
   850 855 860
   Ile Tyr Glu Gly Asn Ala Leu Pro His Ala Thr Leu Arg Leu Asp Leu
   865 870 875 880
   Ala Gly Arg Glu Leu Pro Asp Tyr Leu Met Lys Ile Leu Thr Glu Arg
   885 890 895
   Gly Tyr Arg Phe Thr Thr Met Ala Glu Arg Glu Ile Val Arg Asp Ile
   900 905 910
   Lys Glu Lys Leu Cys Tyr Val Ala Leu Asp Phe Glu Gln Glu Met Ala
   915 920 925
   Thr Ala Ala Ser Ser Ser Ser Leu Glu Lys Ser Tyr Glu Leu Pro Asp
   930 935 940
   Gly Gln Val Ile Thr Ile Gly Asn Glu Arg Phe Arg Cys Pro Glu Ala
   945 950 955 960
   Leu Phe Gln Pro Cys Phe Leu Gly Met Glu Ser Cys Gly Ile His Glu
   965 970 975
   Thr Thr Phe Asn Ser Ile Met Lys Ser Asp Val Asp Ile Arg Lys Asp
   980 985 990
   Leu Tyr Thr Asn Thr Val Leu Ser Gly Gly Thr Thr Met Tyr Pro Gly
   995 1000 1005
   Met Ala His Arg Met Gln Lys Glu Ile Ala Ala Leu Ala Pro Ser
   1010 1015 1020
   Met Met Lys Ile Arg Ile Ile Ala Pro Pro Lys Arg Lys Tyr Ser
   1025 1030 1035
   Val Trp Val Gly Gly Ser Ile Leu Ala Ser Leu Ser Thr Phe Gln
   1040 1045 1050
   Gln Met Trp Ile Ser Lys Gln Glu Tyr Asp Glu Ser Gly Pro Ser
   1055 1060 1065
   Ile Val His Arg Lys Cys Phe
   1070 1075
<210> 83
   <211> 294
   <212> PRT
   <213> Homo sapiens
<400> 83
   Met Glu Asp Ser Met Asp Met Asp Met Ser Pro Leu Arg Pro Gln Asn
   1 5 10 15
   Tyr Leu Phe Gly Cys Glu Leu Lys Ala Asp Lys Asp Tyr His Phe Lys
               20                  25                  30
   Val Asp Asn Asp Glu Asn Glu His Gln Leu Ser Leu Arg Thr Val Ser
           35                  40                  45
   Leu Gly Ala Gly Ala Lys Asp Glu Leu His Ile Val Glu Ala Glu Ala
       50                  55                  60
   Met Asn Tyr Glu Gly Ser Pro Ile Lys Val Thr Leu Ala Thr Leu Lys
   65                  70                  75                  80
   Met Ser Val Gln Pro Thr Val Ser Leu Gly Gly Phe Glu Ile Thr Pro
                   85                  90                  95
   Pro Val Val Leu Arg Leu Lys Cys Gly Ser Gly Pro Val His Ile Ser
               100                 105                 110
   Gly Gln His Leu Val Ala Val Glu Glu Asp Ala Glu Ser Glu Asp Glu
           115                 120                 125
   Glu Glu Glu Asp Val Lys Leu Leu Ser Ile Ser Gly Lys Arg Ser Ala
       130                 135                 140
   Pro Gly Gly Gly Ser Lys Val Pro Gln Lys Lys Val Lys Leu Ala Ala
   145                 150                 155                 160
   Asp Glu Asp Asp Asp Asp Asp Asp Glu Glu Asp Asp Asp Glu Asp Asp
                   165                 170                 175
   Asp Asp Asp Asp Phe Asp Asp Glu Glu Ala Glu Glu Lys Ala Pro Val
               180                 185                 190
   Lys Lys Ser Ile Arg Asp Thr Pro Ala Lys Asn Ala Gln Lys Ser Asn
           195                 200                 205
   Gln Asn Gly Lys Asp Ser Lys Pro Ser Ser Thr Pro Arg Ser Lys Gly
       210                 215                 220
   Gln Glu Ser Phe Lys Lys Gln Glu Lys Thr Pro Lys Thr Pro Lys Gly
   225                 230                 235                 240
   Pro Ser Ser Val Glu Asp Ile Lys Ala Lys Met Gln Ala Ser Ile Glu
                   245                 250                 255
   Lys Gly Gly Ser Leu Pro Lys Val Glu Ala Lys Phe Ile Asn Tyr Val
   260 265 270
   Lys Asn Cys Phe Arg Met Thr Asp Gln Glu Ala Ile Gln Asp Leu Trp
   275 280 285
   Gln Trp Arg Lys Ser Leu
   290
<210> 84
   <211> 858
   <212> PRT
   <213> Homo sapiens
<400> 84
<210> 85
   <211> 397
   <212> PRT
   <213> Homo sapiens
<400> 85
   Met Val Lys Glu Thr Thr Tyr Tyr Asp Val Leu Gly Val Lys Pro Asn
   1 5 10 15
   Ala Thr Gln Glu Glu Leu Lys Lys Ala Tyr Arg Lys Leu Ala Leu Lys
   20 25 30
   Tyr His Pro Asp Lys Asn Pro Asn Glu Gly Glu Lys Phe Lys Gln Ile
   35 40 45
   Ser Gln Ala Tyr Glu Val Leu Ser Asp Ala Lys Lys Arg Glu Leu Tyr
   50 55 60
   Asp Lys Gly Gly Glu Gln Ala Ile Lys Glu Gly Gly Ala Gly Gly Gly
   65 70 75 80
   Phe Gly Ser Pro Met Asp Ile Phe Asp Met Phe Phe Gly Gly Gly Gly
   85 90 95
   Arg Met Gln Arg Glu Arg Arg Gly Lys Asn Val Val His Gln Leu Ser
   100 105 110
   Val Thr Leu Glu Asp Leu Tyr Asn Gly Ala Thr Arg Lys Leu Ala Leu
   115 120 125
   Gln Lys Asn Val Ile Cys Asp Lys Cys Glu Gly Arg Gly Gly Lys Lys
   130 135 140
   Gly Ala Val Glu Cys Cys Pro Asn Cys Arg Gly Thr Gly Met Gln Ile
   145 150 155 160
   Arg Ile His Gln Ile Gly Pro Gly Met Val Gln Gln Ile Gln Ser Val
   165 170 175
   Cys Met Glu Cys Gln Gly His Gly Glu Arg Ile Ser Pro Lys Asp Arg
   180 185 190
   Cys Lys Ser Cys Asn Gly Arg Lys Ile Val Arg Glu Lys Lys Ile Leu
   195 200 205
   Glu Val His Ile Asp Lys Gly Met Lys Asp Gly Gln Lys Ile Thr Phe
   210 215 220
   His Gly Glu Gly Asp Gln Glu Pro Gly Leu Glu Pro Gly Asp Ile Ile
   225 230 235 240
   Ile Val Leu Asp Gln Lys Asp His Ala Val Phe Thr Arg Arg Gly Glu
   245 250 255
   Asp Leu Phe Met Cys Met Asp Ile Gln Leu Val Glu Ala Leu Cys Gly
   260 265 270
   Phe Gln Lys Pro Ile Ser Thr Leu Asp Asn Arg Thr Ile Val Ile Thr
   275 280 285
   Ser His Pro Gly Gln Ile Val Lys His Gly Asp Ile Lys Cys Val Leu
   290 295 300
   Asn Glu Gly Met Pro Ile Tyr Arg Arg Pro Tyr Glu Lys Gly Arg Leu
   305 310 315 320
   Ile Ile Glu Phe Lys Val Asn Phe Pro Glu Asn Gly Phe Leu Ser Pro
   325 330 335
   Asp Lys Leu Ser Leu Leu Glu Lys Leu Leu Pro Glu Arg Lys Glu Val
   340 345 350
   Glu Glu Thr Asp Glu Met Asp Gln Val Glu Leu Val Asp Phe Asp Pro
   355 360 365
   Asn Gln Glu Arg Arg Arg His Tyr Asn Gly Glu Ala Tyr Glu Asp Asp
   370 375 380
   Glu His His Pro Arg Gly Gly Val Gln Cys Gln Thr Ser
   385 390 395
<210> 86
   <211> 375
   <212> PRT
   <213> Homo sapiens
<400> 86
<210> 87
   <211> 406
   <212> PRT
   <213> Homo sapiens
<400> 87
   Met Ala Leu Asp Gly Pro Glu Gln Met Glu Leu Glu Glu Gly Lys Ala
   1 5 10 15
   Gly Ser Gly Leu Arg Gln Tyr Tyr Leu Ser Lys Ile Glu Glu Leu Gln
   20 25 30
   Leu Ile Val Asn Asp Lys Ser Gln Asn Leu Arg Arg Leu Gln Ala Gln
   35 40 45
   Arg Asn Glu Leu Asn Ala Lys Val Arg Leu Leu Arg Glu Glu Leu Gln
   50 55 60
   Leu Leu Gln Glu Gln Gly Ser Tyr Val Gly Glu Val Val Arg Ala Met
   65 70 75 80
   Asp Lys Lys Lys Val Leu Val Lys Val His Pro Glu Gly Lys Phe Val
   85 90 95
   Val Asp Val Asp Lys Asn Ile Asp Ile Asn Asp Val Thr Pro Asn Cys
   100 105 110
   Arg Val Ala Leu Arg Asn Asp Ser Tyr Thr Leu His Lys Ile Leu Pro
   115 120 125
   Asn Lys Val Asp Pro Leu Val Ser Leu Met Met Val Glu Lys Val Pro
   130 135 140
   Asp Ser Thr Tyr Glu Met Ile Gly Gly Leu Asp Lys Gln Ile Lys Glu
   145 150 155 160
   Ile Lys Glu Val Ile Glu Leu Pro Val Lys His Pro Glu Leu Phe Glu
   165 170 175
   Ala Leu Gly Ile Ala Gln Pro Lys Gly Val Leu Leu Tyr Gly Pro Pro
   180 185 190
   Gly Thr Gly Lys Thr Leu Leu Ala Arg Ala Val Ala His His Thr Asp
   195 200 205
   Cys Thr Phe Ile Arg Val Ser Gly Ser Glu Leu Val Gln Lys Phe Ile
   210 215 220
   Gly Glu Gly Ala Arg Met Val Arg Glu Leu Phe Val Met Ala Arg Glu
   225 230 235 240
   His Ala Pro Ser Ile Ile Phe Met Asp Glu Ile Asp Ser Ile Gly Ser
   245 250 255
   Ser Arg Leu Glu Gly Gly Ser Gly Gly Asp Ser Glu Val Gln Arg Thr
   260 265 270
   Met Leu Glu Leu Leu Asn Gln Leu Asp Gly Phe Glu Ala Thr Lys Asn
   275 280 285
   Ile Lys Val Ile Met Ala Thr Asn Arg Ile Asp Ile Leu Asp Ser Ala
   290 295 300
   Leu Leu Arg Pro Gly Arg Ile Asp Arg Lys Ile Glu Phe Pro Pro Pro
   305 310 315 320
   Asn Glu Glu Ala Arg Leu Asp Ile Leu Lys Ile His Ser Arg Lys Met
   325 330 335
   Asn Leu Thr Arg Gly Ile Asn Leu Arg Lys Ile Ala Glu Leu Met Pro
   340 345 350
   Gly Ala Ser Gly Ala Glu Val Lys Gly Val Cys Thr Glu Ala Gly Met
   355 360 365
   Tyr Ala Leu Arg Glu Arg Arg Val His Val Thr Gln Glu Asp Phe Glu
   370 375 380
   Met Ala Val Ala Lys Val Met Gln Lys Asp Ser Glu Lys Asn Met Ser
   385 390 395 400
   Ile Lys Lys Leu Trp Lys
   405
<210> 88
   <211> 563
   <212> PRT
   <213> Homo sapiens
<400> 88